# EUROPEAN PATENT SPECIFICATION

(11) **EP 1 919 512 B1**
(45) Date of publication and mention of the grant of the patent: **12.11.2014**
(21) Application number: 06789691.0
(22) Date of filing: 10.08.2006
(51) Int. Cl.: C12N 15/113

(54) **CHEMICALLY MODIFIED OLIGONUCLEOTIDES FOR USE IN MODULATING MICRO RNA AND USES THEREOF**
CHEMISCH MODIFIZIERTE OLIGONUKLEOTIDE ZUR VERWENDUNG BEI DER MODULIERUNG VON MIKRO-RNA UND IHRE VERWENDUNGEN
OLIGONUCLÉOTIDES CHIMIQUEMENT MODIFIÉS POUVANT ÊTRE EMPLOYÉS DANS LA MODULATION DE MICRO-ARN ET APPLICATIONS

(30) Priority: 10.08.2005 US 706866 P; 28.10.2005 US 731554 P; 26.01.2006 US 763201 P
(43) Date of publication of application: 14.05.2008
(73) Proprietor: Alnylam Pharmaceuticals Inc., Cambridge, MA 02142 (US); THE ROCKEFELLER UNIVERSITY, New York, NY 10021 (US)
(72) Inventor: STOFFEL, Markus, New York, NY 10021 (US); MANOHARAN, Muthiah, Weston, MA 02493 (US); RAJEEV, Kallanthottathil G., Wayland, MA 01778 (US)
(74) Representative: Grünecker, Kinkeldey, Stockmair & Schwanhäusser
(86) International application number: PCT/US2006/031313
(87) International publication number: WO 2007/021896

(56) References cited:
- WO-A2-2005/107816
- CHANG JINHONG ET AL.: "miR-122, a mammalian liver-specific microRNA, is processed from hcr mRNA and may downregulate the high affinity cationic amino acid transporter CAT-1" RNA BIOLOGY, vol. 1, no. 2, July 2004 (2004-07), pages 106-113, XP002591841 ISSN: 1555-8584
- LEWIS BENJAMIN P. ET AL.: "Conserved seed pairing, often flanked by adenosines, indicates that thousands of human genes are microRNA targets" CELL 14 JAN 2005 LNKD- PUBMED:15652477, vol. 120, no. 1, 14 January 2005 (2005-01-14), pages 15-20, XP002591842 ISSN: 0092-8674
- KREK AZRA ET AL.: "Combinatorial microRNA target predictions" NATURE GENETICS, vol. 37, no. 5, May 2005 (2005-05), pages 495-500, XP002591843 ISSN: 1061-4036
- KLOOSTERMAN ET AL.: 'Targeted Inhibition of miRNA Maturation with Morpholinos Reveals a Role for mir-375 in Pancreatic Islet Development.' PLOS BIOLOGY vol. 5, August 2007, pages 1738 - 1749, XP008125606
- KRUTZFELDT ET AL.: 'Silencing of microRNAs in vivo with 'antagomirs'.' NATURE. vol. 438, no. 7068, December 2005, pages 685 - 689, XP008125598
- PAROO ET AL.: 'Challenges for RNAi in vivo.' TRENDS IN BIOTECHNOLOGY. vol. 22, no. 8, August 2004, pages 390 - 394, XP004551069
- CAPLEN ET AL.: 'RNAi as a gene therapy approach.' EXPERT OPIN. BIOL. THER. vol. 3, no. 4, 2003, pages 575 - 586, XP008055014
- STANKOV ET AL.: 'Antisense antiviral agents: about the optimal approach.' MEDICAL HYPOTHESIS. vol. 54, no. 3, 2000, pages 501 - 502, XP008126093
- Hommes: "Inborn errors of fructose metabolism.", American Journal of Clinical Nutrition, vol. 58, no. 5 Suppl, 1 November 1993 (1993-11-01), pages 788S-795S, XP055064250, ISSN: 0002-9165

## Description

### GOVERNMENT SUPPORT

The work described herein was carried out, at least in part, using funds from the United States government under grant number 1 P01 GM073047-01, awarded by the National Institutes of Health (NIH). The United States government may therefore have certain rights in the invention.

### TECHNICAL FIELD

This invention relates generally to chemically modified oligonucleotides (antagomirs) useful for modulating expression of microRNAs. More particularly, the invention relates to single stranded, double stranded, partially double stranded and hairpin structured chemically modified oligonucleotides for inhibiting microRNA expression and to methods of making and using the modified oligonucleotides.

### BACKGROUND

A variety of nucleic acid species are capable of modifying gene expression. These include antisense RNA, siRNA, microRNA, RNA and DNA aptamers, and decoy RNAs. Each of these nucleic acid species can inhibit target nucleic acid activity, including gene expression.

MicroRNAs (miRNAs) are a class of 18-24 nt non-coding RNAs (ncRNAs) that exist in a variety of organisms, including mammals, and are conserved in evolution. miRNAs are processed from hairpin precursors of 70 nt (pre-miRNA) which are derived from primary transcripts (pri-miRNA) through sequential cleavage by the RNAse III enzymes drosha and dicer. miRNAs can be encoded in intergenic regions, hosted within introns of pre-mRNAs or within ncRNA genes. Many microRNAs can be encoded in intergenic regions, hosted within introns of pre-mRNAs or within ncRNA genes. Many miRNAs also tend to be clustered and transcribed as polycistrons and often have similar spatial temporal expression patterns. MiRNAs have been found to have roles in a variety of biological processes including developmental timing, differentiation, apoptosis, cell proliferation, organ development, and metabolism.

CHANG JINHONG et al.: RNA BIOLOGY, vol. 1, no. 2, July 2004, pages 106-113 discloses miR-122 is a microRNA derived from a liver-specifically expressed gene *hcr* and its possible involvement on the downregulation of the high affinity cationic amino acid transporter CAT-1.

LEWIS BENJAMIN P. et al. CELL, vol. 120, no. 1, 14 January 2005, pages 15-20 discloses a technique for predicting targets of vertebrate microRNAs by identifying mRNAs with conserved complementarity to the seed nucleotides of the miRNA.

KREK AZRA et al. NATURE GENETICS, vol. 37, no. 5, May 2005, pages 495-500 describes a method of combinatorial microRNA target prediction.

WO 2005/107816 discloses miRNA inhibitory agents for use in treating viral infections; miR-122 is disclosed as being one target of such inhibitory agents (anti-miRNA oligonucleotides).

HOMMES, F.A., et al. THE AM. J. OF CLINICAL NUTRITION, vol. 58, no. 5 Suppl, 1993 discloses inborn errors of fructose metabolism and reviews disorders related to the deficiency of Aldolase A.

### SUMMARY

The present invention is based in part on the discovery that expression of endogenous microRNAs (miRNAs) or pre-microRNAs (pre-miRNAs) can be inhibited by an agent herein defined as an antagomir, *e.g.*, through systemic administration of the antagomir, as well as by parenteral administration of such agents. Based on these findings, the present invention provides specific compositions and methods that are useful in reducing miRNA and pre-miRNA levels, in *e.g.,* a mammal, such as a human. In particular, the present invention provides specific compositions and methods that are useful for reducing levels of the miRNAmiR-122.

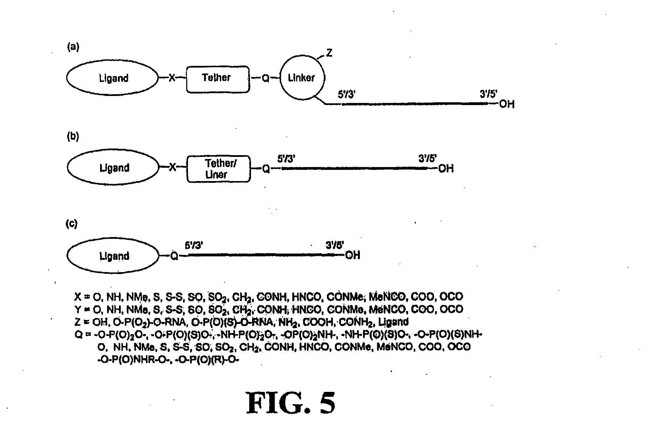

In one aspect, the invention features antagomirs. Antagomirs are single stranded, double stranded, partially double stranded and hairpin structured chemically modified oligonucleotides that target a microRNA. Figures 5-11 provides repsresentative structures of antagomirs.

An antagomir consisting essentially of or comprising at least 12 or more contiguous nucleotides substantially complementary to an endogenous miRNA and more particularly agents that include 12 or more contiguous nucleotides substantially complementary to a target sequence of an miRNA or pre-miRNA nucleotide sequence. Preferably, an antagomir featured in the invention includes a nucleotide sequence sufficiently complementary to hybridize to a miRNA target sequence of about 12 to 25 nucleotides, preferably about 15 to 23 nucleotides. More preferably, the target sequence differs by no more than 1, 2, or 3 nucleotides from a sequence shown in Table 1, and in one embodiment, the antagomir is an agent shown in Table 2a-e and Table 4. In one embodiment, the antagomir includes a non-nucleotide moiety, *e.g.,* a cholesterol moiety. The non-nucleotide moiety can be attached, *e.g.,* to the 3' or 5' end of the oligonucleotide agent. In a preferred embodiment, a cholesterol moiety is attached to the 3' end of the oligonucleotide agent.

Antagomirs are stabilized against nucleolytic degradation such as by the incorporation of a modification, e.g., a nucleotide modification. In another embodiment, the antagomir includes a phosphorothioate at at least the first, second, or third internucleotide linkage at the 5' or 3' end of the nucleotide sequence. In yet another embodiment, the antagomir includes a 2'-modified nucleotide, e.g., a 2'-deoxy, 2'-deoxy-2'-fluoro, 2'-O-methyl, 2'-O-methoxyethyl (2'-O-MOE), 2'-O-aminopropyl (2'-O-AP), 2'-O-dimethylaminoethyl (2'-O-DMAOE), 2'-O-dimethylaminopropyl (2'-O-DMAP), 2'-O-dimethylaminoethyloxyethyl (2'-O-DMAEOE), or 2'-O-N-methylacetamido (2'-O-NMA). In a particularly preferred embodiment, the antagomir includes at least one 2'-O-methyl-modified nucleotide, and in some embodiments, all of the nucleotides of the antagomir include a 2'-O-methyl modification.

An antagomir that is substantially complementary to a nucleotide sequence of an miRNA can be delivered to a cell or a human to inhibit or reduce the activity of an endogenous miRNA, such as when aberrant or undesired miRNA activity, or insufficient activity of a target mRNA that hybridizes to the endogenous miRNA, is linked to a disease or disorder. In one embodiment, an antagomir featured in the invention has a nucleotide sequence that is substantially complementary to miR-122 (see Table 1), which hybridizes to numerous RNAs, including aldolase A mRNA, N-myc downstram regulated gene (Ndrg3) mRNA, IQ motif containing GTPase activating protein-1 (Iqgap1) mRNA, HMG-CoA-reductase (Hmgcr) mRNA, and citrate synthase mRNA and others. In a preferred embodiment, the antagomir that is substantially complementary to miR-122 is antagomir-122 (Table 2a-e and Table 4). Aldolase A deficiencies have been found to be associated with a variety of disorders, including hemolytic anemia, arthrogryposis complex congenita, pituitary ectopia, rhabdomyolysis, hyperkalemia. Humans suffering from aldolase A deficiencies also experience symptoms that include growth and developmental retardation, midfacial hypoplasia, hepatomegaly, as well as myopathic symptoms. Thus a human who has or who is diagnosed as having any of these disorders or symptoms is a candidate to receive treatment with an antagomir that hybridizes to miR-122.

In some instances , an antagomir has a nucleotide sequence that is substantially complementary to miR-16, miR-192, or miR-194.

In one aspect, the invention features a method of reducing the levels of an miRNA or pre-miRNA in a cell of a subject, e.g., a human subject. The method includes the step of administering an antagomir to the subject, where the antagomir is substantially single-stranded and includes a sequence that is substantially complementary to 12 to 23 contiguous nucleotides, and preferably 15 to 23 contiguous nucleotides, of a target sequence of an miRNA or pre-miRNA nucleotide sequence. Preferably, the target sequence differs by no more than 1, 2, or 3 nucleotides from a microRNA or pre-microRNA sequence, such as a microRNA sequence shown in Table 1.

In one embodiment, the methods featured in the invention are useful for reducing the level of an endogenous miRNA (miR-122, ) or pre-miRNA in a cell, e.g, in a cell of a subject, such as a human subject. Such methods include contacting the cell with an antagomir described herein for a time sufficient to allow uptake of the antagomir into the cell.

In another aspect, the invention features a pharmaceutical composition including an antagomir described herein, and a pharmaceutically acceptable carrier. In a preferred embodiment, the antagomir included in the pharmaceutical composition hybridizes to miR-122.

In another aspect the invention features a method of inhibiting miRNA expression ( miR-122, expression) or pre-miRNA expression in a cell, *e.g.,* a cell of a subject. The method includes contacting the cell with an effective amount of an antagomir described herein, which is substantially complementary to the nucleotide sequence of the target miRNA or the target pre-miRNA. Such methods can be performed on a mammalian subject by administering to a subject one of the oligonucleotide agents/pharmaceutical compositions described herein.

In another aspect the invention features a method of increasing levels of an RNA or protein that are encoded by a gene whose expression is down-regulated by an miRNA, e.g., an endogenous miRNA, such as miR-122 . The method includes contacting the cell with an effective amount of an antagomir described herein, which is substantially complementary to the nucleotide sequence of the miRNA that binds to and effectively inhibits translation of the RNA transcribed from the gene. For example, the invention features a method of increasing aldolase A protein levels in a cell. Similarly, the invention features a method of increasing Ndrg3, Iqgap1, Hmgcr, and/or citrate synthase protein levels in a cell. The methods include contacting the cell with an effective amount of an antagomir described herein (e.g., antagomir-122, described in Table 2a-e and Table 4), which is substantially complementary to the nucleotide sequence of miR-122 (see Table 1).

In another aspect, the invention provides methods of increasing expression of a target gene by providing an antagomir to which a lipophilic moiety is conjugated, *e.g.,* a lipophilic conjugated antagomir described herein, to a cell. The antagomir preferably hybridizes to an miRNA (miR-122) or a pre-miRNA. In a preferred embodiment the conjugated antagomir can be used to increase expression of a target gene in an organism, *e.g.,* a mammal, *e.g.,* a human, or to increase expression of a target gene in a cell line or in cells which are outside an organism. An mRNA transcribed from the target gene hybridizes to an endogenous miRNA, which consequently results in downregulation of mRNA expression. An antagomir featured in the invention hybridizes to the endogenous miRNA and consequently causes an increase in mRNA expression. In the case of a whole organism, the method can be used to increase expression of a gene and treat a condition associated with a low level of expression of the gene. For example, an antagomir that targets miR-122 (e.g., antagomir-122) can be used to increase expression of an aldolase A gene to treat a subject having, or at risk for developing, hemolytic anemia, arthrogryposis complex congenita, pituitary ectopia, rhabdomyolysis, hyperkalemia, or any other disorder associated with aldolase A deficiency. Administration of an antagomir that targets miR-122 (e.g., antagomir-122) can be also be used to increase expression of an Ndrg3, Iqgap1, Hmgcr, or citrate synthase gene to treat a subject having, or at risk for developing, a disorder associated with a decreased expression of any one of these genes.

### DESCRIPTION OF DRAWINGS

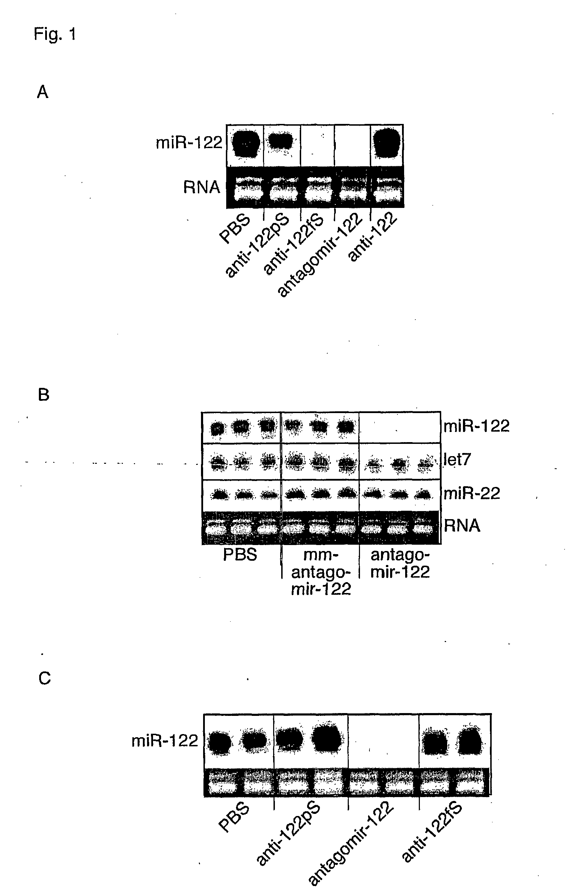

FIG. 1A is a panel of Northern blots of total RNA (15 µg) isolated from mouse liver 24 h after injection of differently modified RNAs (240 mg/kg) targeting miR-122. Samples were separated in 14%-polyacrylamide gels in the absence of formamide, and the membranes were probed for miR-122. Ethidium bromide staining of tRNA is shown as a loading control.
FIG. 1B is a panel of Northern blots of total RNA (15 µg) isolated from mouse liver 24 h after injection of differently modified RNAs (240 mg/kg) against miR-122. Samples were separated in 14%-polyacrylamide gels in the absence of formamide, and the membranes were probed for miR-122, let7, and miR-22 RNAs. Ethidium bromide staining of tRNA is shown as a loading control.
FIG. 1C is a panel of Northern blots of total RNA (15 µg) isolated from mouse liver 24 h after injection of differently modified RNAs (240 mg/kg) against miR-122. Samples were separated in 14%-polyacrylamide gels in the presence of 20% formamide, and the membranes were probed for miR-122. Ethidium bromide staining of tRNA is shown as a loading control.

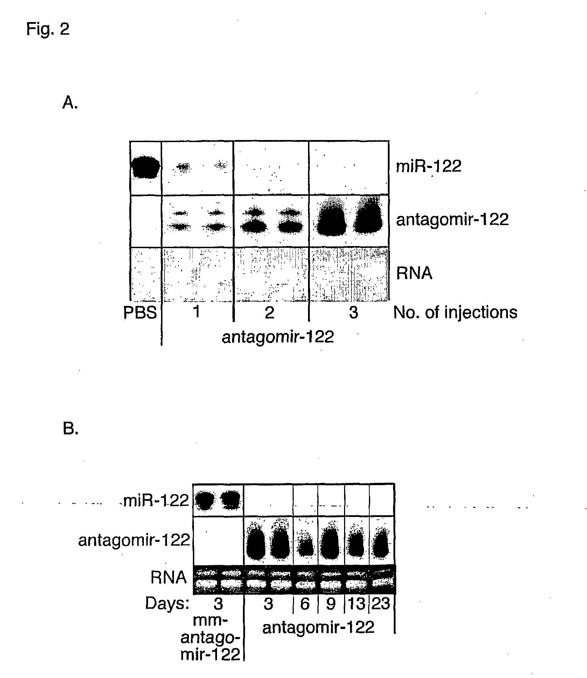

FIG. 2A is a panel of Northern blots of total RNA (15 µg) isolated from mouse livers. RNA was isolated 24 h after injection of 80 mg/kg bodyweight antagomir-122 (n=2) on 1,2, or 3 consecutive days as indicated. Membranes were probed for both the endogenous miR-122 and the injected antagomir-122. Ethidium bromide staining of tRNA is shown as a loading control.
FIG. 2B is a panel of Northern blots of total RNA (15 µg) isolated from mouse livers. RNA was isolated 3, 6, 9, 13, and 23 days after injection of antagomir-122. Membranes were probed for both the endogenous miR-122 and the injected antagomir-122. Ethidium bromide staining of tRNA is shown as a loading control.

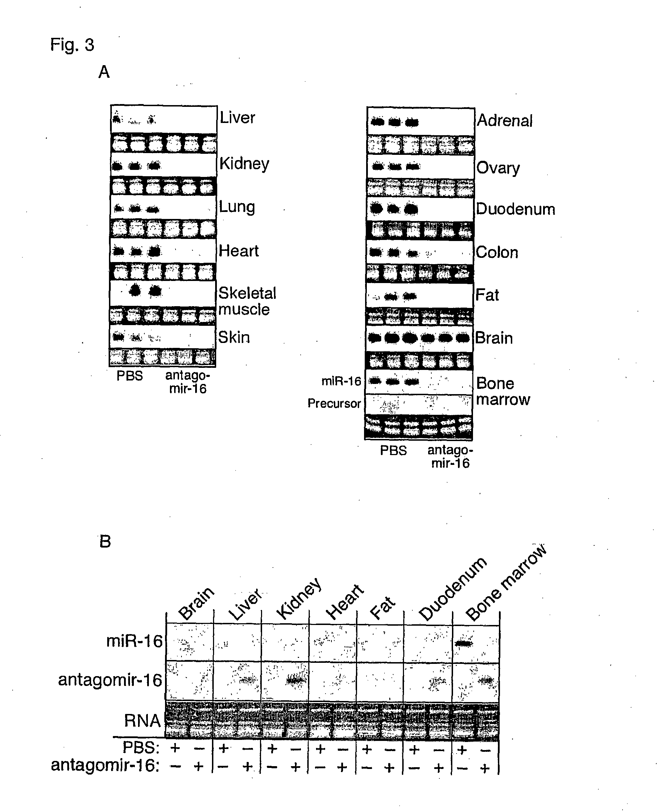

FIG. 3A is a panel of Northern blots of total RNA (10-30 µg) isolated from different mouse tissues 24 h after injection of antagomir-16 (n=3). Membranes were probed for miR-16. The precursor miR-16 transcript was visible on Northern blots of bone marrow and expression was similar in all mice. Ethidium bromide staining of tRNA is shown as a loading control.
FIG. 3B is a panel of Northern blots of total RNA (10-30 µg) isolated from different mouse tissues 24 h after injection of antagomir-16 (n=3). Total RNA from 3 mice were pooled for the detection of miR-16 and the injected antagomir-16. Ethidium bromide staining of tRNA is shown as a loading control.

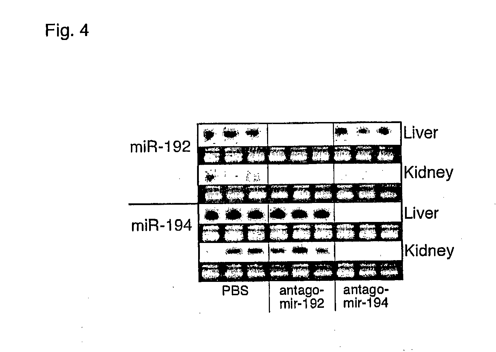

FIG. 4 includes a panel of Northern blots of total RNA isolated from livers of mice injected with antagomiR-122, mm-antagomir-122, or PBS. RNA was extracted 24 h after injection by a bDNA lysis method. Northern blots were probed with miR-122 and let7 microRNAs. Ethidium bromide staining of tRNA is shown as a loading control.
FIG 5. Ligand conjugated oligonucleotide to modulate expression of miRNA: (a) ligand of interest is conjugated to the oligonucleotide via a tether and linker; (b) ligand of interest is conjugated to the oligonucleotide via a linker without a tether or tether without an additional linker and (c) a ligand of interest is attached directly to the oligonucleotide.

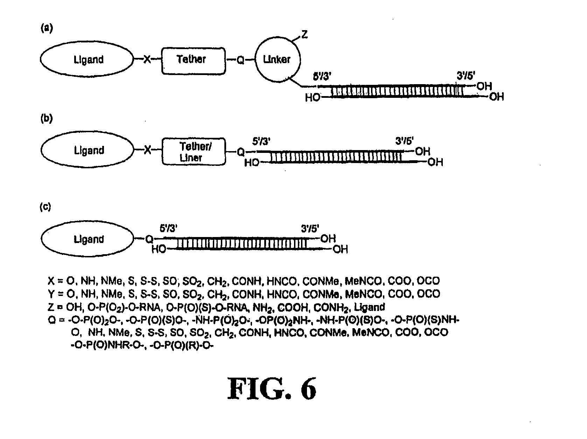

FIG 6. Ligand conjugated double stranded oligonucleotide to modulate expression of miRNA: (a) ligand of interest is conjugated to the oligonucleotide via a tether and linker; (b) ligand of interest is conjugated to the oligonucleotide via a linker without a tether or tether without an additional linker and (c) a ligand of interest is attached directly to the oligonucleotide.

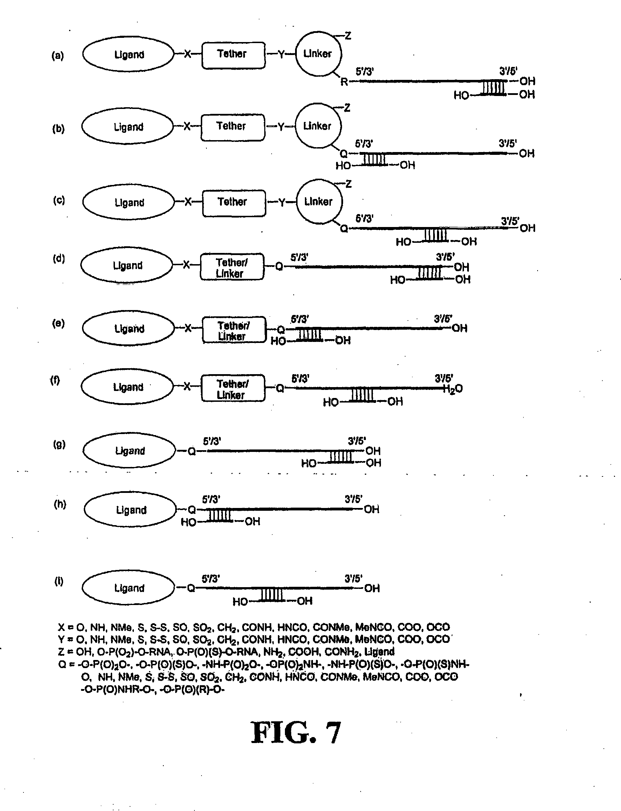

FIG 7. Ligand conjugated antisense strand comprising partially double stranded oligonucleotides to modulate expression of miRNA. (a-c) ligand of interest is conjugated to the oligonucleotide via a tether and linker; (d-f) ligand of interest is conjugated to the oligonucleotide via a linker without a tether or tether without an additional linker and (g-i) a ligand of interest is attached directly to the oligonucleotide.

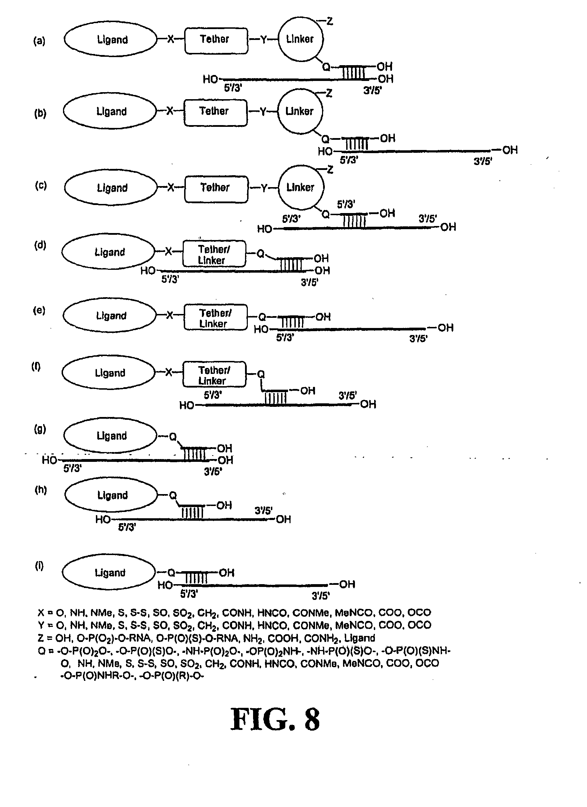

FIG 8. Ligand conjugated partial sense strand comprising partially double stranded oligonucleotides to modulate expression of miRNA. (a-c) ligand of interest is conjugated to the oligonucleotide via a tether and linker; (d-f) ligand of interest is conjugated to the oligonucleotide via a linker without a tether or tether without an additional linker and (g-i) a ligand of interest is attached directly to the oligonucleotide.

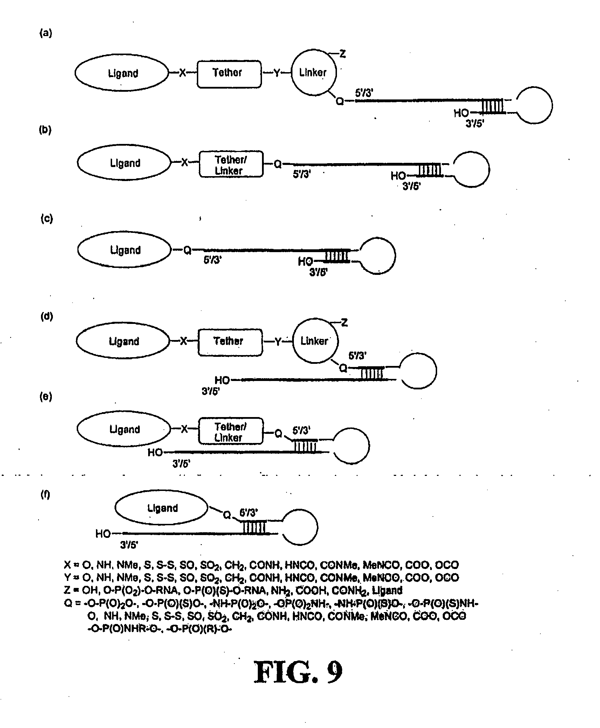

FIG 9. Ligand conjugated partial hairpin oligonucleotides to modulate expression of miRNA. (a-b) ligand of interest is conjugated to either 3' or 5' end of the hairpin via a tether and linker; (c-d) ligand of interest is conjugated to the hairpin via a linker without a tether or tether without an additional linker and (e-f) a ligand of interest is attached directly to the oligonucleotide. The hairpin is comprised of nucleotides or non-nucleotide linkages.

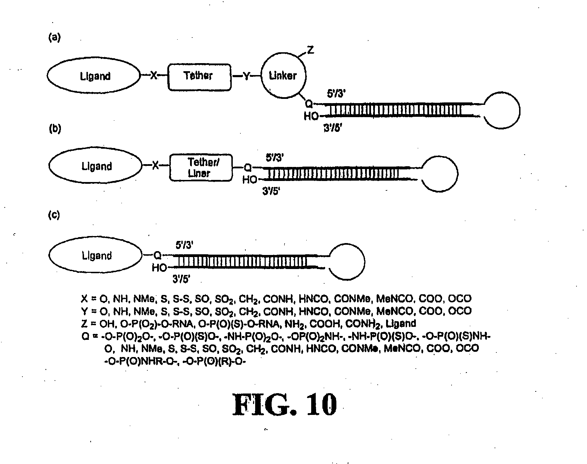

FIG 10. Ligand conjugated hairpin oligonucleotides to modulate expression of miRNA. (a) ligand of interest is conjugated to either 3' or 5' end of the hairpin via a tether and linker; (b) ligand of interest is conjugated to the hairpin via a linker without a tether or tether without an additional linker and (c) a ligand of interest is attached directly to the oligonucleotide. The hairpin is comprised of nucleotides or non-nucleotide linkages.

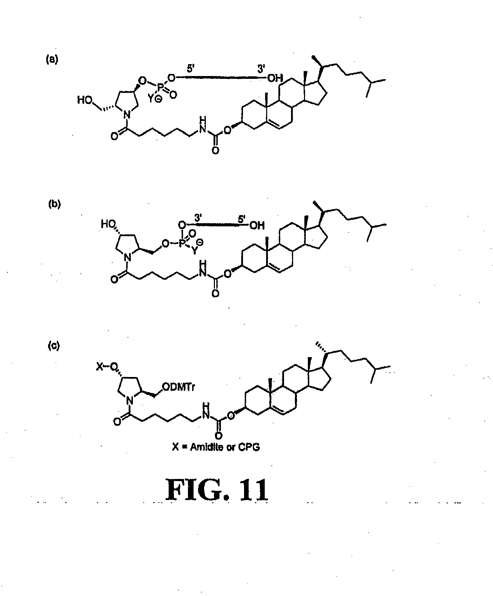

FIG. 11. Cholesterol conjugated oligonucleotides to modulate expression of miRNA. (a) 5' cholesterol conjugate; (b) 3' cholesterol conjugate and (c) cholesterol conjugate building blocks for oligonucleotide synthesis. The oligonucleotide can be miRNA, anti-miRNA, chemically modified RNA or DNA; DNA or DNA analogues for antisense application.

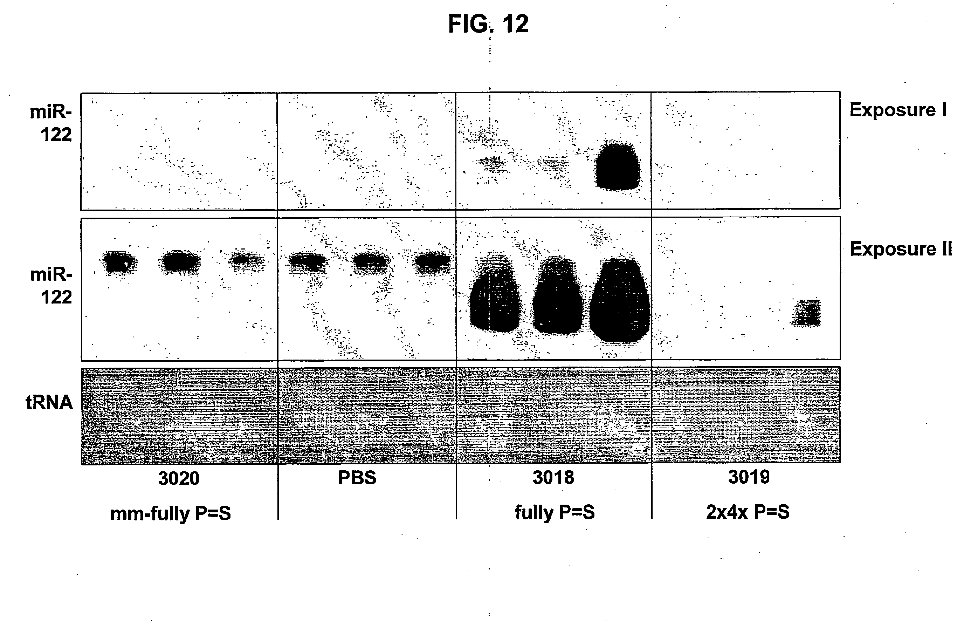

FIG 12. Activity of double-stranded antagomirs (see Table 2f for the description of agents used).

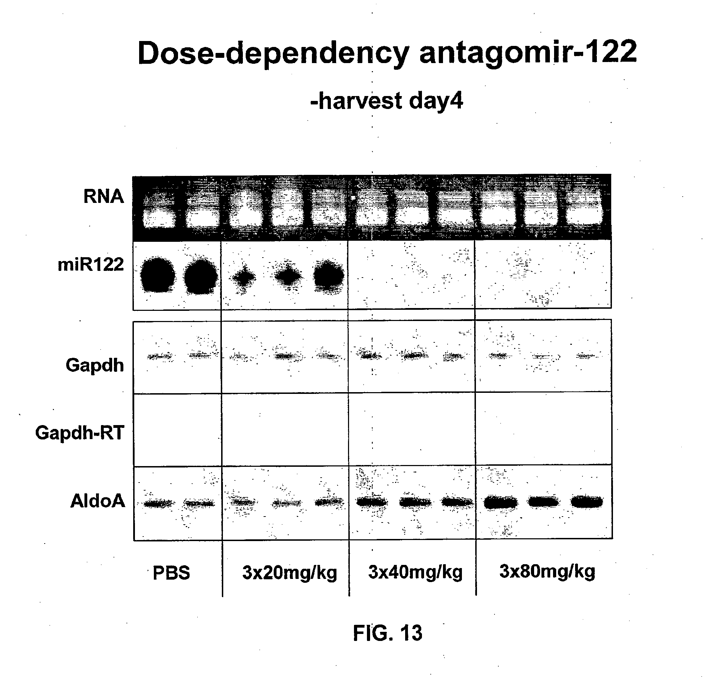

FIG 13. Dose response for antagomir-122.

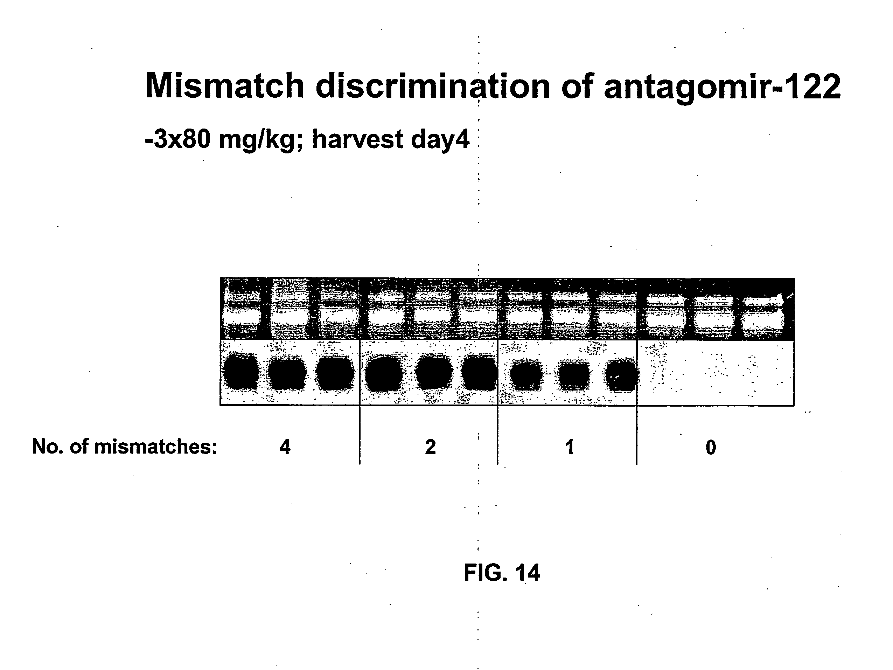

FIG 14. Mismatch control for antagomir-122.

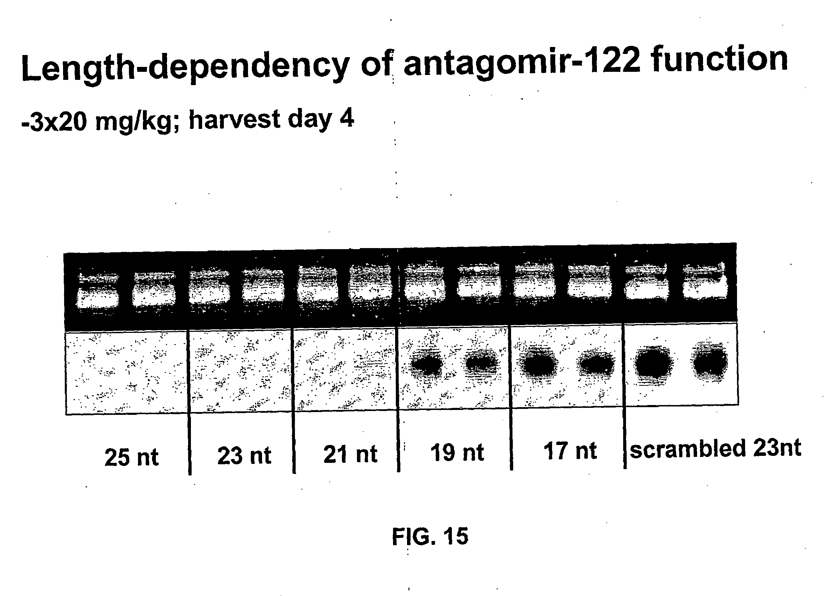

FIG 15. Length effect on activity of antagomir-122.

### DETAILED DESCRIPTION

The present invention is based in part on the discovery that expression of endogenous microRNAs (miRNAs) or pre-microRNAs (pre-miRNAs) can be inhibited by an antagomir, *e.g.*, through systemic administration of an antagomir, as well as by parenteral administration of such agents. Based on these findings, the present invention provides specific compositions and methods that are useful in reducing miRNA and pre-miRNA levels, in *e.g.*, a mammal, such as a human. In particular, the present invention provides specific compositions and methods that are useful for reducing levels of the miRNA miR-122, herein defined as antagomirs.

In one aspect, the invention features antagomirs. An antagomir is a single-stranded, double stranded, partially double stranded or hairpin structured chemically modified oligonucleotide agents that consisting of, consisting essentially of or comprising at least 12 or more contiguous nucleotides substantially complementary to an endogenous miRNA and more particularly agents that include 12 or more contiguous nucleotides substantially complementary to a target sequence of an miRNA or pre-miRNA nucleotide sequence. As used herein partially double stranded referes to double stranded structures that contain less nucleotides than the complementary strand. In general, such partial double stranded agents will have less than 75% double stranded structure, preferably less than 50%, and more preferably less than 25%, 20 % or 15% double stranded structure. Figures 5-11 provides repsresentative structures of antagomirs.

Preferably, an antagomir featured in the invention includes a nucleotide sequence sufficiently complementary to hybridize to an miRNA target sequence of about 12 to 25 nucleotides, preferably about 15 to 23 nucleotides. More preferably, the target sequence differs by no more than 1, 2, or 3 nucleotides from a sequence shown in Table 1, and in one embodiment, the antagomir is an agent shown in Table 2a-e and Table 4. In one embodiment, the antagomir includes a non-nucleotide moiety, *e.g.,* a cholesterol moiety. The non-nucleotide moiety can be attached, *e.g.,* to the 3' or 5' end of the oligonucleotide agent. In a preferred embodiment, a cholesterol moiety is attached to the 3' end of the oligonucleotide agent.

The antagomir antagomir is further stabilized against nucleolytic degradation such as by the incorporation of a modification, e.g., a nucleotide modification. The antagomir includes a phosphorothioate at at least the first, second, or third internucleotide linkage at the 5' or 3' end of the nucleotide sequence. In one embodiment, the antagomir includes a 2'-modified nucleotide, e.g., a 2'-deoxy, 2'-deoxy-2'-fluoro, 2'-O-methyl, 2'-O-methoxyethyl (2'-O-MOE), 2'-O-aminopropyl (2'-O-AP), 2'-O-dimethylaminoethyl (2'-O-DMAOE), 2'-O-dimethylaminopropyl (2'-O-DMAP), 2'-O-dimethylaminoethyloxyethyl (2'-O-DMAEOE), or 2'-O-N-methylacetamido (2'-O-NMA). In a particularly preferred embodiment, the antagomir includes at least one 2'-O-methyl-modified nucleotide, and in some embodiments, all of the nucleotides of the antagomir include a 2'-O-methyl modification.

The antagomir is further modified so as to be attached to a ligand that is selected to improve stability, distribution or cellular uptake of the agent, e.g., cholesterol. The oligonucleotide antagomir can further be in isolated form or can be part of a pharmaceutical composition used for the methods described herein, particularly as a pharmaceutical composition formulated for parental administration. The pharmaceutical compositions can contain one or more oligonucleotide agents, and in some embodiments, will contain two or more oligonucleotide agents, each one directed to a different miRNA.

An antagomir that is substantially complementary to a nucleotide sequence of an miRNA can be delivered to a cell or a human to inhibit or reduce the activity of an endogenous miRNA, such as when aberrant or undesired miRNA activity, or insufficient activity of a target mRNA that hybridizes to the endogenous miRNA, is linked to a disease or disorder. In one embodiment, an antagomir featured in the invention has a nucleotide sequence that is substantially complementary to miR-122 (see Table 1), which hybridizes to numerous RNAs, including aldolase A mRNA, N-myc downstram regulated gene (Ndrg3) mRNA, IQ motif containing GTPase activating protein-1 (Iqgap1) mRNA, HMG-CoA-reductase (Hmgcr) mRNA, and citrate synthase mRNA and others. In a preferred embodiment, the antagomirthat is substantially complementary to miR-122 is antagomir-122 (Table 2a-e and Table 4). Aldolase A deficiencies have been found to be associated with a variety of disorders, including hemolytic anemia, arthrogryposis complex congenita, pituitary ectopia, rhabdomyolysis, hyperkalemia. Humans suffering from aldolase A deficiencies also experience symptoms that include growth and developmental retardation, midfacial hypoplasia, hepatomegaly, as well as myopathic symptoms. Thus a human who has or who is diagnosed as having any of these disorders or symptoms is a candidate to receive treatment with an antagomir, such as a single-stranded oligonucleotide agent, that hybridizes to miR-122.

Also disclosed is an antagomir having a nucleotide sequence that is substantially complementary to miR-16, miR-192, or miR-194 (see Table 1).

In one embodiment, the antagomiris selected from those shown in Table 2a-e and Table 4. The single-stranded oligonucleotide agents of Table 2a-e and Table 4 are complementary to and hybridize to the corresponding miRNAs of Table 1.

**Table 1. Exemplary miRNAs identified in mus musculus**

| miRNA | Sequence | SEQ ID NO: |
|---|---|---|
| miR-122 | 5'-UGGAGUGUGACAAUGGUGUUUGU-3' | 1 |
| miR-16 | 5'-UAGCAGCACGUAAAUAUUGGCG-3' | 2 |
| miR-192 | 5'-CUGACCUAUGAAUUGACAGCC-3' | 3 |
| miR-194 | 5'-UGUAACAGCAACUCCAUGUGGA-3' | 4 |

**Table 2a. Oligonucleotide agents targeting mus musculus miRNAs**

| RNA | Sequence | SEQ ID NO: |
|---|---|---|
| antagomir-122 | 5'-aₛcₛaaacaccauugucacacuₛcₛcₛaₛ-Chol-3' | 5 |
| antagomir-16 | 5'-cₛgₛccaauauuuacgugcugₛcₛuₛaₛ-Chol-3' | 6 |
| antagomir-192 | 5'-gₛgₛcugucaauucauagguₛcₛaₛgₛ-Chol-3' | 7 |
| antagomir-194 | 5'-uₛcₛcacauggaguugcuguuₛaₛcₛaₛ-Chol-3' | 8 |

| | | |
|---|---|---|
| lower case letters represent 2'-O-methyl modified nucleotides; subscript 's' represents a phosphorothioate linkage; "Chol" indicates cholesterol conjugate | | |

**Table 2b. Double stranded oligonucleotides to modulate microRNAs**

| **Duplex ID** | **Sequence ID and sequence** |
|---|---|
| AL-DP-3018 | AL-SQ-3035: UGGAGUGUGACAAUGGUGUUUGU (SEQ ID NO:1) |
| | AL-SQ-3037: oAsoCsoAsoAsoAsoCsoAsoCsoCsoAsoUsoUsoGsoUsoCsoAsoCsoAsoCsoUsoCsoCsoAs-Chol (SEQ ID NO:10) |
| AL-DP-3019 | AL-SQ-3035: UGGAGUGUGACAAUGGUGUUUGU (SEQ ID NO:1) |
| | AL-SQ-3038: oAsoCsoAoAoAoCoAoCoCoAoUoUoGoUoCoAoCoAoCoUsoCsoCsoAs-Chol (SEQ ID NO:11) |
| AL-DP-3020 (mismatch) | AL-SQ-3036: UGGAAUGUGACAGUGUUGUGUGU (SEQ ID NO:12) |
| | AL-SQ-3039: oAsoCsoAsoCsoAsoCsoAsoAsoCsoAsoCsoUsoGsoUsoCsoAsoCsoAsoUsoUsoCsoCsoAs-Chol (SEQ ID NO:13) |
| AL-DP-3021 (mismatch) | AL-SQ-3036: UGGAAUGUGACAGUGUUGUGUGU (SEQ ID NO:12) |
| | AL-SQ-3040: oAsoCsoAoCoAoCoAoAoCoAoCoUoGoUoCoAoCoAoUoUsoCsoCsoAs-Chol (SEQ ID NO:14) |

| | |
|---|---|
| *Note:* oN represents 2'-*O*-Me ribo sugar modification, dN represents deoxyribo sugar modification and 's' stands for phosphorothioate linkage | |

**Table 2c. Partial double stranded and hairpin structured oligonucleotides to modulate microRNA-122**

| **Sequence ID** | **Sequence** |
|---|---|
| AL-SQ-3384 | oAoCoAoAoAoCoAoCoCoAoUoUoGoUoCoAoCoAoCoUoCoCoAdTdTdTdToUoGoGoAs-Chol (SEQ ID NO:15) |
| AL-SQ-3385 | oAoCoAoAoAoCoAoCoCoAoUoUoGoUoCoAoCoAoCoUoCoCoAsdTsdTsdTsdTsoUoGoGoAs-Chol (SEQ ID NO:16) |

| | |
|---|---|
| *Note:* oN represents 2'-*O*-Me ribo sugar modification, dN represents deoxyribo sugar modification and 's' stands for phosphorothioate linkage | |

**Table 2d. Partial double stranded oligonucleotides to modulate microRNA-122**

| **Duplex ID** | **Sequence ID and sequence** | **SEQ ID NO:** |
|---|---|---|
| AL-DP-3043 | AL-SQ-3038: oAsoCsoAoAoAoCoAoCoCoAoUoUoGoUoCoAoCoAoCoUsoCsoCsoAs-Chol | 11 |
| | AL-SQ-3400: oUoGoGoAoGoUoG (7-mer at the 3'-end) | 17 |
| AL-DP-3044 | AL-SQ-3038: oAsoCsoAoAoAoCoAoCoCoAoUoUoGoUoCoAoCoAoCoUsoCsoCsoAs-Chol | 11 |
| | AL-SQ-3401: oGoAoCoAoAoUoG (7-mer at nts 9-15) | 18 |
| AL-DP-3045 | AL-SQ-3040: oAsoCsoAoCoAoCoAoAoCoAoCoUoGoUoCoAoCoAoUoUsoCsoCsoAs-Chol | 14 |
| | AL-SQ-3402: oUoGoGoAoAoUoG (7-mer at the 3'-end) | 19 |
| AL-DP-3046 | AL-SQ-3040: oAsoCsoAoCoAoCoAoAoCoAoCoUoGoUoCoAoCoAoUoUsoCsoCsoAs-Chol | 14 |
| | AL-SQ-3403: oGoAoCoAoGoUoG (7-mer at nts 9-15) | 20 |

| | | |
|---|---|---|
| *Note:* oN represents 2'-*O*-Me ribo sugar modification, dN represents deoxyribo sugar modification and 's' stands for phosphorothioate linkage | | |

**Table 2e. Single stranded oligonucleotides to modulate microRNAs**

| **Sequence ID** | **Sequence** | **SEQ ID NO:** |
|---|---|---|
| AL-SQ-3035 | UGGAGUGUGACAAUGGUGUUUGU | 1 |
| AL-SQ-3036 | UGGAAUGUGACAGUGUUGUGUGU | 12 |
| AL-SQ-3037 | oAsoCsoAsoAsoAsoCsoAsoCsoCsoAsoUsoUsoGsoUsoCsoAsoCsoAsoCsoUsoCsoCsoAs-Chol | 10 |
| AL-SQ-3038 | oAsoCsoA oAoAoC oAoCoC oAoUoU oGoUoC oAoCoA oCoUsoCs oCsoAs-chol | 11 |
| AL-SQ-3039 | oAsoCsoAsoCsoAsoCsoAsoAsoCsoAsoCsoUsoGsoUsoCsoAsoCsoAsoUsoUsoCsoCsoAs-Chol | 13 |
| AL-SQ-3040 | oAsoCsoA oCoAoC oAoAoC oAoCoU oGoUoC oAoCoA oUoUsoCs oCsoAs-chol | 14 |
| AL-SQ-3223 | oUsoGsoG oAoGoU oGoUoG oAoCoA oAoUoG oGoUoG oUoUsoUs oGsoUs-chol | 21 |
| AL-SQ-3224 | oUsoGsoG oAoAoU oGoUoG oAoCoA oGoUoG oUoUoG oUoGsoUs oGsoUs-chol | 22 |
| AL-SQ-3225 | oAsoCsoAs oAsoAsoCs oAsoCsoCs oAsoUsoUs oGsoUsoCs oAsoCsoAs oCsoUsoCs oCsoA | 23 |
| AL-SQ-3226 | oAsoCsoA oAoAoC oAoCoC oAoUoU oGoUoC oAoCoA oC*oU*oC* oC*oA | 24 |
| AL-SQ-3227 | oCsoGsoC oCoAoA oUoAoU oUoUoA oCoGoU oGoCoU oG*oC*oU* oA*-chol | 25 |
| AL-SQ-3228 | oGsoGsoC oUoGoU oCoAoA oUoUoC oAoUoA oGoGoU* oC*oA*oG*-chol | 26 |
| AL-SQ-3229 | oUsoCsoC oAoCoA oUoGoG oAoGoU oUoGoC oUoGoU oU*oA*oC* oA*-chol | 27 |
| AL-SQ-3230 | oUsoCsoA oCoGoC oGoAoG oCoCoG oAoAoC oGoAoA oCsoAsoAs oAs-chol | 28 |
| AL-SQ-3344 | UGGIGUGUGICIIUGGUGUUUGU | 29 |
| AL-SQ-3350 | oAoCoAoAoAoCoAoCoCoAoUoUoGoUoCoAoCoAoCoUoCoCoA-Chol | 30 |
| AL-SQ-3351 | oCsoAsoCoAoAoAoCoAoCoCoAoUoUoGoUoCoAoCoAoCoUoCsoCsoAsoCs-Chol | 31 |
| AL-SQ-3352 | oCsoAsoAoAoCoAoCoCoAoUoUoGoUoCoAoCoAoCsoUsoCsoCs-Chol - | 32 |
| AL-SQ-3353 | oAsoAsoAoCoAoCoCoAoUoUoGoUoCoAoCoAsoCsoUsoCs-Chol | 33 |
| AL-SQ-3354 | oAsoAsoCoAoCoCoAoUoUoGoUoCoAoCsoAsoCsoUs-Chol | 34 |
| AL-SQ-3355 | oAsoCsoAoAoAoCoAoAoCoAoCoUoGoUoCoAoCoAoUoUsoCsoCsoAs-Chol | 35 |
| AL-SQ-3356 | oAsoCsoAoAoAoCoAoCoCoAoCoUoGoUoCoAoCoAoUoUsoCsoCsoAs-Chol | 36 |
| AL-SQ-3357 | oAsoCsoAoAoAoCoAoCoCoAoUoUoGoUoCoAoCoAoUoUsoCsoCsoAs-Chol | 37 |
| AL-SQ-3358 | Cy-5-soAsoCoAoAoAoCoAoCoCoAoUoUoGoUoCoAoCoAoCoUsoCsoCsoAs-Chol | 38 |
| AL-SQ-3359 | Cy-3-soAsoCoAoCoAoCoAoAoCoAoCoUoGoUoCoAoCoAoUoUsoCsoCsoAs-Chol | 39 |

| | | |
|---|---|---|
| Cy-5 and Cy-3 are dyes used for localization studies. | | |

**Table 2f. Description of sequences listed in Table 2b-2e**

| **Sequence #** | **Description** |
|---|---|
| AL-SQ-3035 | complimentary to antagomir-122 |
| AL-SQ-3036 | complimentary to mm-antagomir-122 |
| AL-SQ-3037 | antagomir-122-fullyPS |
| AL-SQ-3038 | antagomir-122 |
| AL-SQ-3039 | mm-antagomir-122-fullyPS |
| AL-SQ-3040 | mm-antagomir-122 |
| AL-SQ-3223 | complementary to antagomir-122 |
| AL-SQ-3224 | complementary to mm-antagomir-122 |
| AL-SQ-3225 | anti-122fs |
| AL-SQ-3226 | anti-122ps |
| AL-SQ-3227 | antagomir-16 |
| AL-SQ-3228 | antagomir-192 |
| AL-SQ-3229 | antagomir-194 |
| AL-SQ-3230 | antagomir-375 |
| AL-SQ-3344 | complementary to antagomir-122 with A->I modification |
| AL-SQ-3350 | antagomir-122-noPS |
| AL-SQ-3351 | antagomir-122-25mer |
| AL-SQ-3352 | antagomir-122-21mer |
| AL-SQ-3353 | antagomir-122-19mer |
| AL-SQ-3354 | antagomir-122-17mer |
| AL-SQ-3355 | mismatch-antagomir-122-3mm |
| AL-SQ-3356 | mismatch -antagomir-122-2mm |
| AL-SQ-3357 | mismatch -antagomir-122-1mm |
| AL-SQ-3358 | antagomir-122-5'-Cy5 |
| AL-SQ-3359 | antagomir-122-5'-Cy3_ |
| AL-SQ-3400 | 7-mer complementary to 3'-end of antagomir-122 |
| AL-SQ-3401 | 7-mer complementary to nucleotides 9-15 of antagomir-122 |
| AL-SQ-3402 | 7-mer complementary to 3'-end of mismatch -antagomir-122 |
| AL-SQ-3403 | 7-mer complementary to nucleotides 9-15 of mismatch-antagomir-122 |

In one aspect, the invention features an antagomir, such as a single-stranded oligonucleotide agent, that includes a nucleotide sequence that is substantially identical to a nucleotide sequence of an miRNA, such as miR-122 listed in Table 1. An oligonucleotide sequence that is substantially identical to an endogenous miRNA sequence is 70%, 80%, 90%, or more identical to the endogenous miRNA sequence. Preferably, the agent is identical in sequence with an endogenous miRNA. An antagomir that is substantially identical to a nucleotide sequence of an miRNA can be delivered to a cell or a human to replace or supplement the activity of an endogenous miRNA, such as when an miRNA deficiency is linked to a disease or disorder, or aberrant or unwanted expression of the mRNA that is the target of the endogenous miRNA is linked to a disease or disorder. In one embodiment, an antagomir agent featured in the invention can have a nucleotide sequence that is substantially identical to miR-122 (see Table 1). An miR-122 binds to numerous RNAs including aldolase A mRNA, which has been shown to be overexpressed in different cancers, including lung cancer and breast cancer, and is overexpressed in adenocarcinomas of various different tissues origins. Thus a single stranded antagomir that is substantially identical to miR-122 can be administered as a therapeutic composition to a subject having or at risk for developing lung cancer or breast cancer, for example.

An miR-122 binds other mRNAs, including N-myc downstram regulated gene (Ndrg3) mRNA, IQ motif containing GTPase activating protein-1 (Iqgap1) mRNA, HMG-CoA-reductase (Hmgcr) mRNA, and citrate synthase mRNA. Iqgap1 overexpression is associated with gastric cancer and colorectal cancer. Thus a single stranded antagomir that is substantially identical to miR-122 can be useful for downregulating Iqgap1 expression, and can be administered as a therapeutic composition to a subject having or at risk for developing gastric cancer and colorectal cancer. Hmgcr inhibitors are useful to treat hyperglycemia and to reduce the risk of stroke and bone fractures. Thus a single stranded antagomir that is substantially identical to miR-122 can be useful for downregulating Hmgcr expression, and can be administered as a therapeutic composition to a subject having or at risk for developing hyperglycemia, stroke, or a bone fracture. A single stranded antagomir that is substantially identical to miR-122 can be administered as a therapeutic composition to a subject having or at risk for developing a disorder characterized by the aberrant or unwanted expression of any of these genes, or any other gene downregulated by miR-122.

An antagomir, such as a single-stranded oligonucleotide agent, can have a nucleotide sequence that is substantially identical to miR-16, miR-192, or miR-194. Single-stranded oligonucleotide agents that are substantially identical to at least a portion of an miRNA, such as those described above, can be administered to a subject to treat the disease or disorder associated with the downregulation of an endogenous miRNA, or the aberrant or unwanted expression of an mRNA target of the endogenous miRNA.

In one aspect, the invention features a method of reducing the levels of an miRNA or pre-miRNA in a cell of a subject, e.g., a human subject. The method includes the step of administering an antagomir to the subject, where the antagomir is substantially single-stranded and includes a sequence that is substantially complementary to 12 to 23 contiguous nucleotides, and preferably 15 to 23 contiguous nucleotides, of a target sequence of an miRNA or pre-miRNA nucleotide sequence. Preferably, the target sequence differs by no more than 1, 2, or 3 nucleotides from a microRNA or pre-microRNA sequence, such as a miR-122 sequence shown in Table 1.

In one embodiment, the methods featured in the invention are useful for reducing the level of an endogenous miRNA (miR-122 ) or pre-miRNA in a cell, e.g, in a cell of a subject, such as a human subject. Such methods include contacting the cell with an antagomir, such as a single-stranded oligonucleotide agent, described herein for a time sufficient to allow uptake of the antagomir into the cell.

In another aspect, the invention features a method of making an antagomir, such as a single-stranded oligonucleotide agent, described herein. In one embodiment, the method includes synthesizing an oligonucleotide agent, including incorporating a nucleotide modification that stabilizes the antagomir against nucleolytic degradation.

In another aspect, the invention features a pharmaceutical composition including an antagomir, such as a single-stranded oligonucleotide agent, described herein, and a pharmaceutically acceptable carrier. In a preferred embodiment, the antagomir, such as a single-stranded oligonucleotide agent,included in the pharmaceutical composition hybridizes to miR-122.

In another aspect the invention features a method of inhibiting miRNA expression (miR-122, expression) or pre-miRNA expression in a cell, *e.g.*, a cell of a subject. The method includes contacting the cell with an effective amount of an antagomir, such as a single-stranded oligonucleotide agent, described herein, which is substantially complementary to the nucleotide sequence of the target miRNA or the target pre-miRNA. Such methods can be performed on a mammalian subject by administering to a subject one of the oligonucleotide agents/pharmaceutical compositions described herein.

In another aspect the invention features a method of increasing levels of an RNA or protein that are encoded by a gene whose expression is down-regulated by an miRNA, e.g., an endogenous miRNA, such as miR-122.

The method includes contacting the cell with an effective amount of an antagomir, such as a single-stranded oligonucleotide agent, described herein, which is substantially complementary to the nucleotide sequence of the miRNA that binds to and effectively inhibits translation of the RNA transcribed from the gene. For example, the invention features a method of increasing aldolase A protein levels in a cell. Similarly, the invention features a method of increasing Ndrg3, Iqgap1, Hmgcr, and/or citrate synthase protein levels in a cell. The methods include contacting the cell with an effective amount of an antagomir described herein (e.g., antagomir-122, described in Table 2a-e and Table 4), which is substantially complementary to the nucleotide sequence of miR-122 (see Table 1).

Preferably, an antagomir, such as a single-stranded oligonucleotide agent, (a term which is defined below) will include a ligand that is selected to improve stability, distribution or cellular uptake of the agent. Compositions featured in the invention can include conjugated single-stranded oligonucleotide agents as well as conjugated monomers that are the components of or can be used to make the conjugated oligonucleotide agents. The conjugated oligonucleotide agents can modify gene expression by targeting and binding to a nucleic acid, such as an miRNA (miR-122) or pre-miRNA.

In a preferred embodiment, the ligand is a lipophilic moiety, e.g., cholesterol, which enhances entry of the antagomir, such as a single-stranded oligonucleotide agent,into a cell, such as a hepatocyte, synoviocyte, myocyte, keratinocyte, leukocyte, endothelial cell *(e.g.,* a kidney cell), B-cell, T-cell, epithelial cell, mesodermal cell, mycloid cell, neural cell, neoplastic cell, mast cell, or fibroblast cell. In some embodiments, a myocyte is a smooth muscle cell or a cardiac myocyte. A fibroblast cell can be a dermal fibroblast, and a leukocyte can be a monocyte. In another embodiment, the cell is from an adherent tumor cell line derived from a tissue, such as bladder, lung, breast, cervix, colon, pancreas, prostate, kidney, liver, skin, or nervous system (e.g., central nervous system).

In another aspect, the invention provides methods of increasing expression of a target gene by providing an antagomir to which a lipophilic moiety is conjugated, e.g., a lipophilic conjugated antagomir described herein, to a cell. The antagomir preferably hybridizes to an miRNA (miR-122) or a pre-miRNA. In a preferred embodiment the conjugated antagomir can be used to increase expression of a target gene in an organism, *e.g.,* a mammal, *e.g.,* a human, or to increase expression of a target gene in a cell line or in cells which are outside an organism. An mRNA transcribed from the target gene hybridizes to an endogenous miRNA, which consequently results in downregulation of mRNA expression. An antagomir, such as a single-stranded oligonucleotide agent, featured in the invention hybridizes to the endogenous miRNA and consequently causes an increase in mRNA expression. In the case of a whole organism, the method can be used to increase expression of a gene and treat a condition associated with a low level of expression of the gene. For example, an antagomir, such as a single-stranded oligonucleotide agent, that targets miR-122 (e.g., antagomir-122) can be used to increase expression of an aldolase A gene to treat a subject having, or at risk for developing, hemolytic anemia, arthrogryposis complex congenita, pituitary ectopia, rhabdomyolysis, hyperkalemia, or any other disorder associated with aldolase A deficiency. Administration of an antagomir, such as a single-stranded oligonucleotide agent, that targets miR-122 (e.g., antagomir-122) can be also be used to increase expression of an Ndrg3, Iqgap1, Hmgcr, or citrate synthase gene to treat a subject having, or at risk for developing, a disorder associated with a decreased expression of any one of these genes.

In one embodiment, the antagomir, such as a single-stranded oligonucleotide agent,to which a lipophilic moiety is conjugated is used to increase expression of a gene in a cell that is not part of a whole organism, such as when the cell is part of a primary cell line, secondary cell line, tumor cell line, or transformed or immortalized cell line. Cells that are not part of a whole organism can be used in an initial screen to effective in increasing target gene expression levels, or decreasing levels of a target miRNA or pre-miRNA. A test in cells that are not part of a whole organism can be followed by test of the antagomir in a whole animal. In some embodiments, the antagomir that is conjugated to a lipophilic moiety is administered to an organism, or contacted with a cell that is not part of an organism, in the absence of (or in a reduced amount of) other reagents that facilitate or enhance delivery, e.g., a compound which enhances transit through the cell membrane. (A reduced amount can be an amount of such reagent which is reduced in comparison to what would be needed to get an equal amount of nonconjugated antagomir into the target cell). For example, the antagomir that is conjugated to a lipophilic moiety is administered to an organism, or contacted with a cell that is not part of an organism, in the absence (or reduced amount) of (i) an additional lipophilic moiety; (ii) a transfection agent (e.g., an ion or other substance which substantially alters cell permeability to an oligonucleotide agent); or (iii) a commercial transfecting agent such as Lipofectamine™ (Invitrogen, Carlsbad, CA), Lipofectamine 2000™, TransIT-TKO™ (Mirus, Madison, WI), FuGENE 6 (Roche, Indianapolis, IN), polyethylenimine, X-tremeGENE Q2 (Roche, Indianapolis, IN), DOTAP, DOSPER, Metafectene™ (Biontex, Munich, Germany), and the like.

Cationic lipid particles have been used to encapsulate oligonucleotide reagents. For e.g. Cationic lipid saturation influences intracellular delivery of encapsulated nucleic acids. Heyes, James; Palmer, Lorne; Bremner, Kaz; MacLachlan, Ian., Journal of Controlled Release (2005), 107(2), 276-287.

An analogous series of cationic lipids (1,2-distearyloxy-N,N-dimethyl-3-aminopropane (DSDMA), 1,2-dioleyloxy-N,N-dimethyl-3-aminopropane (DODMA), 1,2-dilinoleyloxy-N,N-dimethyl-3-aminopropane (DLinDMA) and 1,2-dilinolenyloxy-N,N-dimethyl-3-aminopropane (DLenDMA)) possessing 0, 1, 2 or 3 double bonds per alkyl chain resp., was synthesized to det. the correlation between lipid satn., fusogenicity and efficiency of intracellular nucleic acid delivery. 31P-NMR anal. suggests that as satn. increases, from 2 to 0 double bonds, lamellar (Lα) to reversed hexagonal (HII) phase transition temp. increases, indicating decreasing fusogenicity. This trend is largely reflected by the efficiency of gene silencing obsd. in vitro when the lipids are formulated as Stable Nucleic Acid Lipid Particles (SNALPs) encapsulating small inhibitory RNA (siRNA). Uptake expts. suggest that despite their lower gene silencing efficiency, the less fusogenic particles are more readily internalized by cells. Microscopic visualization of fluorescently labeled siRNA uptake was supported by quant. data acquired using radiolabeled prepns. Since electrostatic binding is a precursor to uptake, the pKa of each cationic lipid was detd. The results support a transfection model in which endosomal release, mediated by fusion with the endosomal membrane, results in cytoplasmic translocation of the nucleic acid payload.

In a preferred embodiment, the antagomir is suitable for delivery to a cell in *vivo, e.g.*, to a cell in an organism. In another embodiment, the antagomir is suitable for delivery to a cell *in vitro, e.g.*, to a cell in a cell line.

An antagomir to which a lipophilic moiety is attached can target any miRNA (e.g., miR-122, miR-16, miR-192, or miR-194) or pre-miRNA described herein and can be delivered to any cell type described herein, e.g., a cell type in an organism, tissue, or cell line. Delivery of the antagomir can be *in vivo, e.g.,* to a cell in an organism, or *in vitro, e.g.,* to a cell in a cell line.

In another aspect, the invention provides compositions including single-stranded oligonucleotide agents described herein, and in particular, compositions including an antagomir to which a lipophilic moiety is conjugated, e.g., a lipophilic conjugated antagomir that hybridizes to miR-122. In a preferred embodiment the composition is a pharmaceutically acceptable composition.

In one embodiment the composition is suitable for delivery to a cell *in vivo, e.g.,* to a cell in an organism. In another aspect, the antagomir is suitable for delivery to a cell *in vitro, e.g.,* to a cell in a cell line.

An "antagomir" or "oligonucleotide agent" of the present invention referes to a single stranded, double stranded or partially double stranded oligomer or polymer of ribonucleic acid (RNA) or deoxyribonucleic acid (DNA) or both or modifications thereof, which is antisense with respect to its target. This term includes oligonucleotides composed of naturally-occurring nucleobases, sugars and covalent internucleoside (backbone) linkages and which contain at least one non-naturally-occurring portions which function similarly. Such modified or substituted oligonucleotides are preferred over native forms because of desirable properties such as, for example, enhanced cellular uptake, enhanced affinity for nucleic acid target and increased stability in the presence of nucleases. In a preferred embodiment, the antagomir does not include a sense strand, and in another preferred embodiment, the antagomir does not self-hybridize to a significant extent. An antagomir featured in the invention can have secondary structure, but it is substantially single-stranded under physiological conditions. An antagomir that is substantially single-stranded is single-stranded to the extent that less than about 50% *(e.g.,* less than about 40%, 30%, 20%, 10%, or 5%) of the antagomir is duplexed with itself. Figures 5-11 provides repsresentative structures of antagomirs.

"Substantially complementary" means that two sequences are substantially complementary that a duplex can be formed between them. The duplex may have one or more mismatches but the region of duplex formation is sufficient to down-regulate expression of the target nucleic acid. The region of substantial complementarity can be perfectly paired. In other embodiments, there will be nucleotide mismatches in the region of substantial complementarity. In a preferred embodiment, the region of substantial complementarity will have no more than 1, 2, 3, 4, or 5 mismatches.

The antagomirs featured in the invention include oligomers or polymers of ribonucleic acid (RNA) or deoxyribonucleic acid (DNA) or both or modifications thereof. This term includes oligonucleotides composed of naturally occurring nucleobases, sugars, and covalent internucleoside (backbone) linkages as well as oligonucleotides having non-naturally-occurring portions that function similarly. Such modified or substituted oligonucleotides are often preferred over native forms because of desirable properties such as, for example, enhanced cellular uptake, enhanced affinity for nucleic acid target, and/or increased stability in the presence of nucleases. The oligonucleotide agents can be about 12 to about 30 nucleotides long, *e.g.,* about 15 to about 25, or about 18 to about 25 nucleotides long *(e.g.,* about 19,20, 21,22,23,24 nucleotides long).

The antagomirs featured in the invention can target RNA, e.g., an endogenous pre-miRNA or miRNA of the subject or an endogenous pre-miRNA or miRNA of a pathogen of the subject. For example, the oligonucleotide agents can target an miRNA of the subject, such as miR-122. Such single-stranded oligonucleotide can be useful for the treatment of diseases involving biological processes that are regulated by miRNAs, including developmental timing, differentiation, apoptosis, cell proliferation, organ development, and metabolism.

### MicroRNA-type Oligonucleotide Agents

The antagomir featured in the invention include microRNA-type (miRNA-type) oligonucleotide agents, e.g., the miRNA-type oligonucleotide agents listed in Table 2a-f. MicroRNAs are small noncoding RNA molecules that are capable of causing post-transcriptional silencing of specific genes in cells such as by the inhibition of translation or through degradation of the targeted mRNA. An miRNA can be completely complementary or can have a region of noncomplementarity with a target nucleic acid, consequently resulting in a "bulge" at the region of noncomplementarity. The region of noncomplementarity (the bulge) can be flanked by regions of sufficient complementarity, preferably complete complementarity to allow duplex formation. Preferably, the regions of complementarity are at least 8, 9, or 10 nucleotides long. An miRNA can inhibit gene expression by repressing translation, such as when the microRNA is not completely complementary to the target nucleic acid, or by causing target RNA degradation, which is believed to occur only when the miRNA binds its target with perfect complementarity. The invention also can include double-stranded precursors of miRNAs that may or may not form a bulge when bound to their targets.

An miRNA or pre-miRNA can be 18-100 nucleotides in length, and more preferably from 18-80 nucleotides in length. Mature miRNAs can have a length of 19-30 nucleotides, preferably 21-25 nucleotides, particularly 21, 22, 23, 24, or 25 nucleotides. MicroRNA precursors typically have a length of about 70-100 nucleotides and have a hairpin conformation. MicroRNAs are generated *in vivo* from pre-miRNAs by the enzymes Dicer and Drosha, which specifically process long pre-miRNA into functional miRNA. The miRNA-type oligonucleotide agents, or pre-miRNA-type oligonucleotide agents featured in the invention can be synthesized *in vivo* by a cell-based system or in vitro by chemical synthesis. MicroRNA-type oligonucleotide agents can be synthesized to include a modification that imparts a desired characteristic. For example, the modification can improve stability, hybridization thermodynamics with a target nucleic acid, targeting to a particular tissue or cell-type, or cell permeability, e.g., by an endocytosis-dependent or-independent mechanism. Modifications can also increase sequence specificity, and consequently decrease off-site targeting. Methods of synthesis and chemical modifications are described in greater detail below.

Given a sense strand sequence (e.g., the sequence of a sense strand of a cDNA molecule), an miRNA-type antagomir can be designed according to the rules of Watson and Crick base pairing. The miRNA-type antagomir can be complementary to a portion of an RNA, e.g., an miRNA, pre-miRNA, or mRNA. For example, the miRNA-type antagomir can be complementary to an miRNA endogenous to a cell, such as miR-122, miR-16, miR-192, or miR-194. An miRNA-type antagomir can be, for example, from about 12 to 30 nucleotides in length, preferably about 15 to 28 nucleotides in length (e.g., 16, 17, 18, 19, 20, 21, 22, 23, 24, 25, 26, or 27 nucleotides in length).

In particular, an miRNA-type antagomir featured in the invention can have a chemical modification on a nucleotide in an internal (*i.e.,* non-terminal) region having noncomplementarity with the target nucleic acid. For example, a modified nucleotide can be incorporated into the region of an miRNA that forms a bulge. The modification can include a ligand attached to the miRNA, e.g., by a linker. The modification can, for example, improve pharmacokinetics or stability of a therapeutic miRNA-type oligonucleotide agent, or improve hybridization properties (e.g., hybridization thermodynamics) of the miRNA-type antagomir to a target nucleic acid. In some embodiments, it is preferred that the orientation of a modification or ligand incorporated into or tethered to the bulge region of an miRNA-type antagomir is oriented to occupy the space in the bulge region. For example, the modification can include a modified base or sugar on the nucleic acid strand or a ligand that functions as an intercalator. These are preferably located in the bulge. The intercalator can be an aromatic, e.g., a polycyclic aromatic or heterocyclic aromatic compound. A polycyclic intercalator can have stacking capabilities, and can include systems with 2, 3, or 4 fused rings. The universal bases described below can be incorporated into the miRNA-type oligonucleotide agents. In some embodiments, it is preferred that the orientation of a modification or ligand incorporated into or tethered to the bulge region of an miRNA-type antagomir is oriented to occupy the space in the bulge region. This orientation facilitates the improved hybridization properties or an otherwise desired characteristic of the miRNA-type oligonucleotide agent.

In one embodiment, an miRNA-type antagomir or a pre-miRNA can include an aminoglycoside ligand, which can cause the miRNA-type antagomir to have improved hybridization properties or improved sequence specificity. Exemplary aminoglycosides include glycosylated polylysine; galactosylated polylysine; neomycin B; tobramycin; kanamycin A; and acridine conjugates of aminoglycosides, such as Neo-N-acridine, Neo-S-acridine, Neo-C-acridine, Tobra-N-acridine, and KanaA-N-acridine. Use of an acridine analog can increase sequence specificity. For example, neomycin B has a high affinity for RNA as compared to DNA, but low sequence-specificity. In some embodiments the guanidine analog (the guanidinoglycoside) of an aminoglycoside ligand is tethered to an oligonucleotide agent. In a guanidinoglycoside, the amine group on the amino acid is exchanged for a guanidine group. Attachment of a guanidine analog can enhance cell permeability of an oligonucleotide agent.

In one embodiment, the ligand can include a cleaving group that contributes to target gene inhibition by cleavage of the target nucleic acid. Preferably, the cleaving group is tethered to the miRNA-type antagomir in a manner such that it is positioned in the bulge region, where it can access and cleave the target RNA. The cleaving group can be, for example, a bleomycin (e.g., bleomycin-A₅, bleomycin-A₂, or bleomycin-B₂), pyrene, phenanthroline (e.g., *O*-phenanthroline), a polyamine, a tripeptide (e.g., lys-tyr-lys tripeptide), or metal ion chelating group. The metal ion chelating group can include, e.g., an Lu(III) or EU(III) macrocyclic complex, a Zn(II) 2,9-dimethylphenanthroline derivative, a Cu(II) terpyridine, or acridine, which can promote the selective cleavage of target RNA at the site of the bulge by free metal ions, such as Lu(III). In some embodiments, a peptide ligand can be tethered to an miRNA or a pre-miRNA to promote cleavage of the target RNA, e.g., at the bulge region. For example, 1,8-dimethyl-1,3,6,8,10,13-hexaazacyclotetradecane (cyclam) can be conjugated to a peptide (e.g., by an amino acid derivative) to promote target RNA cleavage. The methods and compositions featured in the invention include miRNA-type oligonucleotide agents that inhibit target gene expression by a cleavage or non-cleavage dependent mechanism.

An miRNA-type antagomir or pre-miRNA-type antagomir can be designed and synthesized to include a region of noncomplementarity (e.g., a region that is 3, 4, 5, or 6 nucleotides long) flanked by regions of sufficient complementarity to form a duplex (e.g., regions that are 7, 8, 9, 10, or 11 nucleotides long) with a target RNA, e.g., an miRNA, such as miR-122, miR-16, miR-192, or miR-194.

For increased nuclease resistance and/or binding affinity to the target, the single-stranded oligonucleotide agents featured in the invention can include 2'-O-methyl, 2'-fluorine, 2'-O-methoxyethyl, 2'-O-aminopropyl, 2'-amino, and/or phosphorothioate linkages. Inclusion of locked nucleic acids (LNA), ethylene nucleic acids (ENA), *e.g.,* 2'-4'-ethylene-bridged nucleic acids, and certain nucleobase modifications such as 2-amino-A, 2-thio (e.g., 2-thio-U), G-clamp modifications, can also increase binding affinity to the target. The inclusion of pyranose sugars in the oligonucleotide backbone can also decrease endonucleolytic cleavage. An antagomir can be further modified by including a 3' cationic group, or by inverting the nucleoside at the 3'-terminus with a 3'-3' linkage. In another alternative, the 3'-terminus can be blocked with an aminoalkyl group, *e.g.,* a 3' C5-aminoalkyl dT. Other 3' conjugates can inhibit 3'-5' exonucleolytic cleavage. While not being bound by theory, a 3' conjugate, such as naproxen or ibuprofen, may inhibit exonucleolytic cleavage by sterically blocking the exonuclease from binding to the 3' end of the oligonucleotide. Even small alkyl chains, aryl groups, or heterocyclic conjugates or modified sugars (D-ribose, deoxyribose, glucose etc.) can block 3'-5'-exonucleases.

The 5' -terminus can be blocked with an aminoalkyl group, e.g., a 5'-O-alkylamino substituent. Other 5' conjugates can inhibit 5'-3' exonucleolytic cleavage. While not being bound by theory, a 5' conjugate, such as naproxen or ibuprofen, may inhibit exonucleolytic cleavage by sterically blocking the exonuclease from binding to the 5' end of the oligonucleotide. Even small alkyl chains, aryl groups, or heterocyclic conjugates or modified sugars (D-ribose, deoxyribose, glucose etc.) can block 3'-5'-exonucleases.

In one embodiment, an antagomir, such as a single-stranded oligonucleotide agent, includes a modification that improves targeting, e.g. a targeting modification described herein. Examples of modifications that target single-stranded oligonucleotide agents to particular cell types include carbohydrate sugars such as galactose, N-acetylgalactosamine, mannose; vitamins such as folates; other ligands such as RGDs and RGD mimics; and small molecules including naproxen, ibuprofen or other known protein-binding molecules.

An antagomir, such as a single-stranded oligonucleotide agent, featured in the invention can be constructed using chemical synthesis and/or enzymatic ligation reactions using procedures known in the art. For example, an antagomir can be chemically synthesized using naturally occurring nucleotides or variously modified nucleotides designed to increase the biological stability of the molecules or to increase the physical stability of the duplex formed between the antagomir and target nucleic acids, *e.g.,* phosphorothioate derivatives and acridine substituted nucleotides can be used. Other appropriate nucleic acid modifications are described herein. Alternatively, the antagomir can be produced biologically using an expression vector into which a nucleic acid has been subcloned in an antisense orientation (*i. e.,* RNA transcribed from the inserted nucleic acid will be of an antisense orientation to a target nucleic acid of interest (e.g., an miRNA or pre-miRNA)).

### Chemical Definitions

The term "halo" refers to any radical of fluorine, chlorine, bromine or iodine.

The term "alkyl" refers to a hydrocarbon chain that may be a straight chain or branched chain, containing the indicated number of carbon atoms. For example, C₁-C₁₂ alkyl indicates that the group may have from 1 to 12 (inclusive) carbon atoms in it. The term "haloalkyl" refers to an alkyl in which one or more hydrogen atoms are replaced by halo, and includes alkyl moieties in which all hydrogens have been replaced by halo (*e.g.*, perfluoroalkyl). Alkyl and haloalkyl groups may be optionally inserted with O, N, or S. The terms "aralkyl" refers to an alkyl moiety in which an alkyl hydrogen atom is replaced by an aryl group. Aralkyl includes groups in which more than one hydrogen atom has been replaced by an aryl group. Examples of "aralkyl" include benzyl, 9-fluorenyl, benzhydryl, and trityl groups.

The term "alkenyl" refers to a straight or branched hydrocarbon chain containing 2-8 carbon atoms and characterized in having one or more double bonds. Examples of a typical alkenyl include, but not limited to, allyl, propenyl, 2-butenyl, 3-hexenyl and 3-octenyl groups. The term "alkynyl" refers to a straight or branched hydrocarbon chain containing 2-8 carbon atoms and characterized in having one or more triple bonds. Some examples of a typical alkynyl are ethynyl, 2-propynyl, and 3-methylbutynyl, and propargyl. The sp² and sp³ carbons may optionally serve as the point of attachment of the alkenyl and alkynyl groups, respectively.

The terms "alkylamino" and "dialkylamino" refer to -NH(alkyl) and-NH(alkyl)₂ radicals respectively. The term "aralkylamino" refers to a -NH(aralkyl) radical. The term "alkoxy" refers to an -O-alkyl radical, and the terms "cycloalkoxy" and "aralkoxy" refer to an -O-cycloalkyl and O-aralkyl radicals respectively. The term "siloxy" refers to a R₃SiO- radical. The term "mercapto" refers to an SH radical. The term "thioalkoxy" refers to an -S-alkyl radical.

The term "alkylene" refers to a divalent alkyl (*i.e.,* -R-), e.g., -CH₂-, -CH₂CH₂-, and -CH₂CH₂CH₂-. The term "alkylenedioxo" refers to a divalent species of the structure -O-R-O-, in which R represents an alkylene.

The term "aryl" refers to an aromatic monocyclic, bicyclic, or tricyclic hydrocarbon ring system, wherein any ring atom can be substituted. Examples of aryl moieties include, but are not limited to, phenyl, naphthyl, anthracenyl, and pyrenyl.

The term "cycloalkyl" as employed herein includes saturated cyclic, bicyclic, tricyclic,or polycyclic hydrocarbon groups having 3 to 12 carbons, wherein any ring atom can be substituted. The cycloalkyl groups herein described may also contain fused rings. Fused rings are rings that share a common carbon-carbon bond or a common carbon atom (e.g., spiro-fused rings). Examples of cycloalkyl moieties include, but are not limited to, cyclohexyl, adamantyl, and norbornyl, and decalin.

The term "heterocyclyl" refers to a nonaromatic 3-10 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (e.g., carbon atoms and 1-3, 1-6, or 1-9 heteroatoms of N, O, or S if monocyclic, bicyclic, or tricyclic, respectively), wherein any ring atom can be substituted. The heterocyclyl groups herein described may also contain fused rings. Fused rings are rings that share a common carbon-carbon bond or a common carbon atom (e.g., spiro-fused rings). Examples of heterocyclyl include, but are not limited to tetrahydrofuranyl, tetrahydropyranyl, piperidinyl, morpholino, pyrrolinyl and pyrrolidinyl.

The term "cycloalkenyl" as employed herein includes partially unsaturated, nonaromatic, cyclic, bicyclic, tricyclic, or polycyclic hydrocarbon groups having 5 to 12 carbons, preferably 5 to 8 carbons, wherein any ring atom can be substituted. The cycloalkenyl groups herein described may also contain fused rings. Fused rings are rings that share a common carbon-carbon bond or a common carbon atom (e.g., spiro-fused rings). Examples of cycloalkenyl moieties include, but are not limited to cyclohexenyl, cyclohexadienyl, or norbornenyl.

The term "heterocycloalkenyl" refers to a partially saturated, nonaromatic 5-10 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (e.g., carbon atoms and 1-3, 1-6, or 1-9 heteroatoms of N, O, or S if monocyclic, bicyclic, or tricyclic, respectively), wherein any ring atom can be substituted. The heterocycloalkenyl groups herein described may also contain fused rings. Fused rings are rings that share a common carbon-carbon bond or a common carbon atom (e.g., spiro-fused rings). Examples of heterocycloalkenyl include but are not limited to tetrahydropyridyl and dihydropyran.

The term "heteroaryl" refers to an aromatic 5-8 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (*e.g.*, carbon atoms and 1-3, 1-6, or 1-9 heteroatoms of N, O, or S if monocyclic, bicyclic, or tricyclic, respectively), wherein any ring atom can be substituted. The heteroaryl groups herein described may also contain fused rings that share a common carbon-carbon bond.

The term "oxo" refers to an oxygen atom, which forms a carbonyl when attached to carbon, an N-oxide when attached to nitrogen, and a sulfoxide or sulfone when attached to sulfur.

The term "acyl" refers to an alkylcarbonyl, cycloalkylcarbonyl, arylcarbonyl, heterocyclylcarbonyl, or heteroarylcarbonyl substituent, any of which may be further substituted by substituents.

The term "substituents" refers to a group "substituted" on an alkyl, cycloalkyl, alkenyl, alkynyl, heterocyclyl, heterocycloalkenyl, cycloalkenyl, aryl, or heteroaryl group at any atom of that group. Suitable substituents include, without limitation, alkyl, alkenyl, alkynyl, alkoxy, halo, hydroxy, cyano, nitro, amino, SO₃H, sulfate, phosphate, perfluoroalkyl, perfluoroalkoxy, methylenedioxy, ethylenedioxy, carboxyl, oxo, thioxo, imino (alkyl, aryl, aralkyl), S(O)ₙalkyl (where n is 0-2), S(O)ₙ aryl (where n is 0-2), S(O)ₙ heteroaryl (where n is 0-2), S(O)ₙ heterocyclyl (where n is 0-2), amine (mono-, di-, alkyl, cycloalkyl, aralkyl, heteroaralkyl, and combinations thereof), ester (alkyl, aralkyl, heteroaralkyl), amide (mono-, di-, alkyl, aralkyl, heteroaralkyl, and combinations thereof), sulfonamide (mono-, di-, alkyl, aralkyl, heteroaralkyl, and combinations thereof), unsubstituted aryl, unsubstituted heteroaryl, unsubstituted heterocyclyl, and unsubstituted cycloalkyl. In one aspect, the substituents on a group are independently any one single, or any subset of the aforementioned substituents.

The terms "adeninyl, cytosinyl, guaninyl, thyminyl, and uracilyl" and the like refer to radicals of adenine, cytosine, guanine, thymine, and uracil.

A "protected" moiety refers to a reactive functional group, e.g., a hydroxyl group or an amino group, or a class of molecules, *e.g.,* sugars, having one or more functional groups, in which the reactivity of the functional group is temporarily blocked by the presence of an attached protecting group. Protecting groups useful for the monomers and methods described herein can be found, *e.g.,* in Greene, T.W., Projective Groups in Organic Synthesis (John Wiley and Sons: New York), 1981 .

### Antagomir Structure

An antagomir, such as a single-stranded oligonucleotide agent, featured in the invention includes a region sufficient complementarity to the target nucleic acid (e.g., target miRNA, pre-miRNA or mRNA), and is of sufficient length in terms of nucleotides, such that the antagomir forms a duplex with the target nucleic acid. The antagomir can modulate the function of the targeted molecule. For example, when the targeted molecule is an miRNA, such as miR-122, the antagomir can inhibit the gene silencing activity of the target miRNA, which action will up-regulate expression of the mRNA targeted by the target miRNA. When the target is an mRNA, the antagomir can replace or supplement the gene silencing activity of an endogenous miRNA.

For ease of exposition the term nucleotide or ribonucleotide is sometimes used herein in reference to one or more monomeric subunits of an oligonucleotide agent. It will be understood herein that the usage of the term "ribonucleotide" or "nucleotide" herein can, in the case of a modified RNA or nucleotide surrogate, also refer to a modified nucleotide, or surrogate replacement moiety at one or more positions.

An antagomir featured in the invention is, or includes, a region that is at least partially, and in some embodiments fully, complementary to the target RNA. It is not necessary that there be perfect complementarity between the antagomir and the target, but the correspondence must be sufficient to enable the oligonucleotide agent, or a cleavage product thereof, to modulate (e.g., inhibit) target gene expression.

An antagomir will preferably have one or more of the following properties:
(1) it will be of the Formula 1, 2, 3, or 4 described below;
(2) it will have a 5' modification that includes one or more phosphate groups or one or more analogs of a phosphate group;
(3) it will, despite modifications, even to a very large number of bases specifically base pair and form a duplex structure with a homologous target RNA of sufficient thermodynamic stability to allow modulation of the activity of the targeted RNA;
(4) it will, despite modifications, even to a very large number, or all of the nucleosides, still have "RNA-like" properties, *i.e.,* it will possess the overall structural, chemical and physical properties of an RNA molecule, even though not exclusively, or even partly, of ribonucleotide-based content. For example, all of the nucleotide sugars can contain e.g., 2'OMe, 2' fluoro in place of 2' hydroxyl. This deoxyribonucleotide-containing agent can still be expected to exhibit RNA-like properties. While not wishing to be bound by theory, an electronegative fluorine prefers an axial orientation when attached to the C2' position of ribose. This spatial preference of fluorine can, in turn, force the sugars to adopt a C_{3'}*-endo* pucker. This is the same puckering mode as observed in RNA molecules and gives rise to the RNA-characteristic A-family-type helix. Further, since fluorine is a good hydrogen bond acceptor, it can participate in the same hydrogen bonding interactions with water molecules that are known to stabilize RNA structures. (Generally, it is preferred that a modified moiety at the 2' sugar position will be able to enter into hydrogen-bonding which is more characteristic of the 2'-OH moiety of a ribonucleotide than the 2'-H moiety of a deoxyribonucleotide. A preferred antagomir will: exhibit a C_{3'}*-endo* pucker in all, or at least 50, 75,80, 85, 90, or 95 % of its sugars; exhibit a C_{3'}*-endo* pucker in a sufficient amount of its sugars that it can give rise to a the RNA-characteristic A-family-type helix; will have no more than 20, 10, 5, 4, 3, 2, or 1 sugar which is not a C_{3'}*-endo* pucker structure.

Preferred 2'-modifications with C3'-endo sugar pucker include:
2'-OH, 2'-O-Me, 2'-O-methoxyethyl, 2'-O-aminopropyl, 2'-F, 2'-O-CH2-CO-NHMe, 2'-O-CH2-CH2-O-CH2-CH2-N(Me)2, and LNA

Preferred 2'-modifications with a C2'-endo sugar pucker include:
2'-H, 2'-Me, 2'-S-Me, 2'-Ethynyl, 2'-ara-F.

Sugar modifications can also include L-sugars and 2'-5'-linked sugars.

As used herein, "specifically hybridizable" and "complementary" are terms that are used to indicate a sufficient degree of complementarity such that stable and specific binding occurs between an antagomir of the invention and a target RNA molecule, e.g., an miRNA or a pre-miRNA. Specific binding requires a sufficient lack of complementarity to non-target sequences under conditions in which specific binding is desired, *i.e.,* under physiological conditions in the case of *in vivo* assays or therapeutic treatment, or in the case of *in vitro* assays, under conditions in which the assays are performed. It has been shown that a single mismatch between targeted and non-targeted sequences are sufficient to provide discrimination for siRNA targeting of an mRNA *(*Brummelkamp et al., Cancer Cell, 2002, 2:243).

In one embodiment, an antagomir is "sufficiently complementary" to a target RNA, such that the antagomir inhibits production of protein encoded by the target mRNA. The target RNA can be, *e.g.,* a pre-mRNA, mRNA, or miRNA endogenous to the subject. In another embodiment, the antagomir is "exactly complementary" (excluding the SRMS containing subunit(s)) to a target RNA, e.g., the target RNA and the antagomir can anneal to form a hybrid made exclusively of Watson-Crick base pairs in the region of exact complementarity. A "sufficiently complementary" target RNA can include a region (e.g., of at least 7 nucleotides) that is exactly complementary to a target RNA. Moreover, in some embodiments, the antagomir specifically discriminates a single-nucleotide difference. In this case, the antagomir only down-regulates gene expression if exact complementarity is found in the region of the single-nucleotide difference.

Oligonucleotide agents discussed herein include otherwise unmodified RNA and DNA as well as RNA and DNA that have been modified, e.g., to improve efficacy, and polymers of nucleoside surrogates. Unmodified RNA refers to a molecule in which the components of the nucleic acid, namely sugars, bases, and phosphate moieties, are the same or essentially the same as that which occur in nature, preferably as occur naturally in the human body. The art has referred to rare or unusual, but naturally occurring, RNAs as modified RNAs, see, *e.g.,* Limbach et al. (Nucleic Acids Res., 1994, 22:2183-2196). Such rare or unusual RNAs, often termed modified RNAs, are typically the result of a post-transcriptional modification and are within the term unmodified RNA as used herein. Modified RNA, as used herein, refers to a molecule in which one or more of the components of the nucleic acid, namely sugars, bases, and phosphate moieties, are different from that which occur in nature, preferably different from that which occurs in the human body. While they are referred to as "modified RNAs" they will of course, because of the modification, include molecules that are not, strictly speaking, RNAs. Nucleoside surrogates are molecules in which the ribophosphate backbone is replaced with a non-ribophosphate construct that allows the bases to be presented in the correct spatial relationship such that hybridization is substantially similar to what is seen with a ribophosphate backbone, e.g., non-charged mimics of the ribophosphate backbone. Examples of all of the above are discussed herein.

As nucleic acids are polymers of subunits or monomers, many of the modifications described below occur at a position which is repeated within a nucleic acid, e.g., a modification of a base, or a phosphate moiety, or a non-linking O of a phosphate moiety. In some cases the modification will occur at all of the subject positions in the nucleic acid but in many, and in fact in most cases it will not. By way of example, a modification may only occur at a 3' or 5' terminal position, in a terminal region, *e.g.*, at a position on a terminal nucleotide, or in the last 2, 3, 4, 5, or 10 nucleotides of a strand. The ligand can be attached at the 3' end, the 5' end, or at an internal position, or at a combination of these positions. For example, the ligand can be at the 3' end and the 5' end; at the 3' end and at one or more internal positions; at the 5' end and at one or more internal positions; or at the 3' end, the 5' end, and at one or more internal positions. For example, a phosphorothioate modification at a non-linking O position may only occur at one or both termini, or may only occur in a terminal region, **e.g.,** at a position on a terminal nucleotide or in the last 2, 3, 4, 5, or 10 nucleotides of the oligonucleotide. The 5' end can be phosphorylated.

Modifications and nucleotide surrogates are discussed below.

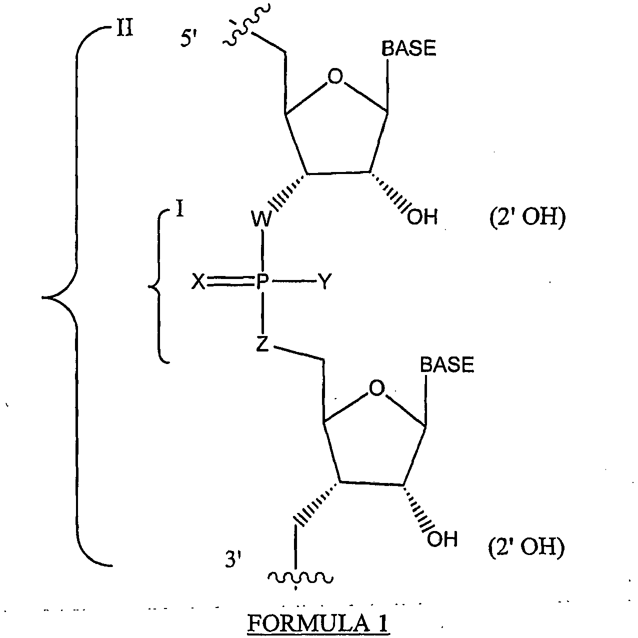

The scaffold presented above in Formula 1 represents a portion of a ribonucleic acid. The basic components are the ribose sugar, the base, the terminal phosphates, and phosphate internucleotide linkers. Where the bases are naturally occurring bases, *e.g.*, adenine, uracil, guanine or cytosine, the sugars are the unmodified 2' hydroxyl ribose sugar (as depicted) and W, X, Y, and Z are all O, Formula 1 represents a naturally occurring unmodified oligoribonucleotide.

Unmodified oligoribonucleotides may be less than optimal in some applications, e.g., unmodified oligoribonucleotides can be prone to degradation by *e.g.*, cellular nucleases. Nucleases can hydrolyze nucleic acid phosphodiester bonds. However, chemical modifications to one or more of the above RNA components can confer improved properties, and, for example, can render oligoribonucleotides more stable to nucleases. Unmodified oligoribonucleotides may also be less than optimal in terms of offering tethering points for attaching ligands or other moieties to an oligonucleotide agent.

Modified nucleic acids and nucleotide surrogates can include one or more of:
(i) alteration, **e.g.,** replacement, of one or both of the non-linking (X and Y) phosphate oxygens and/or of one or more of the linking (W and Z) phosphate oxygens (When the phosphate is in the terminal position, one of the positions W or Z will not link the phosphate to an additional element in a naturally occurring ribonucleic acid. However, for simplicity of terminology, except where otherwise noted, the W position at the 5' end of a nucleic acid and the terminal Z position at the 3' end of a nucleic acid, are within the term "linking phosphate oxygens" as used herein.);
(ii) alteration, *e.g.,* replacement, of a constituent of the ribose sugar, *e.g.,* of the 2' hydroxyl on the ribose sugar, or wholesale replacement of the ribose sugar with a structure other than ribose, **e.g.,** as described herein;
(iii) wholesale replacement of the phosphate moiety (bracket I) with "dephospho" linkers;
(iv) modification or replacement of a naturally occurring base;
(v) replacement or modification of the ribose-phosphate backbone (bracket II);
(vi) modification of the 3' end or 5' end of the RNA, **e.g.,** removal, modification or replacement of a terminal phosphate group or conjugation of a moiety, such as a fluorescently labeled moiety, to either the 3' or 5' end of RNA.

The terms replacement, modification, alteration, and the like, as used in this context, do not imply any process limitation, **e.g.,** modification does not mean that one must start with a reference or naturally occurring ribonucleic acid and modify it to produce a modified ribonucleic acid but rather modified simply indicates a difference from a naturally occurring molecule.

It is understood that the actual electronic structure of some chemical entities cannot be adequately represented by only one canonical form (*i.e.* Lewis structure). While not wishing to be bound by theory, the actual structure can instead be some hybrid or weighted average of two or more canonical forms, known collectively as resonance forms or structures. Resonance structures are not discrete chemical entities and exist only on paper. They differ from one another only in the placement or "localization" of the bonding and nonbonding electrons for a particular chemical entity. It can be possible for one resonance structure to contribute to a greater extent to the hybrid than the others. Thus, the written and graphical descriptions of the embodiments of the present invention are made in terms of what the art recognizes as the predominant resonance form for a particular species. For example, any phosphoroamidate (replacement of a nonlinking oxygen with nitrogen) would be represented by X = O and Y = N in the above figure.

Specific modifications are discussed in more detail below.

### The Phosphate Group

The phosphate group is a negatively charged species. The charge is distributed equally over the two non-linking oxygen atoms (*i.e.,* X and Y in Formula 1 above). However, the phosphate group can be modified by replacing one of the oxygens with a different substituent. One result of this modification to RNA phosphate backbones can be increased resistance of the oligoribonucleotide to nucleolytic breakdown. Thus while not wishing to be bound by theory, it can be desirable in some embodiments to introduce alterations which result in either an uncharged linker or a charged linker with unsymmetrical charge distribution.

Examples of modified phosphate groups include phosphorothioate, phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulfur. Unlike the situation where only one of X or Y is altered, the phosphorus center in the phosphorodithioates is achiral which precludes the formation of oligoribonucleotides diastereomers. Diastereomer formation can result in a preparation in which the individual diastereomers exhibit varying resistance to nucleases. Further, the hybridization affinity of RNA containing chiral phosphate groups can be lower relative to the corresponding unmodified RNA species. Thus, while not wishing to be bound by theory, modifications to both X and Y which eliminate the chiral center, e.g., phosphorodithioate formation, may be desirable in that they cannot produce diastereomer mixtures. Thus, X can be any one of S, Se, B, C, H, N, or OR (R is alkyl or aryl). Thus Y can be any one of S, Se, B, C, H, N, or OR (R is alkyl or aryl). Replacement of X and/or Y with sulfur is preferred.

The phosphate linker can also be modified by replacement of a linking oxygen (*i.e.,* W or Z in Formula 1) with nitrogen (bridged phosphoroamidates), sulfur (bridged phosphorothioates) and carbon (bridged methylenephosphonates). The replacement can occur at a terminal oxygen (position W (3') or position Z (5')). Replacement of W with carbon or Z with nitrogen is preferred.

Candidate agents can be evaluated for suitability as described below.

### The Sugar Group

A modified RNA can include modification of all or some of the sugar groups of the ribonucleic acid. For example, the 2' hydroxyl group (OH) can be modified or replaced with a number of different "oxy" or "deoxy" substituents. While not being bound by theory, enhanced stability is expected since the hydroxyl can no longer be deprotonated to form a 2' alkoxide ion. The 2' alkoxide can catalyze degradation by intramolecular nucleophilic attack on the linker phosphorus atom. Again, while not wishing to be bound by theory, it can be desirable to some embodiments to introduce alterations in which alkoxide formation at the 2' position is not possible.

Examples of "oxy"-2' hydroxyl group modifications include alkoxy or aryloxy (OR, *e.g.,* R = H, alkyl, cycloalkyl, aryl, aralkyl, heteroaryl or sugar); polyethyleneglycols (PEG), O(CH₂CH₂O)ₙCH₂CH₂OR; "locked" nucleic acids (LNA) in which the 2' hydroxyl is connected, e.g., by a methylene bridge or ethylene bridge (*e.g.*, 2'-4'-ethylene bridged nucleic acid (ENA)), to the 4' carbon of the same ribose sugar; amino, O-AMINE (AMINE = NH₂; alkylamino, dialkylamino, heterocyclyl, arylamino, diaryl amino, heteroaryl amino, or diheteroaryl amino, ethylene diamine, polyamino) and aminoalkoxy, O(CH₂)ₙAMINE, *(e.g.,* AMINE = NH₂; alkylamino, dialkylamino, heterocyclyl, arylamino, diaryl amino, heteroaryl amino, or diheteroaryl amino, ethylene diamine, polyamino). It is noteworthy that oligonucleotides containing only the methoxyethyl group (MOE), (OCH₂CH₂OCH₃, a PEG derivative), exhibit nuclease stabilities comparable to those modified with the robust phosphorothioate modification.

"Deoxy" modifications include hydrogen (*i.e.* deoxyribose sugars); halo *(e.g.,* fluoro); amino (*e.g*. NH₂; alkylamino, dialkylamino, heterocyclyl, arylamino, diaryl amino, heteroaryl amino, diheteroaryl amino, or amino acid); NH(CH₂CH₂NH)ₙCH₂CH₂-AMINE (AMINE = NH₂; alkylamino, dialkylamino, heterocyclyl, arylamino, diaryl amino, heteroaryl amino,or diheteroaryl amino), - NHC(O)R (R = alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with e.g., an amino functionality. Preferred substitutents are 2'-methoxyethyl, 2'-OCH3, 2'-O-allyl, 2'-C- allyl, and 2'-fluoro.

The sugar group can also contain one or more carbons that possess the opposite stereochemical configuration than that of the corresponding carbon in ribose. Thus, a modified RNA can include nucleotides containing e.g., arabinose, as the sugar.

Modified RNAs can also include "abasic" sugars, which lack a nucleobase at C-1'. These abasic sugars can also be further contain modifications at one or more of the constituent sugar atoms.

To maximize nuclease resistance, the 2' modifications can be used in combination with one or more phosphate linker modifications (*e.g.*, phosphorothioate). The so-called "chimeric" oligonucleotides are those that contain two or more different modifications.

The modification can also entail the wholesale replacement of a ribose structure with another entity (an SRMS) at one or more sites in the oligonucleotide agent.

Candidate modifications can be evaluated as described below.

### Replacement of the Phosphate Group

The phosphate group can be replaced by non-phosphorus containing connectors (*cf.* Bracket I in Formula 1 above). While not wishing to be bound by theory, it is believed that since the charged phosphodiester group is the reaction center in nucleolytic degradation, its replacement with neutral structural mimics should impart enhanced nuclease stability. Again, while not wishing to be bound by theory, it can be desirable, in some embodiment, to introduce alterations in which the charged phosphate group is replaced by a neutral moiety.

Examples of moieties which can replace the phosphate group include siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino. Preferred replacements include the methylenecarbonylamino and methylenemethylimino groups.

Candidate modifications can be evaluated as described below.

### Replacement of Ribophosphate Backbone

Oligonucleotide- mimicking scaffolds can also be constructed wherein the phosphate linker and ribose sugar are replaced by nuclease resistant nucleoside or nucleotide surrogates (see Bracket II of Formula 1 above). While not wishing to be bound by theory, it is believed that the absence of a repetitively charged backbone diminishes binding to proteins that recognize polyanions (*e.g*. nucleases). Again, while not wishing to be bound by theory, it can be desirable in some embodiment, to introduce alterations in which the bases are tethered by a neutral surrogate backbone.

Examples include the mophilino, cyclobutyl, pyrrolidine and peptide nucleic acid (PNA) nucleoside surrogates. A preferred surrogate is a PNA surrogate.

Candidate modifications can be evaluated as described below.

### Terminal Modifications

The 3' and 5' ends of an oligonucleotide can be modified. Such modifications can be at the 3' end, 5' end or both ends of the molecule. They can include modification or replacement of an entire terminal phosphate or of one or more of the atoms of the phosphate group. *E.g.*, the 3' and 5' ends of an oligonucleotide can be conjugated to other functional molecular entities such as labeling moieties, e.g., fluorophores (*e.g.*, pyrene, TAMRA, fluorescein, Cy3 or Cy5 dyes) or protecting groups (based e.g., on sulfur, silicon, boron or ester). The functional molecular entities can be attached to the sugar through a phosphate group and/or a spacer. The terminal atom of the spacer can connect to or replace the linking atom of the phosphate group or the C-3' or C-5' O, N, S or C group of the sugar. Alternatively, the spacer can connect to or replace the terminal atom of a nucleotide surrogate (e.g., PNAs). These spacers or linkers can include e.g., -(CH₂)ₙ-, -(CH₂)ₙN-, -(CH₂)ₙO-,-(CH₂)ₙS-, O(CH₂CH₂O)ₙCH₂CH₂OH *(e.g.,* n = 3 or 6), abasic sugars, amide, carboxy, amine, oxyamine, oxyimine, thioether, disulfide, thiourea, sulfonamide, or morpholino, or biotin and fluorescein reagents. While not wishing to be bound by theory, it is believed that conjugation of certain moieties can improve transport, hybridization, and specificity properties. Again, while not wishing to be bound by theory, it may be desirable to introduce terminal alterations that improve nuclease resistance. Other examples of terminal modifications include dyes, intercalating agents *(e.g.* acridines), cross-linkers *(e.g.* psoralene, mitomycin C), porphyrins (TPPC4, texaphyrin, Sapphyrin), polycyclic aromatic hydrocarbons (*e.g*., phenazine, dihydrophenazine), artificial endonucleases *(e.g.* EDTA), lipophilic carriers *(e.g.,* cholesterol, cholic acid, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine)and peptide conjugates (e.g., antennapedia peptide, Tat peptide), alkylating agents, phosphate, amino, mercapto, PEG *(e.g.,* PEG-40K), MPEG, [MPEG]₂, polyamino, alkyl, substituted alkyl, radiolabeled markers, enzymes, haptens (*e.g*. biotin), transport/absorption facilitators *(e.g.,* aspirin, vitamin E, folic acid), synthetic ribonucleases (*e.g*., imidazole, bisimidazole, histamine, imidazole clusters, acridine-imidazole conjugates, Eu3+ complexes of tetraazamacrocycles).

Terminal modifications can be added for a number of reasons, including as discussed elsewhere herein to modulate activity or to modulate resistance to degradation. Preferred modifications include the addition of a methylphosphonate at the 3'-most terminal linkage; a 3' C5-aminoalkyl-dT; 3' cationic group; or another 3' conjugate to inhibit 3'-5' exonucleolytic degradation.

Terminal modifications useful for modulating activity include modification of the 5' end with phosphate or phosphate analogs. *E.g.,* in preferred embodiments oligonucleotide agents are 5' phosphorylated or include a phosphoryl analog at the 5' terminus. 5'-phosphate modifications include those which are compatible with RISC mediated gene silencing. Suitable modifications include: 5'-monophosphate ((HO)2(O)P-O-5'); 5'-diphosphate ((HO)2(O)P-O-P(HO)(O)-O-5'); 5'-triphosphate ((HO)2(O)P-O-(HO)(O)P-O-P(HO)(O)-O-5'); 5'-guanosine cap (7-methylated or non-methylated) (7m-G-O-5'-(HO)(O)P-O-(HO)(O)P-O-P(HO)(O)-O-5'); 5'-adenosine cap (Appp), and any modified or unmodified nucleotide cap structure (N-O-5'-(HO)(O)P-O-(HO)(O)P-O-P(HO)(O)-O-5'); 5'-monothiophosphate (phosphorothioate; (HO)2(S)P-O-5'); 5'-monodithiophosphate (phosphorodithioate; (HO)(HS)(S)P-O-5'), 5'-phosphorothiolate ((HO)2(O)P-S-5'); any additional combination of oxgen/sulfur replaced monophosphate, diphosphate and triphosphates (*e.g.* 5'-alpha-thiotriphosphate, 5'-gamma-thiotriphosphate, etc.), 5'-phosphoramidates ((HO)2(O)P-NH-5', (HO)(NH2)(O)P-O-5'), 5'-alkylphosphonates (R=alkyl=methyl, ethyl, isopropyl, propyl, etc., *e.g*. RP(OH)(O)-O-5'-, (OH)2(O)P-5'-CH2-), 5'-alkyletherphosphonates (R=alkylether=methoxymethyl (MeOCH2-), ethoxymethyl, etc., *e.g.* RP(OH)(O)-O-5'-).

Terminal modifications can also be useful for monitoring distribution, and in such cases the preferred groups to be added include fluorophores, *e.g.,* fluorscein or an Alexa dye, *e.g.,* Alexa 488. Terminal modifications can also be useful for enhancing uptake, useful modifications for this include cholesterol. Terminal modifications can also be useful for cross-linking anantagomir to another moiety; modifications useful for this include mitomycin C.

Candidate modifications can be evaluated as described below.

### The Bases

Adenine, guanine, cytosine and uracil are the most common bases found in RNA. These bases can be modified or replaced to provide RNA's having improved properties. *E.g.,* nuclease resistant oligoribonucleotides can be prepared with these bases or with synthetic and natural nucleobases *(e.g.,* inosine, thymine, xanthine, hypoxanthine, nubularine, isoguanisine, or tubercidine) and any one of the above modifications. Alternatively, substituted or modified analogs of any of the above bases, e.g., "unusual bases" and "universal bases" described herein, can be employed. Examples include without limitation 2-aminoadenine, 6-methyl and other alkyl derivatives of adenine and guanine, 2-propyl and other alkyl derivatives of adenine and guanine, 5-halouracil and cytosine, 5-propynyl uracil and cytosine, 6-azo uracil, cytosine and thymine, 5-uracil (pseudouracil), 4-thiouracil, 5-halouracil, 5-(2-aminopropyl)uracil, 5-amino allyl uracil, 8-halo, amino, thiol, thioalkyl, hydroxyl and other 8-substituted adenines and guanines, 5-trifluoromethyl and other 5-substituted uracils and cytosines, 7-methylguanine, 5-substituted pyrimidines, 6-azapyrimidines and N-2, N-6 and O-6 substituted purines, including 2-aminopropyladenine, 5-propynyluracil and 5-propynylcytosine, dihydrouracil, 3-deaza-5-azacytosine, 2-aminopurine, 5-alkyluracil, 7-alkylguanine, 5-alkyl cytosine,7-deazaadenine, N6, N6-dimethyladenine, 2,6-diaminopurine, 5-amino-allyl-uracil, N3-methyluracil, substituted 1,2,4-triazoles, 2-pyridinone, 5-nitroindole, 3-nitropyrrole, 5-methoxyuracil, uracil-5-oxyacetic acid, 5-methoxycarbonylmethyluracil, 5-methyl-2-thiouracil, 5-methoxycarbonylmethyl-2-thiouracil, 5-methylaminomethyl-2-thiouracil, 3-(3-amino-3carboxypropyl)uracil, 3-methylcytosine, 5-methylcytosine, N⁴-acetyl cytosine, 2-thiocytosine, N6-methyladenine, N6-isopentyladenine, 2-methylthio-N6-isopentenyladenine, N-methylguanines, or O-alkylated bases. Further purines and pyrimidines include those disclosed in U.S. Pat. No. 3,687,808, those disclosed in the Concise Encyclopedia Of Polymer Science And Engineering, pages 858-859, Kroschwitz, J. I., ed. John Wiley & Sons, 1990, and those disclosed by Englisch et al., Angewandte Chemie, International Edition, 1991, 30, 613.

Candidate modifications can be evaluated as described below.

### Evaluation of Candidate Oligonucleotide agents

One can evaluate a candidate single-stranded oligonucleotide agent, e.g., a modified candidate single-stranded oligonucleotide agent, for a selected property by exposing the agent or modified molecule and a control molecule to the appropriate conditions and evaluating for the presence of the selected property. For example, resistance to a degradent can be evaluated as follows. A candidate modified antagomir (and preferably a control single-stranded oligonucleotide agent, usually the unmodified form) can be exposed to degradative conditions, *e.g.,* exposed to a milieu, which includes a degradative agent, *e.g.,* a nuclease. For example, one can use a biological sample, *e.g.,* one that is similar to a milieu, which might be encountered, in therapeutic use, *e.g.,* blood or a cellular fraction, *e.g.,* a cell-free homogenate or disrupted cells. The candidate and control can then be evaluated for resistance to degradation by any of a number of approaches. For example, the candidate and control could be labeled, preferably prior to exposure, with, *e.g.,* a radioactive or enzymatic label, or a fluorescent label, such as Cy3 or Cy5. Control and modified oligonucleotide agents can be incubated with the degradative agent, and optionally a control, *e.g.,* an inactivated, *e.g.,* heat inactivated, degradative agent. A physical parameter, *e.g.,* size, of the modified and control molecules are then determined. They can be determined by a physical method, *e.g.,* by polyacrylamide gel electrophoresis or a sizing column, to assess whether the molecule has maintained its original length, or assessed functionally. Alternatively, Northern blot analysis can be used to assay the length of an unlabeled modified molecule.

A functional assay can also be used to evaluate the candidate agent. A functional assay can be applied initially or after an earlier non-functional assay, *(e.g.,* assay for resistance to degradation) to determine if the modification alters the ability of the molecule to inhibit gene expression. For example, a cell, *e.g.,* a mammalian cell, such as a mouse or human cell, can be co-transfected with a plasmid expressing a fluorescent protein, *e.g.,* GFP, and a candidate antagomir homologous to the transcript encoding the fluorescent protein (see, *e.g.,* WO 00/44914). For example, a modified antagomir homologous to the GFP mRNA can be assayed for the ability to inhibit GFP expression by monitoring for a decrease in cell fluorescence, as compared to a control cell, in which the transfection did not include the candidate oligonucleotide agent, *e.g.,* controls with no agent added and/or controls with a non-modified RNA added. Efficacy of the candidate agent on gene expression can be assessed by comparing cell fluorescence in the presence of the modified and unmodified oligonucleotide agent.

In an alternative functional assay, a candidate antagomir homologous to an endogenous mouse gene, preferably a maternally expressed gene, such as *c-mos,* can be injected into an immature mouse oocyte to assess the ability of the agent to inhibit gene expression *in vivo* (see, *e.g.,* WO 01/36646). A phenotype of the oocyte, *e.g.,* the ability to maintain arrest in metaphase II, can be monitored as an indicator that the agent is inhibiting expression. For example, cleavage of *c-mos* mRNA by an antagomir would cause the oocyte to exit metaphase arrest and initiate parthenogenetic development (Colledge et al. Nature 370: 65-68, 1994; Hashimoto et al. Nature, 370:68-71, 1994). The effect of the modified agent on target RNA levels can be verified by Northern blot to assay for a decrease in the level of target RNA, or by Western blot to assay for a decrease in the level of target protein, as compared to a negative control. Controls can include cells in which with no agent is added and/or cells in which a non-modified RNA is added.

### Preferred oligonucleotide agents

Preferred single-stranded oligonucleotide agents have the following structure (see Formula 2 below):

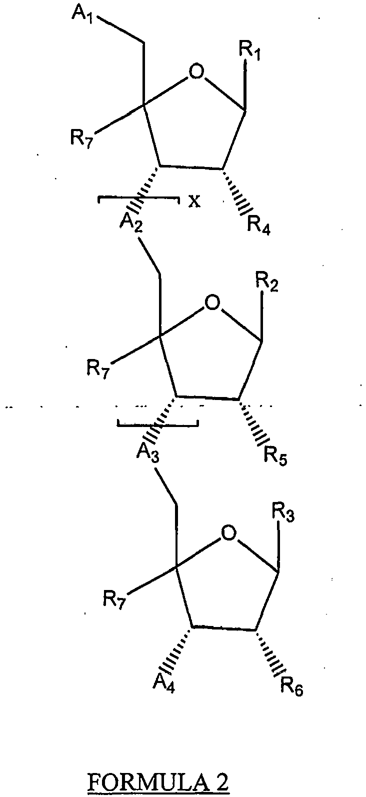

Referring to Formula 2 above, R¹, R², and R³ are each, independently, H, (*i.e.* abasic nucleotides), adenine, guanine, cytosine arid uracil, inosine, thymine, xanthine, hypoxanthine, nubularine, tubercidine, isoguanisine, 2-aminoadenine, 6-methyl and other alkyl derivatives of adenine and guanine, 2-propyl and other alkyl derivatives of adenine and guanine, 5-halouracil and cytosine, 5-propynyl uracil and cytosine, 6-azo uracil, cytosine and thymine, 5-uracil (pseudouracil), 4-thiouracil, 5-halouracil, 5-(2-aminopropyl)uracil, 5-amino allyl uracil, 8-halo, amino, thiol, thioalkyl, hydroxyl and other 8-substituted adenines and guanines, 5-trifluoromethyl and other 5-substituted uracils and cytosines, 7-methylguanine, 5-substituted pyrimidines, 6-azapyrimidines and N-2, N-6 and O-6 substituted purines, including 2-aminopropyladenine, 5-propynyluracil and 5-propynylcytosine, dihydrouracil, 3-deaza-5-azacytosine, 2-aminopurine, 5-alkyluracil, 7-alkylguanine, 5-alkyl cytosine,7-deazaadenine, 7-deazaguanine, N6, N6-dimethyladenine, 2,6-diaminopurine, 5-amino-allyl-uracil, N3-methyluracil, substituted 1,2,4-triazoles, 2-pyridinone, 5-nitroindole, 3-nitropyrrole, 5-methoxyuracil, uracil-5-oxyacetic acid, 5-methoxycarbonylmethyluracil, 5-methyl-2-thiouracil, 5-methoxycarbonylmethyl-2-thiouracil, 5-methylaminomethyl-2-thiouracil, 3-(3-amino-3carboxypropyl)uracil, 3-methylcytosine, 5-methylcytosine, N⁴-acetyl cytosine, 2-thiocytosine, N6-methyladenine, N6-isopentyladenine, 2-methylthio-N6-isopentenyladenine, N-methylguanines, or O-alkylated bases.

R⁴, R⁵, and R⁶ are each, independently, OR⁸, O(CH₂CH₂O)ₘCH₂CH₂OR⁸; O(CH₂)ₙR⁹; O(CH₂)ₙOR⁹, H; halo; NH₂; NHR⁸; N(R⁸)₂; NH(CH₂CH₂NH)ₘCH₂CH₂NHR⁹; NHC(O)R⁸; ; cyano; mercapto, SR⁸; alkyl-thio-alkyl; alkyl, aralkyl, cycloalkyl, aryl, heteroaryl, alkenyl, alkynyl, each of which may be optionally substituted with halo, hydroxy, oxo, nitro, haloalkyl, alkyl, alkaryl, aryl, aralkyl, alkoxy, aryloxy, amino, alkylamino, dialkylamino, heterocyclyl, arylamino, diaryl amino, heteroaryl amino, diheteroaryl amino, acylamino, alkylcarbamoyl, arylcarbamoyl, aminoalkyl, alkoxycarbonyl, carboxy, hydroxyalkyl, alkanesulfonyl, alkanesulfonamido, arenesulfonamido, aralkylsulfonamido, alkylcarbonyl, acyloxy, cyano, or ureido; or R⁴, R⁵, or R⁶ together combine with R⁷ to form an [-O-CH₂-] covalently bound bridge between the sugar 2' and 4' carbons.
A¹ is: 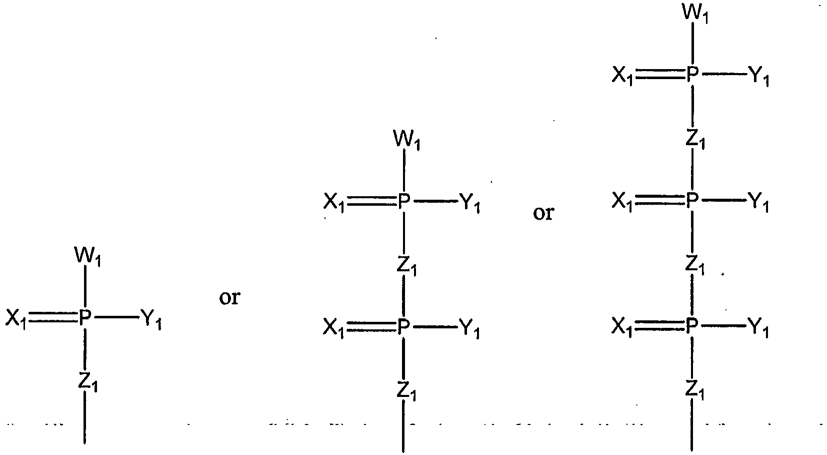 ; H; OH; OCH₃; W¹; an abasic nucleotide; or absent;
   (a preferred A1 , especially with regard to anti-sense strands, is chosen from 5'-monophosphate ((HO)₂(O)P-O-5'), 5'-diphosphate ((HO)₂(O)P-O-P(HO)(O)-O-5'), 5'-triphosphate ((HO)₂(O)P-O-(HO)(O)P-O-P(HO)(O)-O-5'), 5'-guanosine cap (7-methylated or non-methylated) (7m-G-O-5'-(HO)(O)P-O-(HO)(O)P-O-P(HO)(O)-O-5'), 5'-adenosine cap (Appp), and any modified or unmodified nucleotide cap structure (N-O-5'-(HO)(O)P-O-(HO)(O)P-O-P(HO)(O)-O-5'), 5'-monothiophosphate (phosphorothioate; (HO)₂(S)P-O-5'), 5'-monodithiophosphate (phosphorodithioate; (HO)(HS)(S)P-O-5'), 5'-phosphorothiolate ((HO)₂(O)P-S-5'); any additional combination of oxgen/sulfur replaced monophosphate, diphosphate and triphosphates (*e.g.* 5'-alpha-thiotriphosphate, 5'-gamma-thiotriphosphate, etc.), 5'-phosphoramidates ((HO)₂(O)P-NH-5', (HO)(NH₂)(O)P-O-5'), 5'-alkylphosphonates (R=alkyl=methyl, ethyl, isopropyl, propyl, etc., *e.g*. RP(OH)(O)-O-5'-, (OH)₂(O)P-5'-CH₂-), 5'-alkyletherphosphonates (R=alkylether=methoxymethyl (MeOCH₂-), ethoxymethyl, etc., *e.g.* RP(OH)(O)-O-5'-)).
A² is: 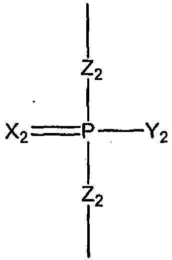
A³ is: 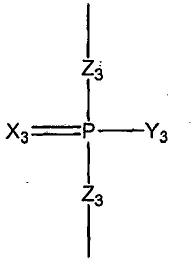 ; and
A⁴ is: 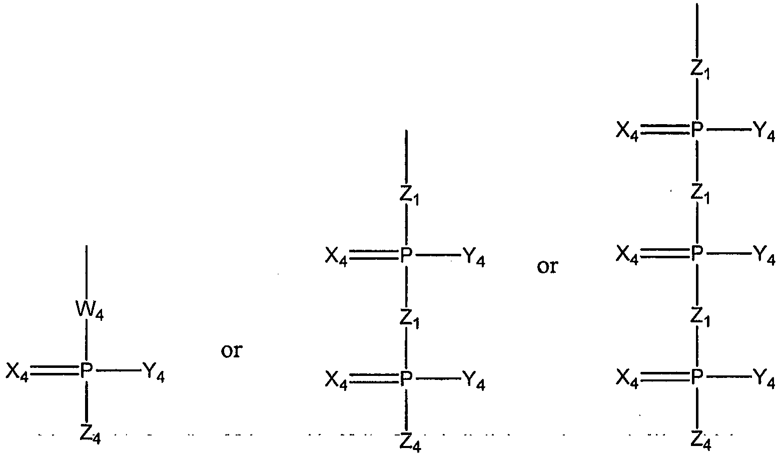 ; H; Z⁴; an inverted nucleotide; an abasic nucleotide; or absent.
W¹ is OH, (CH₂)ₙR¹⁰, (CH₂)ₙNHR¹⁰, (CH₂)ₙ OR¹⁰, (CH₂)ₙ SR¹⁰; O(CH₂)ₙR¹⁰; O(CH₂)ₙOR¹⁰, O(CH₂)ₙNR¹⁰, O(CH₂)ₙSR¹⁰; O(CH₂)ₙSS(CH₂)ₙOR¹⁰, O(CH₂)ₙC(O)OR¹⁰, NH(CH₂)ₙR¹⁰; NH(CH₂)ₙNR¹⁰ ;NH(CH₂)ₙOR¹⁰, NH(CH₂)ₙSR¹⁰; S(CH₂)ₙR¹⁰, S(CH₂)ₙNR¹⁰, S(CH₂)ₙOR¹⁰, S(CH₂)ₙSR¹⁰ O(CH₂CH₂O)ₘCH₂CH₂OR¹⁰; O(CH₂CH₂O)ₘCH₂CH₂NHR¹⁰, NH(CH₂CH₂NH)ₘCH₂CH₂NHR¹⁰; Q-R¹⁰, O-Q-R¹⁰ N-Q-R¹⁰, S-Q-R¹⁰ or -O-. W⁴ is O, CH₂, NH, or S.
X¹, X², X³, and X⁴ are each, independently, O or S.
Y¹, Y², Y³, and Y⁴ are each, independently, OH, O⁻, OR⁸, S, Se, BH₃⁻, H, NHR⁹, N(R⁹)₂ alkyl, cycloalkyl, aralkyl, aryl, or heteroaryl, each of which may be optionally substituted.
Z¹, Z², and Z³ are each independently O, CH₂, NH, or S. Z⁴ is OH, (CH₂)ₙR¹⁰, (CH₂)ₙNHR¹⁰, (CH₂)ₙ OR¹⁰, (CH₂)ₙ SR¹⁰; O(CH₂)ₙR¹⁰; O(CH₂)ₙOR¹⁰, O(CH₂)ₙNR¹⁰, O(CH₂)ₙSR¹⁰, O(CH₂)ₙSS(CH₂)ₙOR¹⁰, O(CH₂)ₙC(O)OR¹⁰; NH(CH₂)ₙR¹⁰; NH(CH₂)ₙNR¹⁰ ;NH(CH₂)ₙOR¹⁰, NH(CH₂)ₙSR¹⁰; S(CH₂)ₙR¹⁰, S(CH₂)ₙNR¹⁰, S(CH₂)ₙOR¹⁰, S(CH₂)ₙSR¹⁰ O(CH₂CH₂O)ₘCH₂CH₂OR¹⁰, O(CH₂CH₂O)ₘCH₂CH₂NHR¹⁰, NH(CH₂CH₂NH)ₘCH₂CH₂NHR¹⁰; Q-R¹⁰, O-Q-R¹⁰ N-Q-R¹⁰, S-Q-R¹⁰.
X is 5-100, chosen to comply with a length for an antagomir described herein.
R⁷ is H; or is together combined with R⁴, R⁵, or R⁶ to form an [-O-CH₂-] covalently bound bridge between the sugar 2' and 4' carbons.
R⁸ is alkyl, cycloalkyl, aryl, aralkyl, heterocyclyl, heteroaryl, amino acid, or sugar; R⁹ is NH₂, alkylamino, dialkylamino, heterocyclyl, arylamino, diaryl amino, heteroaryl amino, diheteroaryl amino, or amino acid; and R¹⁰ is H; fluorophore (pyrene, TAMRA, fluorescein, Cy3 or Cy5 dyes); sulfur, silicon, boron or ester protecting group; intercalating agents (*e.g.* acridines), cross-linkers *(e.g.* psoralene, mitomycin C), porphyrins (TPPC4,texaphyrin, Sapphyrin), polycyclic aromatic hydrocarbons *(e.g.,* phenazine, dihydrophenazine), artificial endonucleases *(e.g.* EDTA), lipophilic carriers (cholesterol, cholic acid, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine)and peptide conjugates (*e.g*., antennapedia peptide, Tat peptide), alkylating agents, phosphate, amino, mercapto, PEG *(e.g.,* PEG-40K), MPEG, [MPEG]₂, polyamino; alkyl, cycloalkyl, aryl, aralkyl, heteroaryl; radiolabelled markers, enzymes, haptens (*e.g*. biotin), transport/absorption facilitators *(e.g.,* aspirin, vitamin E, folic acid), synthetic ribonucleases *(e.g.,* imidazole, bisimidazole, histamine, imidazole clusters, acridine-imidazole conjugates, Eu3+ complexes of tetraazamacrocycles); or an oligonucleotide agent. M is 0-1,000,000, and n is 0-20. Q is a spacer selected from the group consisting of abasic sugar, amide, carboxy, oxyamine, oxyimine, thioether, disulfide, thiourea, sulfonamide, or morpholino, biotin or fluorescein reagents.

Preferred oligonucleotide agents in which the entire phosphate group has been replaced have the following structure (see Formula 3 below):

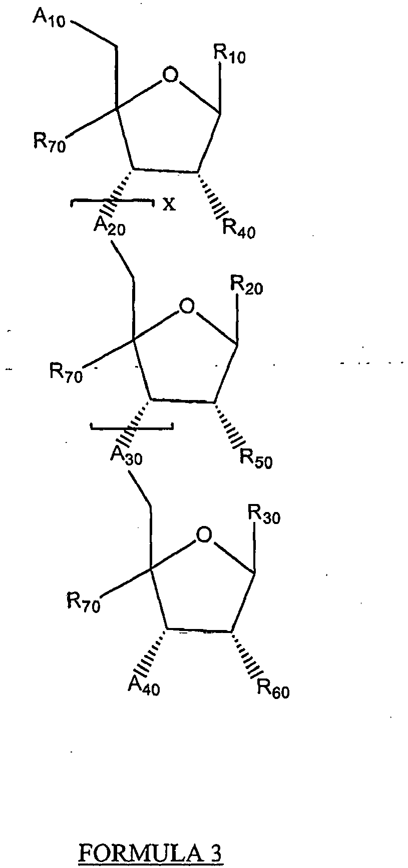

Referring to Formula 3, A¹⁰-A⁴⁰ is L-G-L; A¹⁰ and/or A⁴⁰ may be absent, in which L is a linker, wherein one or both L may be present or absent and is selected from the group consisting of CH₂(CH₂)_{g}; N(CH₂)_{g}; O(CH₂)_{g}; S(CH₂)_{g}. G is a functional group selected from the group consisting of siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino.

R¹⁰, R²⁰, and R³⁰ are each, independently, H, *(i.e.* abasic nucleotides), adenine, guanine, cytosine and uracil, inosine, thymine, xanthine, hypoxanthine, nubularine, tubercidine, isoguanisine, 2-aminoadenine, 6-methyl and other alkyl derivatives of adenine and guanine, 2-propyl and other alkyl derivatives of adenine and guanine, 5-halouracil and cytosine, 5-propynyl uracil and cytosine, 6-azo uracil, cytosine and thymine, 5-uracil (pseudouracil), 4-thiouracil, 5-halouracil, 5-(2-aminopropyl)uracil, 5-amino allyl uracil, 8-halo, amino, thiol, thioalkyl, hydroxyl and other 8-substituted adenines and guanines, 5-trifluoromethyl and other 5-substituted uracils and cytosines, 7-methylguanine, 5-substituted pyrimidines, 6-azapyrimidines and N-2, N-6 and O-6 substituted purines, including 2-aminopropyladenine, 5-propynyluracil and 5-propynylcytosine, dihydrouracil, 3-deaza-5-azacytosine, 2-aminopurine, 5-alkyluracil, 7-alkylguanine, 5-alkyl cytosine,7-deazaadenine, 7-deazaguanine, N6, N6-dimethyladenine, 2,6-diaminopurine, 5-amino-allyl-uracil, N3-methyluracil substituted 1,2,4-triazoles, 2-pyridinone, 5-nitroindole, 3-nitropyrrole, 5-methoxyuracil, uracil-5-oxyacetic acid, 5-methoxycarbonylmethyluracil, 5-methyl-2-thiouracil, 5-methoxycarbonylmethyl-2-thiouracil, 5-methylaminomethyl-2-thiouracil, 3-(3-amino-3carboxypropyl)uracil, 3-methylcytosine, 5-methylcytosine, N⁴-acetyl cytosine, 2-thiocytosine, N6-methyladenine, N6-isopentyladenine, 2-methylthio-N6-isopentenyladenine, N-methylguanines, or O-alkylated bases.

R⁴⁰, R⁵⁰, and R⁶⁰ are each, independently, OR⁸, O(CH₂CH₂O)ₘCH₂CH₂OR⁸; O(CH₂)ₙR⁹; O(CH₂)ₙOR⁹, H; halo; NH₂; NHR⁸; N(R⁸)₂; NH(CH₂CH₂NH)ₘCH₂CH₂R⁹; NHC(O)R⁸;; cyano; mercapto, SR⁷; alkyl-thio-alkyl; alkyl, aralkyl, cycloalkyl, aryl, heteroaryl, alkenyl, alkynyl, each of which may be optionally substituted with halo, hydroxy, oxo, nitro, haloalkyl, alkyl, alkaryl, aryl, aralkyl, alkoxy, aryloxy, amino, alkylamino, dialkylamino, heterocyclyl, arylamino, diaryl amino, heteroaryl amino, diheteroaryl amino, acylamino, alkylcarbamoyl, arylcarbamoyl, aminoalkyl, alkoxycarbonyl, carboxy, hydroxyalkyl, alkanesulfonyl, alkanesulfonamido, arenesulfonamido, aralkylsulfonamido, alkylcarbonyl, acyloxy, cyano, and ureido groups; or R⁴⁰, R⁵⁰, or R⁶⁰ together combine with R⁷⁰ to form an [-O-CH₂-] covalently bound bridge between the sugar 2' and 4' carbons.

X is 5-100 or chosen to comply with a length for an antagomir described herein.

R⁷⁰ is H; or is together combined with R⁴⁰, R⁵⁰, or R⁶⁰ to form an [-O-CH₂-] covalently bound bridge between the sugar 2' and 4' carbons.

R⁸ is alkyl, cycloalkyl, aryl, aralkyl, heterocyclyl, heteroaryl, amino acid, or sugar; and R⁹ is NH₂, alkylamino, dialkylamino, heterocyclyl, arylamino, diaryl amino, heteroaryl amino, diheteroaryl amino, or amino acid. M is 0-1,000,000, n is 0-20, and g is 0-2.

Preferred nucleoside surrogates have the following structure (see Formula 4 below):

SLR¹⁰⁰-(M-SLR²⁰⁰)ₓ-M-SLR³⁰⁰ FORMULA 4

S is a nucleoside surrogate selected from the group consisting of mophilino, cyclobutyl, pyrrolidine and peptide nucleic acid. L is a linker and is selected from the group consisting of CH₂(CH₂)_{g}; N(CH₂)_{g}; O(CH₂)_{g}; S(CH₂)_{g}; -C(O)(CH₂)ₙ-or may be absent. M is an amide bond; sulfonamide; sulfinate; phosphate group; modified phosphate group as described herein; or may be absent.

R¹⁰⁰, R²⁰⁰, and R³⁰⁰ are each, independently, H (*i.e.,* abasic nucleotides), adenine, guanine, cytosine and uracil, inosine, thymine, xanthine, hypoxanthine, nubularine, tubercidine, isoguanisine, 2-aminoadenine, 6-methyl and other alkyl derivatives of adenine and guanine, 2-propyl and other alkyl derivatives of adenine and guanine, 5-halouracil and cytosine, 5-propynyl uracil and cytosine, 6-azo uracil, cytosine and thymine, 5-uracil (pseudouracil), 4-thiouracil, 5-halouracil, 5-(2-aminopropyl)uracil, 5-amino allyl uracil, 8-halo, amino, thiol, thioalkyl, hydroxyl and other 8-substituted adenines and guanines, 5-trifluoromethyl and other 5-substituted uracils and cytosines, 7-methylguanine, 5-substituted pyrimidines, 6-azapyrimidines and N-2, N-6 and O-6 substituted purines, including 2-aminopropyladenine, 5-propynyluracil and 5-propynylcytosine, dihydrouracil, 3-deaza-5-azacytosine, 2-aminopurine, 5-alkyluracil, 7-alkylguanine, 5-alkyl cytosine,7-deazaadenine, 7-deazaguanine, N6, N6-dimethyladenine, 2,6-diaminopurine, 5-amino-allyl-uracil, N3-methyluracil substituted 1, 2, 4,-triazoles, 2-pyridinones, 5-nitroindole, 3-nitropyrrole, 5-methoxyuracil, uracil-5-oxyacetic acid, 5-methoxycarbonylmethyluracil, 5-methyl-2-thiouracil, 5-methoxycarbonylmethyl-2-thiouracil, 5-methylaminomethyl-2-thiouracil, 3-(3-amino-3carboxypropyl)uracil, 3-methylcytosine, 5-methylcytosine, N⁴-acetyl cytosine, 2-thiocytosine, N6-methyladenine, N6-isopentyladenine, 2-methylthio-N6-isopentenyladenine, N-methylguanines, or O-alkylated bases.

X is 5-100, or chosen to comply with a length for an antagomir described herein; and g is 0-2.

An antagomir can include an internucleotide linkage (*e.g***.**, the chiral phosphorothioate linkage) useful for increasing nuclease resistance. In addition, or in the alternative, an antagomir can include a ribose mimic for increased nuclease resistance. Exemplary internucleotide linkages and ribose mimics for increased nuclease resistance are described in co-owned PCT Application No. PCT/US2004/07070 filed on March 8, 2004.

An antagomir can include ligand-conjugated monomer subunits and monomers for oligonucleotide synthesis. Exemplary monomers are described in co-owned U.S. Application No. 10/916,185, filed on August 10, 2004.

An antagomir can have a ZXY structure, such as is described in co-owned PCT Application No. PCT/US2004/07070 filed on March 8, 2004.

An antagomir can be complexed with an amphipathic moiety. Exemplary amphipathic moieties for use with oligonucleotide agents are described in co-owned PCT Application No. PCT/US2004/07070 filed on March 8, 2004.

In another embodiment, the antagomir can be complexed to a delivery agent that features a modular complex. The complex can include a carrier agent linked to one or more of (preferably two or more, more preferably all three of): (a) a condensing agent *(e.g.,* an agent capable of attracting, *e.g.,* binding, a nucleic acid, *e.g.,* through ionic or electrostatic interactions); (b) a fusogenic agent *(e.g.,* an agent capable of fusing and/or being transported through a cell membrane); and (c) a targeting group, *e.g.,* a cell or tissue targeting agent, *e.g.,* a lectin, glycoprotein, lipid or protein, *e.g.,* an antibody, that binds to a specified cell type. oligonucleotide agents complexed to a delivery agent are described in co-owned PCT Application No. PCT/US2004/07070 filed on March 8, 2004.

### Enhanced nuclease resistance

An antagomir, such as a single-stranded oligonucleotide agent, featured in the invention can have enhanced resistance to nucleases.

For increased nuclease resistance and/or binding affinity to the target, an oligonucleotide agent, e.g., the oligonucleotide agent, can include, for example, 2'-modified ribose units and/or phosphorothioate linkages. *E.g.,* the 2' hydroxyl group (OH) can be modified or replaced with a number of different "oxy" or "deoxy" substituents.

Examples of "oxy"-2' hydroxyl group modifications include alkoxy or aryloxy (OR, *e.g.,* R = H, alkyl, cycloalkyl, aryl, aralkyl, heteroaryl or sugar); polyethyleneglycols (PEG), O(CH₂CH₂O)ₙCH₂CH₂OR; "locked" nucleic acids (LNA) in which the 2' hydroxyl is connected, *e.g.,* by a methylene bridge, to the 4' carbon of the same ribose sugar; amine, O-AMINE and aminoalkoxy, O(CH₂)ₙAMINE, (*e.g*., AMINE = NH₂; alkylamino, dialkylamino, heterocyclyl amino, arylamino, diaryl amino, heteroaryl amino, or diheteroaryl amino, ethylene diamine, polyamino). It is noteworthy that oligonucleotides containing only the methoxyethyl group (MOE), (OCH₂CH₂OCH₃, a PEG derivative), exhibit nuclease stabilities comparable to those modified with the robust phosphorothioate modification.

"Deoxy" modifications include hydrogen (*i.e.* deoxyribose sugars); halo *(e.g.,* fluoro); amino (*e.g.* NH₂; alkylamino, dialkylamino, heterocyclyl, arylamino, diaryl amino, heteroaryl amino, diheteroaryl amino, or amino acid); NH(CH₂CH₂NH)ₙCH₂CH₂-AMINE (AMINE = NH₂; alkylamino, dialkylamino, heterocyclyl amino, arylamino, diaryl amino, heteroaryl amino,or diheteroaryl amino), -NHC(O)R (R = alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with *e.g.,* an amino functionality.

Preferred substitutents are 2'-methoxyethyl, 2'-OCH3, 2'-O-allyl, 2'-C- allyl, and 2'-fluoro.

One way to increase resistance is to identify cleavage sites and modify such sites to inhibit cleavage, as described in co-owned U.S. Application No. 60/559,917, filed on May 4, 2004. For example, the dinucleotides 5'-UA-3', 5'-UG-3', 5'-CA-3', 5'-UU-3', or 5'-CC-3' can serve as cleavage sites. Enhanced nuclease resistance can therefore be achieved by modifying the 5' nucleotide, resulting, for example, in at least one 5'-uridine-adenine-3' (5'-UA-3') dinucleotide wherein the uridine is a 2'-modified nucleotide; at least one 5'-uridine-guanine-3' (5'-UG-3') dinucleotide, wherein the 5'-uridine is a 2'-modified nucleotide; at least one 5'-cytidine-adenine-3' (5'-CA-3') dinucleotide, wherein the 5'-cytidine is a 2'-modified nucleotide; at least one 5'-uridine-uridine-3' (5'-UU-3') dinucleotide, wherein the 5'-uridine is a 2'-modified nucleotide; or at least one 5'-cytidine-cytidine-3' (5'-CC-3') dinucleotide, wherein the 5'-cytidine is a 2'-modified nucleotide. The antagomir can include at least 2, at least 3, at least 4 or at least 5 of such dinucleotides. In certain embodiments, all the pyrimidines of an antagomir carry a 2'-modification, and the antagomir therefore has enhanced resistance to endonucleases.

To maximize nuclease resistance, the 2' modifications can be used in combination with one or more phosphate linker modifications (*e.g.,* phosphorothioate). The so-called "chimeric" oligonucleotides are those that contain two or more different modifications.

The inclusion of furanose sugars in the oligonucleotide backbone can also decrease endonucleolytic cleavage. An antagomir can be further modified by including a 3' cationic group, or by inverting the nucleoside at the 3'-terminus with a 3'-3' linkage. In another alternative, the 3'-terminus can be blocked with an aminoalkyl group, e.g., a 3' C5-aminoalkyl dT. Other 3' conjugates can inhibit 3'-5' exonucleolytic cleavage. While not being bound by theory, a 3' conjugate, such as naproxen or ibuprofen, may inhibit exonucleolytic cleavage by sterically blocking the exonuclease from binding to the 3'-end of oligonucleotide. Even small alkyl chains, aryl groups, or heterocyclic conjugates or modified sugars (D-ribose, deoxyribose, glucose etc.) can block 3'-5'-exonucleases.

Similarly, 5' conjugates can inhibit 5'-3' exonucleolytic cleavage. While not being bound by theory, a 5' conjugate, such as naproxen or ibuprofen, may inhibit exonucleolytic cleavage by sterically blocking the exonuclease from binding to the 5'-end of oligonucleotide. Even small alkyl chains, aryl groups, or heterocyclic conjugates or modified sugars (D-ribose, deoxyribose, glucose etc.) can block 3'-5'-exonucleases.

Thus, an antagomir can include modifications so as to inhibit degradation, e.g., by nucleases, e.g., endonucleases or exonucleases, found in the body of a subject. These monomers are referred to herein as NRMs, or Nuclease Resistance promoting Monomers, the corresponding modifications as NRM modifications. In many cases these modifications will modulate other properties of the antagomir as well, e.g., the ability to interact with a protein, e.g., a transport protein, e.g., serum albumin, or a member of the RISC, or the ability of the antagomir to form a duplex with another sequence, e.g., a target molecule, such as an miRNA or pre-miRNA.

One or more different NRM modifications can be introduced into an antagomir or into a sequence of an oligonucleotide agent. An NRM modification can be used more than once in a sequence or in an oligonucleotide agent.

NRM modifications include some which can be placed only at the terminus and others which can go at any position. Some NRM modifications that can inhibit hybridization are preferably used only in terminal regions, and more preferably not at the cleavage site or in the cleavage region of the oligonucleotide agent.

Modifications which interfere with or inhibit endonuclease cleavage should not be inserted in the region which is subject to RISC mediated cleavage, e.g., the cleavage site or the cleavage region (As described in Elbashir et al., Genes and Dev. 15: 188, 2001). Cleavage of the target occurs about in the middle of a 20 or 21 nt oligonucleotide agent, or about 10 or 11 nucleotides upstream of the first nucleotide on the target mRNA which is complementary to the oligonucleotide agent. As used herein, cleavage site refers to the nucleotides on either side of the site of cleavage, on the target mRNA or on the antagomir which hybridizes to it. Cleavage region means the nucleotides within 1, 2, or 3 nucleotides of the cleavage site, in either direction.

Such modifications can be introduced into the terminal regions, e.g., at the terminal position or with 2, 3, 4, or 5 positions of the terminus, of a sequence which targets or a sequence which does not target a sequence in the subject.

### Delivery of single-stranded oligonucleotide agents to tissues and cells

### Formulation

The single-stranded oligonucleotide agents described herein can be formulated for administration to a subject.

For ease of exposition, the formulations, compositions, and methods in this section are discussed largely with regard to unmodified oligonucleotide agents. It should be understood, however, that these formulations, compositions, and methods can be practiced with other oligonucleotide agents, *e.g*., modified oligonucleotide agents, and such practice is within the invention.

A formulated antagomir composition can assume a variety of states. In some examples, the composition is at least partially crystalline, uniformly crystalline, and/or anhydrous (*e.g.*, less than 80, 50, 30,20, or 10% water). In another example, the antagomir is in an aqueous phase, *e.g.,* in a solution that includes water, this form being the preferred form for administration via inhalation.

The aqueous phase or the crystalline compositions can be incorporated into a delivery vehicle, *e.g*., a liposome (particularly for the aqueous phase), or a particle *(e.g.,* a microparticle as can be appropriate for a crystalline composition). Generally, the antagomir composition is formulated in a manner that is compatible with the intended method of administration.

An antagomir preparation can be formulated in combination with another agent, *e.g.,* another therapeutic agent or an agent that stabilizes an oligonucleotide agent, *e.g.,* a protein that complexes with the oligonucleotide agent. Still other agents include chelators, *e.g.,* EDTA *(e.g.,* to remove divalent cations such as Mg²⁺), salts, RNAse inhibitors (*e.g.*, a broad specificity RNAse inhibitor such as RNAsin) and so forth.

In one embodiment, the antagomir preparation includes another antagomir, *e.g.,* a second antagomir that can down-regulate expression of a second gene. Still other preparations can include at least three, five, ten, twenty, fifty, or a hundred or more different oligonucleotide species. In some embodiments, the agents are directed to the same target nucleic acid but different target sequences. In another embodiment, each antagomir is directed to a different target. In one embodiment the antagomir preparation includes a double stranded RNA that targets an RNA (e.g., an mRNA) for donwregulation by an RNAi silencing mechanism.

### Treatment Methods and Routes of Delivery

A composition that includes an antagomir featured in the invention, e.g., an antagomir that targets an miRNA or pre-miRNA (e.g., miR-122, miR-16, miR-192, or miR-194) can be delivered to a subject by a variety of routes. Exemplary routes include inhalation, intrathecal, parenchymal, intravenous, nasal, oral, and ocular delivery.

An antagomir can be incorporated into pharmaceutical compositions suitable for administration. For example, compositions can include one or more oligonucleotide agents and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The pharmaceutical compositions featured in the invention may be administered in a number of ways depending upon whether local or systemic treatment is desired and upon the area to be treated. Administration may be topical (including ophthalmic, intranasal, transdermal, intrapulmonary), oral or parenteral. Parenteral administration includes intravenous drip, subcutaneous, intraperitoneal or intramuscular injection, or intrathecal or intraventricular administration.

In general, delivery of an antagomir featured in the invention directs the agent to the site of infection in a subject. The preferred means of delivery is through local administration directly to the site of infection, or by systemic administration, e.g. parental administration.

Formulations for direct injection and parenteral administration are well known in the art. Such formulations may include sterile aqueous solutions which may also contain buffers, diluents and other suitable additives. For intravenous use, the total concentration of solutes should be controlled to render the preparation isotonic.

*Administration of Oligonucleotide Agents* A patient who has been diagnosed with a disorder characterized by unwanted miRNA expression (e.g., unwanted expression of miR-122, miR-16, miR-192, or miR-194) can be treated by administration of an antagomir described herein to block the negative effects of the miRNA, thereby alleviating the symptoms associated with the unwanted miRNA expression. Similarly, a human who has or is at risk for deleveloping a disorder characterized by underexpression of a gene that is regulated by an miRNA can be treated by the administration of an antagomir that targets the miRNA. For example, a human diagnosed with hemolytic anemia, and who carries a mutation in the aldolase A gene, expresses a compromised form of the enzyme. The patient can be administered an antagomir that targets endogenous miR-122, which binds aldolase A RNA *in vivo,* presumably to downregulate translation of the aldolase A mRNA and consequently downregulate aldolase A protein levels. Administration of an antagomir that targets the endogenous miR-122 in a patient having hemolytic anemia will decrease miR-122 activity, which will result in the upregulation of aldolase A expression and an increase in aldolase A protein levels. Although the enzyme activity of the mutant aldolase A is suboptimal, an increase in protein levels may be sufficient to relieve the disease symptoms. A human who has or who is at risk for developing arthrogryposis multiplex congenital, pituitary ectopia, rhabdomyolysis, or hyperkalemia, or who suffers from a myopathic symptom, is also a suitable candidate for treatment with an antagomir that targets miR-122. A human who carries a mutation in the aldolase A gene can be a candidate for treatment with an antagomir that targets miR-122. A human who carries a mutation in the aldolase A gene can have a symptom characterizing aldolase A deficiency including growth and developmental retardation, midfacial hypoplasia, and hepatomegaly.

In another example, a human who has or who is at risk for developing a disorder associated with overexpression of a gene regulated by an miRNA or by an miRNA deficiency, e.g., an miR-122, miR-16, miR-192, or miR-194 deficiency, can be treated by the administration of an antagomir, such as a single-stranded oligonucleotide agent, that is substantially identical to the deficient miRNA.

The single-stranded oligonucleotide agents featured in the invention can be administered systemically, *e.g.,* orally or by intramuscular injection or by intravenous injection, in admixture with a pharmaceutically acceptable carrier adapted for the route of administration. An antagomir can include a delivery vehicle, such as liposomes, for administration to a subject, carriers and diluents and their salts, and/or can be present in pharmaceutically acceptable formulations. Methods for the delivery of nucleic acid molecules are described in Akhtar et al., Trends in Cell Bio. 2:139, 1992; Delivery Strategies for Antisense Oligonucleotide Therapeutics, ed. Akhtar, 1995; Maurer et al., Mol. Membr. Biol., 16:129, 1999; Hofland and Huang, Handb. Exp. Pharmacol. 137:165, 1999; and Lee et al., ACS Symp. Ser. 752:184, 2000. Beigelman et al., U.S. Pat. No. 6,395,713 and Sullivan et al., PCT WO 94/02595 further describe the general methods for delivery of nucleic acid molecules. Nucleic acid molecules can be administered to cells by a variety of methods known to those of skill in the art, including, but not restricted to, encapsulation in liposomes, by ionophoresis, or by incorporation into other vehicles, such as hydrogels, cyclodextrins (see for example Gonzalez et al., Bioconjugate Chem. 10:1068, 1999), biodegradable nanocapsules, and bioadhesive microspheres, or by proteinaceous vectors (O'Hare and Normand, International PCT Publication No. WO 00/53722).

In the present methods, the antagomir can be administered to the subject either as a naked oligonucleotide agent, in conjunction with a delivery reagent, or as a recombinant plasmid or viral vector which expresses the oligonucleotide agent. Preferably, the antagomir is administered as a naked oligonucleotide agent.

An antagomir featured in the invention can be administered to the subject by any means suitable for delivering the agent to the cells of the tissue at or near the area of unwanted target nucleic acid expression (e.g., target miRNA or pre-miRNA expression). For example, an antagomir that targets miR-122 can be delivered directly to the liver, or can be conjugated to a molecule that targets the liver. Exemplary delivery methods include administration by gene gun, electroporation, or other suitable parenteral administration route.

Suitable enteral administration routes include oral delivery.

Suitable parenteral administration routes include intravascular administration (e.g., intravenous bolus injection, intravenous infusion, intra-arterial bolus injection, intra-arterial infusion and catheter instillation into the vasculature); peri- and intra-tissue injection (*e.g*., intraocular injection, intra-retinal injection, or sub-retinal injection); subcutaneous injection or deposition including subcutaneous infusion (such as by osmotic pumps); direct application to the area at or near the site of neovascularization, for example by a catheter or other placement device (*e.g*., a retinal pellet or an implant comprising a porous, non-porous, or gelatinous material).

An antagomir featured in the invention can be delivered using an intraocular implant. Such implants can be biodegradable and/or biocompatible implants, or may be non-biodegradable implants. The implants may be permeable or impermeable to the active agent, and may be inserted into a chamber of the eye, such as the anterior or posterior chambers, or may be implanted in the sclera, transchoroidal space, or an avascularized region exterior to the vitreous. In a preferred embodiment, the implant may be positioned over an avascular region, such as on the sclera, so as to allow for transscleral diffusion of the drug to the desired site of treatment, *e.g.,* the intraocular space and macula of the eye. Furthermore, the site of transscleral diffusion is preferably in proximity to the macula.

An antagomir featured in the invention can also be administered topically, for example, by patch or by direct application to the eye, or by iontophoresis. Ointments, sprays, or droppable liquids can be delivered by ocular delivery systems known in the art such as applicators or eyedroppers. The compositions can be administered directly to the surface of the eye or to the interior of the eyelid. Such compositions can include mucomimetics such as hyaluronic acid, chondroitin sulfate, hydroxypropyl methylcellulose or poly(vinyl alcohol), preservatives such as sorbic acid, EDTA or benzylchronium chloride, and the usual quantities of diluents and/or carriers.

An antagomir featured in the invention may be provided in sustained release compositions, such as those described in, for example, U.S. Patent Nos. 5,672,659 and 5,595,760. The use of immediate or sustained release compositions depends on the nature of the condition being treated. If the condition consists of an acute or over-acute disorder, treatment with an immediate release form will be preferred over a prolonged release composition. Alternatively, for certain preventative or long-term treatments, a sustained release composition may be appropriate.

An antagomir can be injected into the interior of the eye, such as with a needle or other delivery device.

An antagomir featured in the invention can be administered in a single dose or in multiple doses. Where the administration of the antagomir is by infusion, the infusion can be a single sustained dose or can be delivered by multiple infusions. Injection of the agent can be directly into the tissue at or near the site of aberrant or unwanted target gene expression (e.g., aberrant or unwanted miRNA or pre-miRNA expression). Multiple injections of the agent can be made into the tissue at or near the site.

Dosage levels on the order of about 1 µg/kg to 100 mg/kg of body weight per administration are useful in the treatment of a disease. One skilled in the art can also readily determine an appropriate dosage regimen for administering the antagomir of the invention to a given subject. For example, the antagomir can be administered to the subject once, *e.g.,* as a single injection or deposition at or near the site on unwanted target nucleic acid expression. Alternatively, the antagomir can be administered once or twice daily to a subject for a period of from about three to about twenty-eight days, more preferably from about seven to about ten days. In a preferred dosage regimen, the antagomir is injected at or near a site of unwanted target nucleic acid expression once a day for seven days. Where a dosage regimen comprises multiple administrations, it is understood that the effective amount of antagomir administered to the subject can include the total amount of antagomir administered over the entire dosage regimen. One skilled in the art will appreciate that the exact individual dosages may be adjusted somewhat depending on a variety of factors, including the specific antagomir being administered, the time of administration, the route of administration, the nature of the formulation, the rate of excretion, the particular disorder being treated, the severity of the disorder, the pharmacodynamics of the oligonucleotide agent, and the age, sex, weight, and general health of the patient. Wide variations in the necessary dosage level are to be expected in view of the differing efficiencies of the various routes of administration. For instance, oral administration generally would be expected to require higher dosage levels than administration by intravenous or intravitreal injection. Variations in these dosage levels can be adjusted using standard empirical routines of optimization, which are well-known in the art. The precise therapeutically effective dosage levels and patterns are preferably determined by the attending physician in consideration of the above-identified factors.

In addition to treating pre-existing diseases or disorders, oligonucleotide agents featured in the invention (*e.g.,* single-stranded oligonucleotide agents targeting miR-122) can be administered prophylactically in order to prevent or slow the onset of a particular disease or disorder. In prophylactic applications, an antagomir is administered to a patient susceptible to or otherwise at risk of a particular disorder, such as disorder associated with aberrant or unwanted expression of an miRNA or pre-miRNA.

The oligonucleotide agents featured by the invention are preferably formulated as pharmaceutical compositions prior to administering to a subject, according to techniques known in the art. Pharmaceutical compositions featured in the present invention are characterized as being at least sterile and pyrogen-free. As used herein, "pharmaceutical formulations" include formulations for human and veterinary use. Methods for preparing pharmaceutical compositions are within the skill in the art, for example as described in Remington's Pharmaceutical Science, 18th ed., Mack Publishing Company, Easton, Pa. (1990), and The Science and Practice of Pharmacy, 2003, Gennaro *et al.*

The present pharmaceutical formulations include an antagomir featured in the invention (*e.g.*, 0.1 to 90% by weight), or a physiologically acceptable salt thereof, mixed with a physiologically acceptable carrier medium. Preferred physiologically acceptable carrier media are water, buffered water, normal saline, 0.4% saline, 0.3% glycine, hyaluronic acid and the like.

Pharmaceutical compositions featured in the invention can also include conventional pharmaceutical excipients and/or additives. Suitable pharmaceutical excipients include stabilizers, antioxidants, osmolality adjusting agents, buffers, and pH adjusting agents. Suitable additives include physiologically biocompatible buffers (*e.g.,* tromethamine hydrochloride), additions of chelants (such as, for example, DTPA or DTPA-bisamide) or calcium chelate complexes (as for example calcium DTPA, CaNaDTPA-bisamide), or, optionally, additions of calcium or sodium salts (for example, calcium chloride, calcium ascorbate, calcium gluconate or calcium lactate). Pharmaceutical compositions can be packaged for use in liquid form, or can be lyophilized.

For solid compositions, conventional non-toxic solid carriers can be used; for example, pharmaceutical grades of mannitol, lactose, starch, magnesium stearate, sodium saccharin, talcum, cellulose, glucose, sucrose, magnesium carbonate, and the like.

For example, a solid pharmaceutical composition for oral administration can include any of the carriers and excipients listed above and 10-95%, preferably 25%-75%, of one or more single-stranded oligonucleotide agents featured in the invention.

By "pharmaceutically acceptable formulation" is meant a composition or formulation that allows for the effective distribution of the nucleic acid molecules of the instant invention in the physical location most suitable for their desired activity. Non-limiting examples of agents suitable for formulation with the nucleic acid molecules of the instant invention include: P-glycoprotein inhibitors (such as PluronicP85), which can enhance entry of drugs into the CNS (Jolliet-Riant and Tillement, Fundam. Clin. Pharmacol. 13:16, 1999); biodegradable polymers, such as poly (DL-lactide-coglycolide) microspheres for sustained release delivery. Other non-limiting examples of delivery strategies for the nucleic acid molecules featured in the instant invention include material described in Boado et al., J. Pharm. Sci. 87:1308, 1998; Tyler et al., FEBS Lett. 421:280, 1999; Pardridge et al., PNAS USA. 92:5592, 1995; Boado, Adv. Drug Delivery Rev. 15:73, 1995; Aldrian-Herrada et al., Nucleic Acids Res. 26:4910, 1998; and Tyler et al., PNAS USA 96:7053, 1999.

The invention also features the use of a composition that includes surface-modified liposomes containing poly (ethylene glycol) lipids (PEG-modified, or long-circulating liposomes or stealth liposomes). These formulations offer a method for increasing the accumulation of drugs in target tissues. This class of drug carriers resists opsonization and elimination by the mononuclear phagocytic system (MPS or RES), thereby enabling longer blood circulation times and enhanced tissue exposure for the encapsulated drug (Lasic et al., Chem. Rev. 95:2601, 1995; Ishiwata et al., Chem.Phare. Bull. 43:1005, 1995).

Such liposomes have been shown to accumulate selectively in tumors, presumably by extravasation and capture in the neovascularized target tissues (Lasic et al., Science 267:1275, 1995; Oku et al., Biochim. Biophys. Acta 1238:86, 1995). The long-circulating liposomes enhance the pharmacokinetics and pharmacodynamics of DNA and RNA, particularly compared to conventional cationic liposomes which are known to accumulate in tissues of the MPS (Liu et al., J. Biol. Chem. 42:24864, 1995; Choi et al., International PCT Publication No. WO 96/10391; Ansell et al., International PCT Publication No. WO 96/10390 ; Holland et al., International PCT Publication No. WO 96/10392). Long-circulating liposomes are also likely to protect drugs from nuclease degradation to a greater extent compared to cationic liposomes, based on their ability to avoid accumulation in metabolically aggressive MPS tissues such as the liver and spleen.

The present invention also features compositions prepared for storage or administration that include a pharmaceutically effective amount of the desired oligonucleotides in a pharmaceutically acceptable carrier or diluent. Acceptable carriers or diluents for therapeutic use are well known in the pharmaceutical art, and are described, for example, in Remington's Pharmaceutical Sciences, Mack Publishing Co. (A. R. Gennaro edit. 1985). For example, preservatives, stabilizers, dyes and flavoring agents can be provided. These include sodium benzoate, sorbic acid and esters of p-hydroxybenzoic acid. In addition, antioxidants and suspending agents can be used.

The nucleic acid molecules of the present invention can also be administered to a subject in combination with other therapeutic compounds to increase the overall therapeutic effect. The use of multiple compounds to treat an indication can increase the beneficial effects while reducing the presence of side effects.

Alternatively, certain single-stranded oligonucleotide agents featured in the instant invention can be expressed within cells from eukaryotic promoters (*e.g*., Izant and Weintraub, Science 229:345, 1985; McGarry and Lindquist, Proc. Natl. Acad. Sci. USA 83:399, 1986; Scanlon et al., Proc.Natl. Acad. Sci. USA 88:10591, 1991; Kashani-Sabet et al., Antisense Res. Dev. 2:3, 1992; Dropulic et al., J. Virol. 66:1432, 1992; Weerasinghe et al., J. Virol. 65:5531, 1991; Ojwang et al., Proc. Natl. Acad. Sci. USA 89:10802, 1992; Chen et al., Nucleic Acids Res. 20:4581, 1992; Sarver et al., Science 247:1222, 1990; Thompson et al., Nucleic Acids Res. 23:2259, 1995; Good et al., Gene Therapy 4:45, 1997). Those skilled in the art realize that any nucleic acid can be expressed in eukaryotic cells from the appropriate DNA/RNA vector. The activity of such nucleic acids can be augmented by their release from the primary transcript by a enzymatic nucleic acid (Draper et al., PCT WO 93/23569, and Sullivan et al., PCT WO 94/02595; Ohkawa et al., Nucleic Acids Symp. Ser. 27:156, 1992; Taira et al., Nucleic Acids Res. 19:5125, 1991; Ventura et al., Nucleic Acids Res. 21:3249, 1993; Chowrira et al., J. Biol. Chem. 269:25856, 1994).

In another aspect of the invention, RNA molecules of the present invention can be expressed from transcription units (see for example Couture et al., Trends in Genetics 12:510, 1996) inserted into DNA or RNA vectors. The recombinant vectors can be DNA plasmids or viral vectors. Oligonucleotide agent-expressing viral vectors can be constructed based on, but not limited to, adeno-associated virus, retrovirus, adenovirus, or alphavirus. In another embodiment, pol III based constructs are used to express nucleic acid molecules of the invention (see for example Thompson, U. S. Pats. Nos. 5,902,880 and 6,146,886). The recombinant vectors capable of expressing the oligonucleotide agents can be delivered as described above, and can persist in target cells. Alternatively, viral vectors can be used that provide for transient expression of nucleic acid molecules. Such vectors can be repeatedly administered as necessary. Once expressed, the antagomir interacts with the target RNA (e.g., miRNA or pre-miRNA) and inhibits miRNA activity. In a preferred embodiment, the antagomir forms a duplex with the target miRNA, which prevents the miRNA from binding to its target mRNA, which results in increased translation of the target mRNA. Delivery of oligonucleotide agent-expressing vectors can be systemic, such as by intravenous or intra-muscular administration, by administration to target cells ex-planted from a subject followed by reintroduction into the subject, or by any other means that would allow for introduction into the desired target cell (for a review see Couture et al., Trends in Genetics 12:510, 1996).

The term "therapeutically effective amount" is the amount present in the composition that is needed to provide the desired level of drug in the subject to be treated to give the anticipated physiological response.

The term "physiologically effective amount" is that amount delivered to a subject to give the desired palliative or curative effect.

The term "pharmaceutically acceptable carrier" means that the carrier can be taken into the subject with no significant adverse toxicological effects on the subject.

The term "co-administration" refers to administering to a subject two or more single-stranded oligonucleotide agents. The agents can be contained in a single pharmaceutical composition and be administered at the same time, or the agents can be contained in separate formulation and administered serially to a subject. So long as the two agents can be detected in the subject at the same time, the two agents are said to be co-administered.

The types of pharmaceutical excipients that are useful as carrier include stabilizers such as human serum albumin (HSA), bulking agents such as carbohydrates, amino acids and polypeptides; pH adjusters or buffers; salts such as sodium chloride; and the like. These carriers may be in a crystalline or amorphous form or may be a mixture of the two.

Bulking agents that are particularly valuable include compatible carbohydrates, polypeptides, amino acids or combinations thereof. Suitable carbohydrates include monosaccharides such as galactose, D-mannose, sorbose, and the like; disaccharides, such as lactose, trehalose, and the like; cyclodextrins, such as 2-hydroxypropyl-.beta.-cyclodextrin; and polysaccharides, such as raffinose, maltodextrins, dextrans, and the like; alditols, such as mannitol, xylitol, and the like. A preferred group of carbohydrates includes lactose, threhalose, raffinose maltodextrins, and mannitol. Suitable polypeptides include aspartame. Amino acids include alanine and glycine, with glycine being preferred.

Suitable pH adjusters or buffers include organic salts prepared from organic acids and bases, such as sodium citrate, sodium ascorbate, and the like; sodium citrate is preferred.

*Dosage.* An antagomir can be administered at a unit dose less than about 75 mg per kg of bodyweight, or less than about 70, 60, 50, 40, 30, 20, 10, 5, 2, 1, 0.5, 0.1, 0.05, 0.01, 0.005, 0.001, or 0.0005 mg per kg of bodyweight, and less than 200 nmol of antagomir (e.g., about 4.4 x 10¹⁶ copies) per kg of bodyweight, or less than 1500, 750, 300, 150, 75, 15, 7.5, 1.5, 0.75, 0.15, 0.075, 0.015, 0.0075, 0.0015, 0:00075, 0.00015 nmol of antagomir per kg of bodyweight. The unit dose, for example, can be administered by injection (e.g., intravenous or intramuscular, intrathecally, or directly into an organ), inhalation, or a topical application.

Delivery of an antagomir directly to an organ (e.g., directly to the liver) can be at a dosage on the order of about 0.00001 mg to about 3 mg per organ, or preferably about 0.0001-0.001 mg per organ, about 0.03- 3.0 mg per organ, about 0.1-3.0 mg per organ or about 0.3-3.0 mg per organ.

The dosage can be an amount effective to treat or prevent a disease or disorder.

In one embodiment, the unit dose is administered less frequently than once a day, *e.g.,* less than every 2, 4, 8 or 30 days. In another embodiment, the unit dose is not administered with a frequency (*e.g.,* not a regular frequency). For example, the unit dose may be administered a single time. Because oligonucleotide agent-mediated silencing can persist for several days after administering the antagomir composition, in many instances, it is possible to administer the composition with a frequency of less than once per day, or, for some instances, only once for the entire therapeutic regimen.

In one embodiment, a subject is administered an initial dose, and one or more maintenance doses of an antagomir. The maintenance dose or doses are generally lower than the initial dose, *e.g.,* one-half less of the initial dose. A maintenance regimen can include treating the subject with a dose or doses ranging from 0.01 µg to 75 mg/kg of body weight per day, *e.g.,* 70, 60, 50, 40, 30, 20, 10, 5, 2, 1, 0.5, 0.1, 0.05, 0.01, 0.005, 0.001, or 0.0005 mg per kg of bodyweight per day. The maintenance doses are preferably administered no more than once every 5, 10, or 30 days. Further, the treatment regimen may last for a period of time which will vary depending upon the nature of the particular disease, its severity and the overall condition of the patient. In preferred embodiments the dosage may be delivered no more than once per day, *e.g.,* no more than once per 24, 36, 48, or more hours, *e.g.,* no more than once every 5 or 8 days. Following treatment, the patient can be monitored for changes in his condition and for alleviation of the symptoms of the disease state. The dosage of the compound may either be increased in the event the patient does not respond significantly to current dosage levels, or the dose may be decreased if an alleviation of the symptoms of the disease state is observed, if the disease state has been ablated, or if undesired side-effects are observed.

The effective dose can be administered in a single dose or in two or more doses, as desired or considered appropriate under the specific circumstances. If desired to facilitate repeated or frequent infusions, implantation of a delivery device, *e.g.,* a pump, semi-permanent stent *(e.g.,* intravenous, intraperitoneal, intracisternal or intracapsular), or reservoir may be advisable.

Following successful treatment, it may be desirable to have the patient undergo maintenance therapy to prevent the recurrence of the disease state, wherein the compound of the invention is administered in maintenance doses, ranging from 0.01 µg to 100 g per kg of body weight (see US 6,107,094).

The concentration of the antagomir composition is an amount sufficient to be effective in treating or preventing a disorder or to regulate a physiological condition in humans. The concentration or amount of antagomir administered will depend on the parameters determined for the agent and the method of administration, *e.g.* direct administration to the eye. For example, eye formulations tend to require much lower concentrations of some ingredients in order to avoid irritation or burning of the ocular tissues. It is sometimes desirable to dilute an oral formulation up to 10-100 times in order to provide a suitable ocular formulation.

Certain factors may influence the dosage required to effectively treat a subject, including but not limited to the severity of the disease or disorder, previous treatments, the general health and/or age of the subject, and other diseases present. It will also be appreciated that the effective dosage of the antagomir used for treatment may increase or decrease over the course of a particular treatment. Changes in dosage may result and become apparent from the results of diagnostic assays. For example, the subject can be monitored after administering an antagomir composition. Based on information from the monitoring, an additional amount of the antagomir composition can be administered.

Dosing is dependent on severity and responsiveness of the disease condition to be treated, with the course of treatment lasting from several days to several months, or until a cure is effected or a diminution of disease state is achieved. Optimal dosing schedules can be calculated from measurements of drug accumulation in the body of the patient. Persons of ordinary skill can easily determine optimum dosages, dosing methodologies and repetition rates. Optimum dosages may vary depending on the relative potency of individual compounds, and can generally be estimated based on EC₅₀s found to be effective in *in vitro* and *in vivo* animal models.

The invention is further illustrated by the following examples, which should not be construed as further limiting.

### EXAMPLES

### Example 1. Single stranded oligonucleotide agents inhibited miRNA activity.

Chemically-stabilized, cholesterol-conjugated single-stranded RNAs complementary to miRNAs were designed and synthesized. These single-stranded modified RNAs are referred to herein as "antagomirs" (see below). To explore the potential of these synthetic RNAs to silence endogenous miRNAs, antagomir-122 was designed to target miR-122, an miRNA expressed in the liver. The sequence of antagomir-122 is shown in Table 3. Antagomir-122 was administered to mice by intravenous injection in a small volume (0.2 ml, 80 mg/kg, 3 consecutive days) and normal pressure. Administration of antagomir-122 resulted in a striking reduction of endogenous miR-122 levels as detected by Northern blot analysis (FIG. 1A). Administration of unmodified single-stranded RNA (anti-122) had no effect on hepatic miR-122 expression levels (FIG. 1A), while injection of unconjugated, but chemically-stabilized single-stranded RNAs with partial (pS) or complete (fS) phosphorothioate backbone and 2'-O-methyl sugar modifications (anti-122fS, anti-122pS, see Table 3) led to an incomplete effect (Fig. 1A). The effects of antagomir-122 were found to be specific as animals injected with a control antagomir-122 derivative that harbored four mismatch mutations (mm-antagomir-122) had no effect on miR-122 expression in the liver. Furthermore, expression levels of miR-let7 and miR-22 were unaffected in antagomir-122 and mm-antagomir-122 treated mice, suggesting that silencing was miRNA-specific (FIG. 1B). The structure of the single stranded RNAs injected into mice is described in Table 3.

**Table 3. Antagomirs**

| RNA | Sequence | AL-SQ NO: | SEQ ID NO: |
|---|---|---|---|
| anti-122 | 5'-ACAAACACCAUUGUCACACUCCA-3' | 3033 | 40 |
| anti-122pS | 5'-as_{c}sₐaacaccauugucacacsᵤs_{c}s_{c}sₐ-3' | 3226 | 24 |
| anti-122fS | 5'-aₛcₛaₛaₛaₛcₛaₛcₛcₛaₛuₛuₛgₛuₛcₛaₛcₛaₛcₛuₛcₛcₛa-3' | 3037 | 10 |
| antagomir-122 | 5'-aₛcₛaaacaccauugucaeacuₛcₛcₛaₛ-Chol-3' | 3038 | 5 |
| mm-antagomir-122 | 5'-aₛcₛacacaacacugucacauus_{c}s_{c}sₐs-Chol-3' | 3040 | 14 |
| antagomir-122(I) | 5'-uₛgₛgagugugacaaugguguuₛuₛgₛuₛ-Chol-3' | 3223 | 21 |
| antagomir-122(II) | 5' -uₛgₛgaaggugacaguguuguusuₛgₛuₛ-Chol-3' | 3224 | 22 |
| antagomir-122(III) | 5'-uₛcₛacgcgagccgaacgaacsₐsₐsₐs-Chol-3' | 3230 | 28 |
| antagomir-16 | 5'-cₛgₛccaauauuuacgugcugₛcₛuₛaₛ-Chol-3' | 3227 | 6 |
| antagomir-192 | 5'-gₛgₛcugucaauucauaggus_{c}sₐs_{g}s-Chol-3' | 3228 | 7 |
| antagomir-194 | 5'-uₛcₛcacauggaguugcuguuₛaₛcₛaₛ-Chol-3' | 3229 | 8 |

| | | | |
|---|---|---|---|
| lower case letters represent 2'-O-methyl modified nucleotides; subscript's' represent phosphorothioate linkage; "Chol" indicates cholesterol conjugate | | | |

MiR-122 is expressed at high levels in hepatocytes with over 50,000 copies per cell (Chang J. et al., RNA Biology 1:2, 106-113, 2004). To determine whether the silencing of miR-122 following antagomir treatment was caused by stoichiometric duplex formation between miR-122 and antagomir-122 or by catalytic degradation of miR-122, total RNA from livers of mice treated with unconjugated single-stranded anti-miR-122 RNAs (anti-122fS, anti-122pS) or antagomir-122 were examined under stringent, formamide-containing denaturing conditions (FIG. 1C). No difference in miR-122 levels could be detected between PBS and unconjugated anti-miR-122 RNA-treated livers, showing that the decrease in miR-122 levels observed under non-stringent conditions was not caused by degradation, but instead by the formation of miR-122/RNA duplexes. In contrast, miR-122 remained undetectable in livers of mice treated with antagomir-122. These data suggest that the silencing of miRNA-122 in livers of mice treated with antagomir-122 was due to degradation of the miRNA, and the ability of antagomir-122, but not unconjugated anti-122 RNAs, to result in miR-122 degradation may be due to efficient delivery of antagomirs to hepatocytes.

To determine the dose of antagomir-122 that can completely silence miR-122, mice were injected with 80, 160 or 240 mg/kg bodyweight antagomir-122 and miR-122 expression levels were measured. The highest dose (240 mg/kg bodyweight) resulted in a complete loss of miR-122 signal and was subsequently used for all other experiments (FIG. 2A).

The duration of silencing with antagomir-122 was also measured. Levels of miR-122 were undetectable for as long as 23 days post-injection (FIG. 2B), indicating that silencing of miRNAs using antagomirs is long lasting. The injected antagomirs were well tolerated even during the course of the prolonged treatment; no alterations in bodyweight or serum markers of liver toxicity (alanine aminotransferase) were detected. To test the bioavailability of antagomirs *in vivo* and their ability to silence miRNA expression in different tissues, mice were injected with antagomir-16 directed to miR-16, which is abundantly expressed in all tissues (miR-16 is predicted to target one or both of *Activin type II receptor* gene, which is involved in TGFbeta signaling, and *Hox-A5I* (John et al., PLoS Biology 2:1862-1878, 2004; correction in PLoS Biology 3:1328, 2005)). Tissues were harvested one day after the final injection, and miRNA expression levels were compared to PBS-injected mice. Northern blot analysis revealed that expression of miR-16 was efficiently silenced in all tissues tested except brain (FIG. 3A). Antagomir-16 did not affect the expression of the 89 nt precursor of miR-16 as detected in bone marrow. The bioavailability of antagomir-16 was also assessed by Northern blotting in the above mentioned tissue samples. In concordance with the ability to silence miR-16 levels, significant levels of antagomir-16 were detected in all tissues except brain (FIG. 3B). Together, these data demonstrate that antagomirs achieve broad biodistribution and can efficiently silence miRNAs in most tissues *in vivo.*

Many miRNA genes have been found to be located in close proximity and to be coordinately transcribed. These polycistronic miRNA genes are transcribed to generate long primary transcripts (pri-miRNAs), which are processed by multiple enzymes in the nucleus and cytoplasm to generate the mature miRNA. To investigate if antagomirs targeting polycistronic miRNAs retain their target specificity with no effect on the expression of neighboring miRNAs, mice were injected with antagomirs targeting either miR-192 or miR-194 of the bicistronic cluster miR-192/194. Administration of antagomir-192 into mice resulted in silencing of miR-192 in liver and kidney, with no effect on the expression levels of miR-194. Conversely, injection of antagomir-194 into mice abolished miR-194 expression but had no demonstrable effect on the miR-192 levels compared to PBS-injected mice. These data demonstrate that antagomirs have the ability to differentially silence specific miRNAs that derive from the same primary transcript.

To test whether silencing of an miRNA can cause a corresponding increase in target protein and possibly mRNA levels, the expression of aldolase A, a gene that is repressed in hepatocytes and predicted to be a target of miR-122, was examined. The aldolase-A mRNA has a conserved nucleotide sequence with perfect sequence complementarity to miR-122 between nucleotides 29 and 36 downstream of the open reading frame. Aldolase-A expression was increased 4-5 fold in livers of mice injected with antagomir-122 compared to a scrambled control (mm-antagomir-122). This regulation was observed in multiple experiments and different time points after injection. The target was also independently confirmed by cloning the 3'UTR Aldolase-A downstream of the luciferase open reading frame and cotransfecting this vector with control miRNAs (miR-124 (5'-UAAGGCACGCGGUGAAUGCCA-3 SEQ ID NO:41); see Krek et al., Nature Genetics 37:495-500, 2005, and Lim et al., Nature 433:769-773, 2005) and miR-192) and miR-122 into HEK293 cells, which lack miR-122 expression. Cotransfection of miR-122 resulted in a significant reduction in luciferase activity compared to miR-124 and miR-192 transfected cells. Together, these data indicate that aldolase-A is a physiological target of miR-122.

The upregulation of aldolase-A in mice treated with antagomir-122 demonstrates functional silencing of this miRNA. Aldolase-A is a housekeeping gene expressed in all cells. This gene is produced in large amounts in muscle where it can be as much as 5% of total cellular protein. In adult liver, aldolase-A expression is repressed and aldolase-B is produced. Conversely, dedifferentiated hepatocytes and transformed liver cells have increased aldolase-A expression levels and can even replace aldolase-B. Expression of miR-122 shows an inverse relationship with aldolase-A expression, with highest levels in differentiated adult hepatocytes and complete absence in undifferentiated cells such as HepG2. In contrast, the mRNA levels of aldolase-B, which lacks miR-122 target sites, were unaffected by antagomir-122. These findings provide non-genetic, pharmacologic evidence in mammals that microRNAs define tissue-specific gene expression.

To identify other genes regulated by miR-122, we carried out gene expression analysis using Affymetrix™ arrays (Affymetrix, Inc., Santa Clara, CA) in livers from mm-antagomir-122 and antagomir-122 treated mice. We identified 493 gene transcripts that were up-regulated (≥1.4-fold) and 365 transcripts (≤1.4-fold) that were down-regulated in antagomir-122 treated mice compared to controls. The regulation of genes that were up-regulated in antagomir-122-treated livers was confirmed by RT-PCR. Strikingly, these included those members of gene families that are usually repressed in hepatocytes, including alodolase A (aldo-A), N-Myc downstream regulated gene (Ndrg3), IQ motif containing GTPase activating protein-1 (Iqgapl). MiR-122 could therefore contribute to the maintenance of the adult liver phenotype, as previously suggested for two other specific miRNAs (Lim et al., Nature 433:769-773, 2005). To assess further the motif contents of significantly up- and down-regulated genes, we analyzed the 3'UTR sequences of 9554 mRNAs. Of these, 142 mRNAs were significantly up-regulated and had a fold-change of at least 1.4. We observed a highly significant 2.6-fold increase in the probability to have at least one miR-122 nucleus in the 3'UTR of up-regulated genes compared to genes with no change in mRNA levels. Interestingly, the majority of the miR-122 nuclei in up-regulated genes had not been detected by previous prediction methods (*see, e.g.,* Lewis et al., Cell 120:15-20, 2005), indicating that the number of direct miRNA targets is significantly larger then previously estimated.

To experimentally validate the link between repression and the presence of miR-122 nuclei matches within the 3'UTR, we cloned the 3' UTR of four genes repressed by antagomir-122 and containing a miR-122 nucleus into a luciferase reporter system. When co-transfected with miR-122, all reporters exhibited significant repression relative to co-transfections with control miRNA (si-124), suggesting that miR-122 binding to its nucleus contributes directly to mRNA repression. Surprisingly, we also observed that the probability for down-regulated genes to harbor a miR-122 nucleus was reduced by almost the same factor of 2.7-fold. To further analyze if over-representation and under-representation of miR-122 nuclei is specific, we analyzed the abundance of all 4096 possible 6-mer motifs across down-, up-, and "no change" transcripts. When comparing up-regulated versus no change genes, the miR-122 nucleus (CACTCC) was the most significantly over-represented 6-mer. Similarly, the miR-122 nucleus was within the top 0.5% of under-represented motifs for down-regulated transcripts, indicating an evolutionary tendency of down-regulated genes to lack binding sites for miR-122. These results indicate that up-regulated mRNAs are directly targeted and repressed by miR-122, but also that a significant number of down-regulated genes are likely to be either directly or indirectly activated by miR-122.

To assess the functional significance of altered gene regulation by miR-122 we analyzed the functional annotation of regulated genes for enrichment in Gene Ontology categories (see Methods). The top ranking functional category was "cholesterol biosynthesis" with a p-value of 1.6x10⁻¹¹ and was found for gene transcripts down-regulated by antagomir-122. The expression of at least 13 genes involved in cholesterol biosynthesis was decreased between 1.4 to 2.3-fold in antagomir-122 treated mice; some of these were confirmed by RT-PCR. Interestingly, mice injected with an adenovirus expressing miR-122 (Ad-122) increased expression of some of these genes. One of these gene transcripts down-regulated by antagomir-122 treatment was HMG-CoA-reductase (Hmgcr), a rate-limiting enzyme of endogenous cholesterol biosynthesis and the target for statin-based drugs. We measured the enzymatic activity of Hmgcr in liver extracts and found a ∼45% reduction in Hmgcr activity in antagomir-122 vs. mm-antagomir-122 treated mice (9.7±1.0 versus 17.2±2.3 pmol/mg microsomal protein/min, respectively; n=5, P=0.02). Consistent with this effect on Hmgcr activity, plasma cholesterol levels were decreased >40% in antagomir-122 treated animals while there was no detectable effect on plasma free fatty acids (FFA), triglyceride, bile acid and glucose levels. No decrease in plasma cholesterol was observed with antagomir-192, -194 and -16, showing that together with the absence of effects by mm-antagomir-122, the effects of antagomir-122 are sequence specific and unrelated to the use of a cholesterol-conjugated oligonucleotide *per se.* Reduced plasma cholesterol levels in antagomir-122 treated mice lasted for at least 2 weeks. Together, these data demonstrate that miR-122 is a regulator of the cholesterol biosynthetic pathway.

To test whether double stranded antagomirs were effective, six-week old femaleC57BL/6 mice were injected via the tail vein with 80 mg/kg/day on three consecutive days with either PBS, or compounds AP-3018, -3019, -3020 in a total volume of 0.2 ml. The liver was harvested 24 hrs after the last injection and total RNA was isolated using Trizol (Invitrogen). 10 µg of total RNA was run on formamide containing polyacrylamide gels, blotted and probed for miR-122 using a 32P-labeled antisense oligo. Two different autoradiography exposures are shown (see Figure 12). Each lane represents an individual animal. Double standed antagomirs were able to reduce mircoRNA levels.

Our data demonstrate that antagomirs are effective inhibitors of miRNAs *in vivo.* Silencing of miR-122 by antagomirs allowed us to study gene regulation by a tissue-specific *miRNA in vivo.* Of the genes that were up-regulated, only 12 genes (including AldoA, citrate synthase and Iqgap1) had previously been predicted using bioinformatic approaches (Krek et al., Nat. Genet. 37:495-500, 2005). Since 52% of all up-regulated genes have at least one miR-122 nucleus in their 3'UTR sequence this indicates that they are likely direct targets. Notably, silencing of miR-122 also led to a *reduction* of a significant number of genes. We found that these genes have a drastically reduced probability to contain a miR-122 nucleus in their 3'UTR.

### Methods

*Synthesis of antagomirs* RNAs were synthesized using commercially available 5'-O-(4,4'-dimethoxytrityl)-2'-O-methyl-3'-O-(2-cyanoethyl-N,N-diisopropyl) RNA phosphoramidite monomers of 6-N-benzoyladenosine (ABz), 4-N-benzoylcytidine (CBz), 2-N-isobutyrylguanosine (GiBu), and uridine (U), according to standard solid phase oligonucleotide synthesis protocols (Damha and Ogilvie, Methods Mol. Biol. 20:81-114, 1993). For antagomirs, i.e., cholesterol conjugated RNAs, the synthesis started from a controlled-pore glass solid support carrying a cholesterol- hydroxyprolinol linker (Manoharan et al., U.S. Pat. Appl. Publ. 20050107325). Antagomirs with phosphorothioate backbone at a given position were achieved by oxidation of phosphite with phenylacetyl disulfide (PADS) during oligonucleotide synthesis *(*Cheruvallath et al., Nucleosides Nucleotides 18:485-492, 1999). After cleavage and de-protection, antagomirs were purified by reverse-phase high-performance liquid chromatography, while the unconjugated RNA oligonucleotides were purified by anion-exchange high-performance liquid chromatography. Purified oligonucleotides were characterized by ES mass spectrometry and capillary gel electrophoresis.

*Animals.* All animal models were maintained in C57B1/6J background on a 12 hours light/dark cycle in a pathogen-free animal facility at Rockefeller University. Six week old mice received, on one to three consecutive days, tail vein injections of saline or different RNAs. RNAs were administered at doses of 80 mg/kg body weight in 0.2 ml per injection. Measurements of miRNA levels in tissues were performed 24 h after the last injection unless indicated otherwise. Tissues were harvested, snap frozen and stored at -80°C.

*Generation of recombinant adenovirus.* The recombinant adenovirus used to express miR-122 (Ad-122) was generated by PCR, amplifying a 344 bp miRNA precursor sequence with primers 5'-AGTCAGATGTACAGTTATAAGCACAAGAGGACCAG-3' (SEQ ID NO:42) and 5'-TTATTCAAGATCCCGGGGCTCTTCC-3' (SEQ ID NO:43). The fragment was cloned into vector Ad5CMV-KnpA. Ad-EGFP (ViraQuest, North Liberty, IA) was used as a control. Mice were infected with1x10⁹ pfu/mouse by tail vein injection.

*Gene expression analysis.* Total RNA of mice treated with antagomirs or recombinant adenovirus was isolated three days after treatment. RNA was pooled from four animals for each group. The integrity of the RNA sample was assessed by denaturing formamide gel analysis. First strand cDNA synthesis was completed with total RNA (30 µg) cleaned with RNAeasy columns (Qiagen, Valencia, CA) and the Superscript Choice cDNA synthesis protocol (Invitrogen, Carlsbad, CA), except and HPLC purified T7-promoter-dT30 primer (Proligo LLC, Boulder, CO) was used to initiate the first strand reaction. Biotin labeled cRNA was synthesized from T7 cDNA using the RNA transcript labeling kit (Enzo Biochem, Farmingdale, NY), supplemented with biotin 11-CTP and biotin-UTP (Enzo Biochem, Farmingdale, NY) as specified by the Affymetrix protocol. The sample was cleaned with an RNAeasy column (Qiagen, Valencia, CA) to remove free nucleotides and then quantitated spectrophotometrically. Biotin-labeled cRNA was fragmented and hybridized to Mouse Expression Set 430 arrays (Affymetrix, Inc., Santa Clara, CA) according to the manufacturer's manual with a final concentration of fragmented cRNA of 0.05 µg/µl. The arrays were scanned using a Hewlett Packard confocal laser scanner and analyzed using ArrayAssist Lite and Affymetrix^{®} Microarray Suite v.5 (MAS5) software.

*Northern blotting analysis.* Total RNA was isolated using the Trizol^{®} reagent (Invitrogen, Carlsbad, CA) and ethanol precipitation. RNA was separated at 45 mA on 14%-polyacrylamide gels that contained 8 M urea and 20% formamide. Antisense probes were designed according to the "microRNA registry" (Griffiths-Jones, NAR 32:D109-D111, 2004).

*RT*-*PCR*. Extraction of total RNA, synthesis of cDNA, and PCR were carried out as described in Shih et al. (Proc. Natl. Acad. Sci. U.S.A. 99:3818-3823, 2002).

*Assay of luciferase activity.* The mouse full length adolase-A 3'UTR was PCR-amplified using the following primers: 5' d-(CCAGAGCTGAACTAAGGCTGCTCCA)-3' (SEQ ID NO:44) and 5' d-(CCCCTTAAATAGTTGTTTAT TGGCA)-3'(SEQ ID NO:9) and cloned downstream of the stop codon in pRL-TK (Promega, Madison, WI). HEK293 cells were cultured in 24-well plates and each transfected with 50 ng of pRL-TK (Rr-luc), 50 ng of pGL3 control vector (Pp-luc) (Promega, Madison, WI) and 200 ng of doublestranded siRNA (Dharmacon, Lafayette, CO). Cells were harvested and assayed 24-30 h post-transfection.

*3'UTR sequences and mapping of array probes to transcripts.* We extracted mouse 3' UTRs using the Refseq data set (Pruitt et al., Nucleic Acids Res.33:D501-D504, 2005). 17264 3' UTR sequences of at least 30 nucleotides in length were obtained. Affymetrix probe identifiers were assigned to the Refseq transcripts by using a mapping provided by Ensembl software (Hubbard et al., Nucleic Acids Res. 33 Database issue:D447-D453, 2005). When only one probe identifier mapped to a transcript, the significance call for a fold change and the fold change itself, as provided by the Affymetrix software, was taken at face value. When more than one probe identifier mapped to a transcript, we insisted that the significance call was consistent for all probes. Transcripts were discarded otherwise. The fold change assigned to a transcript was the average of all probes that mapped to the transcript. Finally, a cut-off of 0.5 in the logarithm (base 2) of fold changes was applied.

*Gene Ontology analysis.* Refseq identifiers were mapped to MGI identifiers using a map provided by Ensembl software (Hubbard et al., Nucleic Acids Res. 33 Database issue:D447-D453, 2005). We then used the program FuncAssociate (Castillo-Davis and Hartl, Bioinformatics 19:891-892, 2003) with default settings to search for overrepresented Gene Ontology terms. Results were sorted by LOD scores. Independently, we obtained very similar results with the program GeneMerge v.1.2 and by applying a conservative Bonferroni correction for multiple testing.

*HMG-CoA reductase (HMGR) activity assay.* Hepatic microsomal HMGR activity was assayed by a method modified from a previously published procedure (Nguyen, et al., J. Clin. Invest. 86:923-931, 1990). Briefly, hepatic microsomal protein extracts were preincubated with an NADPH-generating system (3.4 mM NADP+/30 mM glucose 6-phosphate/0.3 units of glucose-6-phosphate dehydrogenase) in buffer (50 mM K₂PHO₄/70 mM KCl/10 mM DTT/30 mM EDTA, pH 7.4). The reaction was started with the addition of 15 µl ¹⁴C-labeled substrate ([¹⁴C]HMG CoA, (Amersham, Piscataway, NJ)). The mixture was incubated for 15 min. and stopped with 15 µl 6 M HCl. [³H⁻]mevalonolactone and unlabeled mevalonolactone were added for recovery standard and product marker, respectively. After lactonization the products were extracted with ether and separated by TLC on Silica Gel 60 plates (VWR Scientific, West Chester, PA) with benzene/acetone (1:1, vol/vol) as the solvent system. The immediate product (¹⁴C-labeled mevalonolactone) was quantitated by scintillation spectrometry.

*Statistical analysis.* Results are given as mean ± s.d. Statistical analyses were performed by using Student's t-test, and the null hypothesis was rejected at the 0.05 level.

Example 2. Trizol^{®} reagent and ethanol can be used to precipitate a miR-122/antagomir-122 duplex. A Trizol^{®} (Invitrogen, Carlsbad, CA) protocol was modified for precipitation of a miR-122/antagomir 122 duplex. A duplex containing a synthetic miR-122 (22 nt, Dharmacon, Lafayette, CO) and antagomir-122 molecule was formed by incubating equal amounts of miR-122 and antagomir-122 in water for 1 min. at 95°C followed by an incubation at 37°C for 1 hr. The duplex was then added to the aqueous phase of a Trizol^{®}/liver extract and aliquots were subjected to different precipitation methods (10 min. at room temperature with 50% or 80% isopropanol followed by 10 min. centrifugation at 13,200 rpm at 4°C; or 30 min. at -80°C with 70% methanol and 0.5 M ammonium acetate or 0.08 M sodium acetate followed by 20 min. centrifugation at 13,200 rpm at 4°C). The precipitates were washed once with 85% ethanol, dissolved in water, separated on a 14% sequencing gel and visualized using ethidium bromide. The respective input of the duplex was loaded in comparison (1.4 mg/lane).

The duplex did precipitate in (i) 80% isopropanol, (ii) in 70% ethanol/ 0.5M NH₄-Acetate, and (iii) in 70% ethanol/ 0.08M Na-acetate. The duplex would not precipitate in 50% isopropanol, which follows the conventional Trizol^{®} protocol.

Example 3. Trizol^{®} did not precipitate a miR/antagomir duplex in the absence of ethanol or isopropanol. Liver tissue was homogenized in Trizol^{®}, aliquoted into eppendorf tubes (1 ml volume each) and preformed duplex miR-122/antagomir-122 or miR-16/antagomir-16 was added. Samples were vortexed and left at room temperature for 10 minutes. 200 ml of chloroform were added and samples vortexed for 2 min at room temperature, followed by centrifugation at 13,200 rpm at room temperature for 15 minutes. 400 ml of the supernatant were added to 1 ml 100% ethanol and 40 ml 3 M sodium acetate, pH 5.2. After 30 min. at -80°C samples were centrifuged for 20 min. at 4°C. Precipitates were washed with 85% ethanol, dissolved in water, separated on a 14% sequencing gel and visualized using ethidium bromide. The input was loaded for comparison (5 mg per lane). The miR/antagomir duplex was not precipitated by this Trizol^{®} protocol, which lacked ethanol or isopropanol, although a faint signal indicated some recovery of single-stranded miR.

In a similar assay, liver/Trizol^{®} homogenates were processed exactly as described, but 4 mg each of (i) miR-122, (ii) antagomir-122, (iii) anti122pS, or (iv) miR-122 (4 mg) and antagomir-122 (4 mg) together were added to the homogenates. After the incubation and precipitation steps, only miR-122 was isolated. This was the result whether miR-122 alone was added to the homogenate, or whether miR-122 was added in combination with antagomir-122.

When preformed duplex of miR-122/antagomir-122 (4 mg of each) were added to the liver/Trizol^{®} homogenates, miR-122, but not antagomir-122, was isolated from the preformed duplex.

To further test the Trizol^{®} precipitation protocol, total liver RNA was isolated (using the protocol), and then the RNA was dissolved in water. 40 mg of the isolated RNA were incubated in a total volume of 50 ml water with the increasing amounts of antagomir-122 for 5 min. at 65°C followed by 2 h at 37°C and 30 min. at room temperature. The amounts of antagomir-122 tested were 6.4 pg, 320 pg, 16 ng, 0.8 µg, 40 µg, and 120 µg. 1 ml of Trizol^{®} was added to the samples and Trizol^{®} extraction was performed again as described above. The precipitates were dissolved in 60 ml water, and then 30 ml of each sample was separated on 14% polyacrylamide gels with or without 20% formamide. miR-122 was then detected using Northern blotting.

The miR-122 was precipitated and detected by gel electrophoresis in the presence of formamide regardless of the amount of antagomir-122 added to the precipitation mix. When 40 µg or 120 µg were used in the precipitation mix, a duplex between miR-122 and antagomir was visible when analyzed on a sequencing gel. Minor amounts of antagomir were always retrieved by the Trizol^{®} protocol.

To test the precipitation protocol with smaller amounts of RNA, total RNA was isolated from liver or kidney using the Trizol^{®} protocol and then the RNA was dissolved in water. 10 mg of RNA were incubated for 3 h at 37°C in a total volume of 50 ml water together with 20 mg antagomir-122. The kidney RNA, which does not contain endogenous miR-122, was spiked with 20 finoles of synthetic miR-122. 1 ml of Trizol^{®} was added and then Trizol^{®} extraction was performed again as described above. Precipitates were dissolved in 30 ml water and separated in a 14% polyacrylamide gel containing 20% formamide. First, miR-122 was detected using Northern blotting, then the membrane was re-probed against antagomir-122, which detected the presence of the antagomir-122. Incubation with antagomir-122 did not alter the miR-122 signal detected on the gel.

Mice were administered 80mg/kg/day antagomir-122 or a scrambled control (mm-antagmir122) via tail-vein on three separate days. Livers were harvested at days 3, 6, 9, 13, and 23 post-injection, and subjected to Trizol^{®} isolation as described above. Approximately 50 mg liver were homogenized in 1 ml Trizol^{®}. Analysis by Northern blot indicated that antagomir-122 could be detected for at least 23 days post-injection.

Example 4. A bDNA lysis protocol allows quantitative isolation of miR-122/antagomir-122 duplexes and miR-122 single-stranded molecules. 100 mg liver were sonicated in 2 ml T+C (Epicentre^{®}, Madison, WI) in the presence of 350 mg proteinase K. 100 ml of the homogenate was supplemented with either miR-122/antagomir-122 duplexes (8 mg per lane), miR-122 (4 mg per lane) or antagomir-122 (4 mg per lane). 200 ml 1xSTE-buffer was added, then 200 ml of phenol, pH 4 or pH 8. Samples were vortexed for 30 sec and left on ice for 2 min. After centrifugation for 10 min at 13,200 rpm (4°C), 280 ml of the supernatant was added to 900 ml 100% ethanol and incubated for 90 min at 90°C. RNA was precipitated for 10 min at 13,200 rpm, washed once with 85% ethanol, dissolved in water and separated on a sequencing gel. miR-122/antagomir-122 duplexes and miR-122 (but not antagomir-122) were detected by ethidium bromide staining. The duplexes and miR-122 were successfully isolated with phenol at pH 4 and at pH 8.

Example 5. Antagomir-122 caused a decrease in miR-122 levels *in vivo.* To test the effect of antagomir-122 on miRNA levels *in vivo,* mice were administered PBS, mm-antagomir-122 or antagomir-122 (80 mg/kg/day) via tail-vein injection for 3 subsequent days. Livers were harvested 24 hrs after the last injection and ∼20 mg of tissue was sonicated in 1 ml T+C (Epicentre^{®}, Madison, WI) and proteinase K and processed as described in example 4. Precipitated RNA was dissolved in water and analyzed on 14% polyacrylamide gels that contained 20% formamide. Northern blotting was performed for miR-122 and let7 miRNAs. Mice injected with antagomir-122 revealed a striking decrease in the amount of miR-122 isolated from liver, as compared to mice injected with PBS or mm-antagomir-122 (FIG. 4). The level of let7 miRNA isolated from liver was similar in the three test groups.

To test the long-term effect of antagomir-122 on miRNA levels *in vivo,* mice were administered PBS, mm-antagomir-122 or antagomir-122 (80 mg/kg/day) via tail-vein injection for 3 subsequent days. Livers were then harvested 72h, 10days or 27days after the last injection. Tissues were processed as described in example 4, and Northern blot analysis revealed a marked reduction of miR-122 in the antagomir-treated mice, even 27 days post-injection. As a further note, the Northern blot analysis revealed an RNA of higher molecular weight specifically detected by the miR-122 probe. This molecule is most likely an miR-122 precursor.

Example 6. Dose response for antagomir-122. Three groups of mice n=3, C57/B16, female, 6-8 weeks old) were injected with antagomir-122 at a dose of 3x20, 3x40 or 3x80 mg/kg body weight in a total volume of 0.2 ml on three consecutive days. Phosphate buffered saline (PBS) injected mice (3x0.2 ml) served as controls. Mice were sacrificed on day 4 and total RNA was extracted from livers for Northern blotting. Mir-122 expression was analyzed following Northern blotting using a ³²P-labeled oligonucleotide with complementary sequence to miR-122. Expression levels of aldolase A, a validated target gene of miR-122, were analyzed by RT-PCR. Gapdh served as a loading control, Gapdh-RT indicates the absence of reverse transcriptase as a control for DNA contamination.

Results demonstrate that reduction of miR-122 can be achieved at a dose of 3x20 mg/kg bodyweight. A >90% reduction in miR-122 levels is required to detect a significant increase in aldolaseA expression (Figure 13).

Example 7. Mismatch control of antagomir-122 activity. Five groups of mice n=3, C57/B16, female, 6-8 weeks old) were injected with antagomir-122 harboring 4,3,2,1 or no nucleotide exchange in the sequence of antagomir-122 (4mm, 3mm, 2mm, 1mm, antagomir-122, respectively). Mice were injected at a dose of 3x80 mg/kg body weight in a total volume of 0.2 ml on three consecutive days. Mice were sacrificed on day 4 and total RNA was extracted from livers for Northern blotting. Mir-122 expression was analyzed by Northern blotting using a ³²P-labeled oligonucleotide with complementary sequence to miR-122.

Results demonstrate that 4, 3 (data not shown), or 2 mismatches in antagomir-122 had no effect on miR-122 expression levels. A single mismatch resulted in a 20-30% reduction in miR-122 expression. These data demonstrate that antagomirs have exquisite target specificity (Figure 14).
4mm:
   oA*oC*oAoCoAoCoAoAoCoAoCoUoGoUoCoAoCoAoUoU*oC*oC*oA*-CHOL (SEQ ID NO:1)
3mm:
   oA*oC*oAoAoAoCoAoAoCoAoCoUoGoUoCoAoCoAoUoU*oC*oC*oA*-CHOL (SEQ ID NO:35)
2mm:
   oA*oC*oAoAoAoCoAoCoCoAoCoUoGoUoCoAoCoAoUoU*oC*oC*oA*-CHOL (SEQ ID NO:36)
1mm:
   oA*oC*oAoAoAoCoAoCoCoAoUoUoGoUoCoAoCoAoUoU*oC*oC*oA*-CHOL (SEQ ID NO:37)
Antagomir-122
   oA*oC*oAoAoAoCoAoCoCoAoUoUoGoUoCoAoCoAoCoU*oC*oC*o A*-CHOL (SEQ OD NO:11)

Example 8. Length effect on antagomir-122 activity. Six groups of mice (n=3, C57/B16, female, 6-8 weeks old; only n=2 shown) were injected with antagomir-122, which differed in length between 25, 23 (antagomir-122), 21, 19 and 17 bp. Mice were injected at a dose of 3x80 mg/kg body weight in a total volume of 0.2 ml on three consecutive days. Mice were sacrificed on day 4 and total RNA was extracted from livers for Northern blotting. Mir-122 expression was analyzed by Northern blotting using a ³²P-labeled oligonucleotide with complementary sequence to miR-122.

Results demonstrate that an additional nucleotide at both 3' and 5' ends of antagomir-122 (25-mer), or a deletion at either end (21-mer) has no effect of the ability of antagomirs to silence miR-122. Further shortening of antagomirs (19- and 17-mers) result in a loss of antagomir activity (Figure 15).
Antagomir-122 (23-mer)
   oA*oC*oAoAoAoCoAoCoCoAoUoUoGoUoCoAoCoAoCoU*oC*oC*oA*-CHOL (SEQ ID NO:5)
25-mer:
   oC*oA*oCoAoAoAoCoAoCoCoAoUoUoGoUoCoAoCoAoCoUoC*oC*oA* oC*-CHOL (SEQ ID NO:31)
21-mer:
   oC*oA*oAoAoCoAoCoCoAoUoUoGoUoCoAoCoAoC*oU*oC*oC*-CHOL (SEQ ID NO:32)
19-mer:
   oA*oA*oAoCoAoCoCoAoUoUoGoUoCoAoCoA*oC*oU*oC*-CHOL (SEQ ID NO:33)
17-mer:
   oA*oA*oCoAoCoCoAoUoUoGoUoCoAoC*oA*oC*oU*-CHOL (SEQ ID NO:34)

### Example 9: Synthesis of Antagomirs

### Step 1. Oligonucleotide Synthesis

All oligonucleotides were synthesized on an AKTAoligopilot synthesizer or on an ABI 394 DNA/RNA synthesizer. Commercially available controlled pore glass solid supports (rU-CPG, 2'-*O*-methly modified rA-CPG and 2'-O-methyl modified rG-CPG from Prime Synthesis) or the in-house synthesized solid support hydroxyprolinol-cholesterol-CPG were used for the synthesis. RNA phosphoramidites and 2'-*O*-methyl modified RNA phosphoramidites with standard protecting groups (5'-*O*-dimethoxytrityl-N6-benzoyl-2'-*t*-butyldimethylsilyl-adenosine-3'-*O*-N,N'-diisopropyl-2-cyanoethylphosphoramidite, 5'-*O*-dimethoxytrityl-N4-acetyl-2'-*t-*butyldimethylsilyl-cytidine-3'-*O*-N,N'-diisopropyl-2-cyanoethylphosphoramidite, 5'-*O*-dimethoxytrityl-N2-isobutryl-2'-*t*-butyldimethylsilyl-guanosine-3'-*O*-N,N'-diisopropyl-2-cyanoethylphosphoramidite, 5'-*O*-dimethoxytrityl-2'-*t-*butyldimethylsilyl-uridine-3'-*O*-N,N'-diisopropyl-2-cyanoethylphosphoramidite, 5'-*O*-dimethoxytrityl-N6-benzoyl-2'-*O*-methyl-adenosine-3'-*O*-N,N'-diisopropyl-2-cyanoethylphosphoramidite, 5'-*O*-dimethoxytrityl-N4-acetyl-2'-*O*-methyl-cytidine-3'-*O*-N,N'-diisopropyl-2-cyanoethylphosphoramidite, 5'-O-dimethoxytrityl-N2-isobutryl-2'-*O*-methyl-guanosine-3'-*O*-N,N'-diisopropyl-2-cyanoethylphosphoramidite, and 5'-*O*-dimethoxytrityl-2'-*O*-methyl-uridine-3'-O-N,N'-diisopropyl-2-cyanoethylphosphoramidite) were obtained from Pierce Nucleic Acids Technologies and ChemGenes Research. The Quasar 570 phosphoramidite was obtained from Biosearch Technologies. The 5'-*O*-dimethoxytrityl-2'-*t-*butyldimethylsilyl-inosine-3'-*O*-N,N'-diisopropyl-2-cyanoethylphosphoramidite was obtained from ChemGenes Research. For the syntheses on AKTAoligopilot synthesizer, all phosphoramidites were used at a concentration of 0.2 M in CH₃CN except for guanosine and 2'-O-methyl-uridine, which were used at 0.2 M concentration in 10% THF/CH₃CN (v/v). Coupling/recycling time of 16 minutes was used for all phosphoramidite couplings. The activator was 5-ethyl-thio-tetrazole (0.75 M, American International Chemicals). For the PO-oxidation, 50 mM iodine in water/pyridine (10:90 v/v) was used and for the PS-oxidation 2% PADS (GL Synthesis) in 2,6-lutidine/CH₃CN (1:1 v/v) was used. For the syntheses on ABI 394 DNA/RNA synthesizer, all phosphoramidites were used at a concentration of 0.15 M in CH₃CN except for 2'-O-methyl-uridine, which was used at 0.15 M concentration in 10% THF/CH₃CN (v/v). Coupling time of 10 minutes was used for all phosphoramidite couplings. The activator was 5-ethyl-thio-tetrazole (0.25 M, Glen Research): For the PO-oxidation, 20 mM iodine in water/pyridine (Glen Research) was used and for the PS-oxidation 0.1M DDTT (AM Chemicals) in pyridine was used. Coupling of the Quasar 570 phosphoramidite was carried out on the ABI DNA/RNA synthesizer. The Quasar 570 phosphoramidite was used at a concentration of 0.1M in CH₃CN with a coupling time of 10 mins. The activator was 5-ethyl-thio-tetrazole (0.25 M, Glen Research) and 0.1M DDTT (AM Chemicals) in pyridine was used for PS oxidation.

### Step 2. Deprotection of oligonucleotides

### A. Sequences synthesized on the AKTAoligopilot synthesizer

After completion of synthesis, the support was transferred to a 100 mL glass bottle (VWR). The oligonucleotide was cleaved from the support with simultaneous deprotection of base and phosphate groups with 40 mL of a 40% aq. methyl amine (Aldrich) 90 mins at 45°C. The bottle was cooled briefly on ice and then the methylamine was filtered into a new 500 mL bottle. The CPG was washed three times with 40 mL portions of DMSO. The mixture was then cooled on dry ice.

In order to remove the *tert-*butyldimethylsilyl (TBDMS) groups at the 2' position, 60 mL triethylamine trihydrofluoride (Et3N-HF) was added to the above mixture. The mixture was heated at 40°C for 60 minutes. The reaction was then quenched with 220 mL of 50 mM sodium acetate (pH 5.5) and stored in the freezer until purification.

### B. Sequences synthesized on the ABI DAN/RNA synthesizer

After completion of synthesis, the support was transferred to a 15 mL tube (VWR). The oligonucleotide was cleaved from the support with simultaneous deprotection of base and phosphate groups with 7 mL of a 40% aq. methyl amine (Aldrich) 15 mins at 65°C. The bottle was cooled briefly on ice and then the methylamine was filtered into a 100 mL bottle (VWR). The CPG was washed three times with 7 mL portions of DMSO. The mixture was then cooled on dry ice.

In order to remove the *tert*-butyldimethylsilyl (TBDMS) groups at the 2' position, 10.5 mL triethylamine trihydrofluoride (Et3N-HF) was added to the above mixture. The mixture was heated at 60°C for 15 minutes. The reaction was then quenched with 38.5 mL of 50 mM sodium acetate (pH 5.5) and stored in the freezer until purification.

### Step 3. Quantitation of Crude Oligonucleotides

For all samples, a 10 µL aliquot was diluted with 990 µL of deionised nuclease free water (1.0 mL) and the absorbance reading at 260 nm was obtained.

### Step 4. Purification of Oligonucleotides

### (a) Unconjugated oligonucleotides

The unconjugated crude oligonucleotides were first analyzed by HPLC (Dionex PA 100). The buffers were 20 mM phosphate, pH 11 (buffer A); and 20 mM phosphate, 1.8 M NaBr, pH 11 (buffer B). The flow rate 1.0 mL/min and monitored wavelength was 260-280 nm. Injections of 5-15 µL were done for each sample.

The unconjugated samples were purified by HPLC on a TSK-Gel SuperQ-5PW (20) column packed in house (17.3 x 5 cm) or on a commercially available TSK-Gel SuperQ-5PW column (15 x 0.215cm) available from TOSOH Bioscience. The buffers were 20 mM phosphate in 10% CH₃CN, pH 8.5 (buffer A) and 20 mM phosphate, 1.0 M NaBr in 10% CH₃CN, pH 8.5 (buffer B). The flow rate was 50.0 mL/min for the in house packed column and 10.0ml/min for the commercially obtained column. Wavelengths of 260 and 294 nm were monitored. The fractions containing the full-length oligonucleotides were pooled together, evaporated, and reconstituted to ∼100 mL with deionised water.

### (b) Cholesterol-conjugated oligonucleotides

The cholesterol-conjugated crude oligonucleotides were first analyzed by LC/MS to determine purity. The cholesterol conjugated sequences were HPLC purified on RPC-Source15 reverse-phase columns packed in house (17.3 x 5 cm or 15 x 2 cm). The buffers were 20 mM NaOAc in 10 % CH₃CN (buffer A) and 20 mM NaOAc in 70% CH₃CN (buffer B). The flow rate was 50.0 mL/min for the 17.3x 5cm column and 12.0ml/min for the 15 x 2 cm column. Wavelengths of 260 and 284 nm were monitored. The fractions containing the full-length oligonucleotides were pooled, evaporated, and reconstituted to 100 mL with deionised water.

### Step 5. Desalting of Purified Oligonucleotides

The purified oligonucleotides were desalted on either an AKTA Explorer or an AKTA Prime system (Amersham Biosciences) using a Sephadex G-25 column packed in house. First, the column was washed with water at a flow rate of 40 mL/min for 20-30 min. The sample was then applied in 40-60 mL fractions. The eluted salt-free fractions were combined, dried, and reconstituted in ∼50 mL of RNase free water.

### Step 6. Purity Analysis by Capillary Gel Electrophoresis (CGE), Ionexchange HPLC (IEX), and Electrospray LC/Ms

Approximately 0.3 OD of each of the desalted oligonucleotides were diluted in water to 300 µL and were analyzed by CGE, ion exchange HPLC, and LC/MS.

### Step 7. Duplex formation

For the fully double stranded duplexes, equal amounts, by weight, of two RNA strands were mixed together. The mixtures were frozen at -80°C and dried under vacuum on a speed vac. Dried samples were then dissolved in 1xPBS to a final concentration of 40mg/ml. The dissolved samples were heated to 95°C for 5 min and slowly cooled to room temperature.

**Table 4: Oligonucleotides synthesized to modulate microRNAs (SEQ ID NOs 1, 12, 10, 11, 13, 14, 21-37, 39, 30 and 10 respectively)**

| **AL**-**SQ #** | **Sequence** | **Target** | **Calc Mass** | **Found Mass** | **Purity (%)** |
|---|---|---|---|---|---|
| 3035 | UGG AGU GUG ACA AUG GUG UUU GU | miR-122A | 7422.44 | 7422.2 0 | 94.1* |
| 3036 | UGG AAU GUG ACA GUG UUG UGU GU | miR-122A | 7422.42 | 7422.2 4 | 95.3* |
| 3037 | A_{OMe}sC_{OMe}sA_{OMe}sA_{OMe}sA_{OMe}sC_{OMe}sA_{OMe}sC_{OMe}sC_{OMe}sA_{OMe}sU_{o Me}sU_{OMe}sG_{OMe}sU_{OMe}sC_{OMe}sA_{OMe}sC_{OMe}sA_{OMe}sC_{OMe}sU_{OMe}sC_{OMe}s C_{OMe}sA_{OMe}s-**Chol** | miR-122A | 8613.43 | 8614.5 3 | 82.7 |
| 3038 | A_{OMe}sC_{OMe}sA_{OMe}A_{OMe}A_{OMe}C_{OMe}A_{OMe}C_{OMe}C_{OMe}A_{OMe}U_{OMe}U_{OMe} G_{OMe}U_{OMe}C_{OMe}A_{OMe}C_{OMe}A_{OMe}C_{OMe}U_{OMe}sC_{OMe}sC_{OMe}sA_{OMe}s-**Chol** | miR-122A | 8340.09 | 8341.2 3 | 99.2 |
| 3039 | A_{OMe}sC_{OMe}sA_{OMe}sC_{OMe}sA_{OMe}sC_{OMe}sA_{OMe}sA_{OMe}sC_{OMe}sA_{OMe}sC_{O Me}sU_{OMe}sG_{OMe}sU_{OMe}sC_{OMe}sA_{OMe}sC_{OMe}sA_{OMe}sU_{OMe}sU_{OMe}sC_{OMe}s C_{OMe}sA_{OMe}s-**Chol** | miR-122A | 8613.43 | 8614.7 5 | 86.6 |
| 3040 | A_{OMe}sC_{OMe}sA_{OMe}C_{OMe}A_{OMe}C_{OMe}A_{OMe}A_{OMe}C_{OMe}A_{OMe}C_{OMe}U_{OMe} G_{OMe}U_{OMe}C_{OMe}A_{OMe}C_{OMe}A_{OMe}U_{OMe}U_{OMe}sC_{OMe}sC_{OMe}sA_{OMe}s-**Chol** | miR-122A | 8340.09 | 8341.1 5 | 85.2 |
| 3223 | U_{OMe}sG_{OMe}sG_{OMe}A_{OMe}G_{OMe}U_{OMe}G_{OMe}U_{OMe}G_{OMe}A_{OMe}C_{OMe}A_{OMe} A_{OMe}U_{OMe}G_{OMe}G_{OMe}U_{OMe}G_{OMe}U_{OMe}U_{OMe}sU_{OMe}sG_{OMe}sU_{OMe}s-**Chol** | miR-122A | 8545.13 | 8546.1 9 | 95.8 |
| 3224 | U_{OMe}sG_{OMe}sG_{OMe}A_{OMe}A_{OMe}U_{OMe}G_{OMe}U_{OMe}G_{OMe}A_{OMe}C_{OMe}A_{OMe} G_{OMe}U_{OMe}G_{OMe}U_{OMe}U_{OMe}G_{OMe}U_{OMe}G_{OMe}sU_{OMe}sG_{OMe}sU_{OMe}s-**Chol** | miR-122A | 8545.13 | 8546.2 8 | 92.3 |
| 3225 | A_{OMe}sC_{OMe}sA_{OMe}sA_{OMe}sA_{OMe}sC_{OMe}sA_{OMe}sC_{OMe}sC_{OMe}sA_{OMe}sU_{o Me}sU_{OMe}sG_{OMe}sU_{OMc}sC_{OMe}sA_{OMe}sC_{OMe}sA_{OMe}sC_{OMe}sU_{OMe}sC_{OMe}s C_{OMe}sA_{OMe} | miR-122A | 7892.09 | 7892.9 2 | 84.0 |
| 3226 | A_{OMe}sC_{OMe}sA_{OMe}A_{OMe}A_{OMe}C_{OMe}A_{OMe}C_{OMe}C_{OMe}A_{OMe}U_{OMe}U_{OMe} G_{OMe}U_{OMe}C_{OMe}A_{OMe}C_{OMe}A_{OMe}C_{OMe}U_{OMe}sC_{OMe}sC_{OMe}sA_{OMe} | miR-122A | 7604.09 | 7604.0 4 | 81.5 |
| 3227 | C_{OMe}sG_{OMe}sC_{OMe}C_{OMe}A_{OMe}A_{OMe}U_{OMe}A_{OMe}U_{OMe}U_{OMe}U_{OMe}A_{OMe} C_{OMe}G_{OMe}U_{OMe}G_{OMe}C_{OMe}U_{OMe}G_{OMe}soC_{OMe}soU_{OMe}soA_{OMe}s-**Chol** | miR-16 | 8047.82 | 8048.8 8 | 94.0* |
| 3228 | G_{OMe}sG_{OMe}sC_{OMe}U_{OMe}G_{OMe}U_{OMe}C_{OMe}A_{OMe}A_{OMe}U_{OMe}U_{OMe}C_{OMe} A_{OMe}U_{OMe}A_{OMe}G_{OMe}G_{OMe}U_{OMe}sC_{OMe}sA_{OMe}sG_{OMe}s-**Chol** | miR-192 | 7807.68 | 7808.4 9 | 97.1* |
| 3229 | U_{OMe}sC_{OMe}sC_{OMe}A_{OMe}C_{OMe}A_{OMe}U_{OMe}G_{OMe}G_{OMe}A_{OMe}G_{OMe}U_{OMe} U_{OMe}G_{OMe}C_{OMe}U_{OMe}G_{OMe}U_{OMe}U_{OMe}sA_{OMe}sC_{OMe}sA_{OMe}s-**Chol** | miR-194 | 8088.84 | 8089.6 9 | 92.7* |
| 3230 | U_{OMe}sC_{OMe}sA_{OMe}C_{OMe}G_{OMe}C_{OMe}G_{OMe}A_{OMe}G_{OMe}C_{OMe}C_{OMe}G_{OMe} A_{OMe}A_{OMe}C_{OMe}G_{OMe}A_{OMe}A_{OMe}C_{OMe}sA_{OMe}sA_{OMe}sA_{OMe}s-**Chol** | miR-375 | 8178.03 | 8178.7 7 | 100* |
| 3344 | UGG IGU GUG ICI IUG GUG UUU GU | miR-122A | 7120.19 | 7119.3 6 | 83.0* |
| 3350 | A_{OMe}C_{OMe}A_{OMe}A_{OMe}A_{OMe}C_{OMe}A_{OMe}C_{OMe}C_{OMe}A_{OMe}U_{OMe}U_{OMe}G_{O Me}U_{OMe}C_{OMe}A_{OMe}C_{OMe}A_{OMe}C_{OMe}U_{OMe}G_{OMe}C_{OMe}A_{OMe}-**Chol** | miR-122A | 8244.09 | 8244.1 3 | 8.0* |
| 3351 | C_{OMe}sA_{OMe}sC_{OMe}A_{OMe}A_{OMe}A_{OMe}C_{OMe}A_{OMe}C_{OMe}C_{OMe}A_{OMe}U_{OMe} U_{OMe}G_{OMe}U_{OMe}C_{OMe}A_{OMe}C_{OMe}A_{OMe}C_{OMe}U_{OMe}C_{OMe}sC_{OMe}sA_{OMe}s C_{OMe}s-**Chol** | miR-122A | 8978.51 | 8979.0 7 | 97.1* |
| 3352 | C_{OMe}sA_{OMe}sA_{OMe}A_{OMe}C_{OMe}A_{OMe}C_{OMe}C_{OMe}A_{OMe}U_{OMe}U_{OMe}G_{OMe} U_{OMe}C_{OMe}A_{OMe}C_{OMe}A_{OMe}C_{OMe}sU_{OMe}sC_{OMe}sC_{OMe}s-**Chol** | miR-122A | 7653.61 | 7653.9 2 | 89.0* |
| 3353 | A_{OMe}sA_{OMe}sA_{OMe}C_{OMe}A_{OMe}C_{OMe}C_{OMe}A_{OMe}U_{OMe}U_{OMe}G_{OMe}U_{OMe} C_{OMe}A_{OMe}C_{OMe}A_{OMe}sC_{OMe}sU_{OMe}sC_{OMe}s-**Chol** | miR-122A | 7015.19 | 7015.7 | 97.9* |
| 3354 | A_{OMe}sA_{OMe}sC_{OMe}A_{OMe}C_{OMe}C_{OMe}A_{OMe}U_{OMe}U_{OMe}G_{OMe}U_{OMe}C_{OMe} A_{OMe}C_{OMe}sA_{OMe}sC_{OMe}sU_{OMe}s-**Chol** | miR-122A | 6352.74 | 6353.2 9 | 97.9* |
| 3355 | A_{OMe}sC_{OMe}sA_{OMe}A_{OMe}A_{OMe}C_{OMe}A_{OMe}A_{OMe}C_{OMe}A_{OMe}C_{OMe}U_{OMe} G_{OMe}U_{OMe}C_{OMe}A_{OMe}C_{OMe}A_{OMe}U_{OMe}U_{OMe}sC_{OMe}sC_{OMe}sA_{OMe}s-**Chol** | miR-122A | 8364.13 | 8364.4 5 | 90.2* |
| 3356 | A_{OMe}sC_{OMe}sA_{oMe}A_{OMe}A_{OMe}C_{OMe}A_{OMe}C_{OMe}C_{OMe}A_{OMe}C_{OMe}U_{OMe} G_{OMe}U_{OMe}C_{OMe}A_{OMe}C_{OMe}A_{OMe}U_{OMe}U_{OMe}sC_{OMe}sC_{OMe}sA_{OMe}s-**Chol** | miR-122A | 8340.09 | 8340.4 6 | 86.0* |
| 3357 | A_{OMe}sC_{OMe}sA_{OMe}A_{OMe}A_{OMe}C_{OMe}A_{OMe}C_{OMe}C_{OMe}A_{OMe}U_{OMe}U_{OMe} G_{OMe}U_{OMe}C_{OMe}A_{OMe}C_{OMe}A_{OMe}U_{OMe}U_{OMe}sC_{OMe}sC_{OMe}sA_{OMe}s-**Chol** | miR-122A | 8341.08 | 8341.4 3 | 79.0* |
| 3359 | Quasar5sA_{OMe}sC_{OMe}A_{OMe}C_{OMe}A_{OMe}C_{OMe}A_{OMe}A_{OMe}C_{OMe}A_{OMe}C_{O Me}U_{OMe}G_{OMe}U_{OMe}C_{OMe}A_{OMe}C_{OMe}A_{OMe}U_{OMe}U_{OMe}sC_{OMe}sC_{OMe}sA_{O Me}s-**Chol** | miR-122A | 8960.91 | 8960.78 | 93.0* |
| 3383 | A_{OMe}C_{OMe}A_{OMe}A_{OMe}A_{OMe}C_{OMe}C_{OMe}C_{OMe}C_{OMe}A_{OMe}U_{OMe}U_{OMe}G_{O Me}U_{OMe}C_{OMe}A_{OMe}C_{OMe}A_{OMe}C_{OMe}U_{OMe}C_{OMe}C_{OMe}A_{OMe}s-**Chol** | miR-122A | 8260.09 | 8260.1 3 | 86.0* |
| 3474 | Quasar5sA_{OMe}sC_{OMc}A_{OMe}A_{OMe}A_{OMe}C_{OMe}A_{OMe}C_{OMe}C_{OMe}A_{OMe}U_{O Me}U_{OMe}G_{OMe}U_{OMe}C_{OMe}A_{OMe}C_{OMe}A_{OMe}C_{OMe}U_{OMe}sC_{OMe}sC_{OMe}sA_{O Me}s-**Chol** | miR-122A | 8960.91 | 8960.6 5 | 98.9* |

The strands are shown written 5' to 3'. Lower case "s" indicates a phosphorothioate linkage. **"Chol-"** indicates a hydroxyprolinol cholesterol conjugate. Subscript "OMe" indicates a 2'-O-methyl sugar. "I" is ribo-Inosine nucleoside. Purity was determined by CGE except for the where indicated by (*), in these cases purity was determined by anion-exchange HPLC.

**Table 5: Double stranded oligonucleotides to modulate microRNAs**

| **AL-DP #** | **Strand 1** | **Strand 2** | **Target** |
|---|---|---|---|
| 3018 | AL-SQ-3035 | AL-SQ-30377 | miR-122A |
| 3019 | AL-SQ-3035 | AL-SQ-3038 | miR-122A |
| 3020 | AL-SQ-3036 | AL-SQ-3039 | miR-122A |
| 3021 | AL-SQ-3036 | AL-SQ-3040 | miR-122A |

**Table 6: Description of oligonucleotides synthesized to modulate microRNAs**

| **Sequence #** | **Description** |
|---|---|
| 3035 | complimentary to antagomir-122 |
| 3036 | complimentary to mm-antagomir-122 |
| 3037 | antagomir-122-fullyPS |
| 3038 | antagomir-122 |
| 3039 | mm-antagomir-122-fullyPS |
| 3040 | mm-antagomir-122 |
| 3223 | complimentary to antagomir-122 |
| 3224 | complimentary to mm-antagomir-122 |
| 3225 | anti-122-fully-PS |
| 3226 | anti-122-partial_PS |
| 3227 | antagomir-16 |
| 3228 | antagomir-192 |
| 3229 | antagomir-194 |
| 3230 | antagomir-375 |
| 3344 | complimentary to antagomir-122 with Adenosine -> Inosine modification |
| 3350 | antagomir-122-noPS |
| 3351 | antagomir-122-25mer |
| 3352 | antagomir-122-21mer |
| 3353 | antagomir-122-19mer |
| 3354 | antagomir-122-17mer |
| 3355 | mm-antagomir-122-3mm |
| 3356 | mm-antagomir-122-2mm |
| 3357 | mm-antagomir-122-1mm |
| 3359 | mm-antagomir-122-5'-Quasar570 |
| 3383 | Same as AL-3350 with P=S between 3'-end and Cholesterol |
| 3474 | antagomir-122-5'-Quasar570 |

### OTHER EMBODIMENTS

A number of embodiments of the invention have been described. Accordingly, other embodiments are within the scope of the following claims.

## Claims

1. An oligonucleotide agent for use in a method of treating a disorder that is associated with an aldolase-A deficiency, wherein the oligonucleotide agent is substantially single-stranded, comprises a sequence which is substantially complementary to 12 to 23 contiguous nucleotides of a microRNA target sequence, and wherein the target sequence differs by no more than 1, 2, or 3 nucleotides from the sequence set forth as SEQ ID NO: 1.

2. The oligonucleotide agent for use according to claim 1 for treating a disorder selected from hemolytic anemia, arthrogryposis complex congenita, pituitary ectopia, rhabdomyolysis, and hyperkalemia.

3. *In vitro* use of an oligonucleotide agent for increasing aldolase-A protein levels in a cell, wherein the oligonucleotide agent is substantially single-stranded, comprises a sequence which is substantially complementary to 12 to 23 contiguous nucleotides of a microRNA target sequence, and wherein the target sequence differs by no more than 1, 2, or 3 nucleotides from the sequence set forth as SEQ ID NO: 1.

4. The oligonucleotide agent for use according to claim 1, or the use according to claim 3, wherein the oligonucleotide agent comprises a nucleotide sequence sufficiently complementary to a microRNA target sequence of about 15 to 23 nucleotides, wherein the target sequence differs by no more than 1, 2, or 3 nucleotides from the sequence set forth as SEQ ID NO: 1.

5. The oligonucleotide agent for use according to claim 1, or the use according to claim 3, wherein the sequence which is substantially complementary to 12 to 23 contiguous nucleotides of a target sequence contains no more than 1, 2, 3, 4 or 5 mismatches to the 12 to 23 contiguous nucleotides of the target sequence.

6. The oligonucleotide agent for use according to claim 1, or the use according to claim 3, wherein the sequence which is substantially complementary to 12 to 23 contiguous nucleotides of a target sequence forms a duplex with the 12 to 23 contiguous nucleotides of the target sequence.

7. The oligonucleotide agent for use according to claim 1, or the use according to claim 3, wherein the oligonucleotide agent further comprises a non-nucleotide moiety.

8. The oligonucleotide agent for use according to claim 1, or the use according to claim 3, wherein the oligonucleotide agent is stabilized against nucleolytic degradation.

9. The oligonucleotide agent for use according to claim 1, or the use according to claim 3, wherein the oligonucleotide agent (i) comprises a phosphorothioate at the first internucleotide linkage at the 5' end of the nucleotide sequence, (ii) comprises a phosphorothioate at the first internucleotide linkage at the 3' end of the nucleotide sequence, or (iii) comprises a phosphorothioate at the first internucleotide linkage at the 5' end of the nucleotide sequence, and a phosphorothioate at the first internucleotide linkage at the 3' end of the nucleotide sequence.

10. The oligonucleotide agent for use according to claim 1, or the use according to claim 3, wherein the oligonucleotide agent comprises a 2'-modified nucleotide.

11. The oligonucleotide agent for use according to claim 1, or the use according to claim 10, wherein the 2' modified nucleotide comprises LNA.

12. The oligonucleotide agent for use according to claim 1, or the use according to claim 10, wherein the 2'-modified nucleotide comprises a modification selected from the group consisting of: 2'-deoxy, 2'-deoxy-2'-fluoro, 2'-O-methyl, 2'-O-methoxyethyl (2'-O-MOE), 2'-O-aminopropyl (2'-O-AP), 2'-O-dimethylaminoethyl (2'-O-DMAOE), 2'-O-dimethylaminopropyl (2'-O-DMAP), 2'-O-dimethylaminoethyloxyethyl (2'-O-DMAEOE), and 2'-O-N-methylacetamido (2'-O-NMA).

13. The oligonucleotide agent for use according to claim 1, or the use according to claim 10, wherein the 2'-modified nucleotide comprises a 2'-O-methyl.

14. The oligonucleotide agent for use according to claim 1, or the use according to claim 3, wherein the oligonucleotide agent further comprises a cholesterol molecule attached to the 3' end of the oligonucleotide agent.

15. Use of the oligonucleotide agent of claim 1 for the preparation of a medicament for treating a disorder selected from hemolytic anemia, arthrogryposis complex congenita, pituitary ectopia, rhabdomyolysis, and hyperkalemia.

## Patentansprüche

1. Ein Oligonukleotidwirkstoff zur Verwendung in einem Verfahren zur Behandlung einer Störung, die mit einer Aldolase-A-Defizienz assoziiert ist, wobei der Oligonukleotidwirkstoff im Wesentlichen einzelsträngig ist, eine Sequenz umfasst, welche im Wesentlichen komplementär zu 12 bis 23 zusammenhängenden Nukleotiden einer microRNA-Zielsequenz ist, und wobei die Zielsequenz in nicht mehr als 1, 2 oder 3 Nukleotiden von der in SEQ ID NO: 1 dargelegten Sequenz abweicht.

2. Der Oligonukleotidwirkstoff zur Verwendung gemäß Anspruch 1 zur Behandlung einer Störung ausgewählt aus hämolytischer Anämie, Arthrogryposis complex congenita, Ektopie der Hypophyse, Rhabdomyolyse, und Hyperkaliämie.

3. In vitro Verwendung eines Oligonukleotidwirkstoffs zur Anhebung des Aldolase-A Proteinspiegels in einer Zelle, wobei der Oligonukleotidwirkstoff im Wesentlichen einzelsträngig ist, eine Sequenz umfasst, welche im Wesentlichen komplementär zu 12 bis 23 zusammenhängenden Nukleotiden einer microRNA-Zielsequenz ist, und wobei die Zielsequenz in nicht mehr als 1, 2 oder 3 Nukleotiden von der in SEQ ID NO: 1 dargelegten Sequenz abweicht.

4. Der Oligonukleotidwirkstoff zur Verwendung gemäß Anspruch 1, oder die Verwendung gemäß Anspruch 3, wobei der Oligonukleotidwirkstoff eine Nukleotidsequenz umfasst, die hinreichend komplementär zu einer microRNA-Zielsequenz von etwa 15 bis 23 Nukleotiden ist, wobei die Zielsequenz in nicht mehr als 1, 2 oder 3 Nukleotiden von der in SEQ ID NO: 1 dargelegten Sequenz abweicht.

5. Der Oligonukleotidwirkstoff zur Verwendung gemäß Anspruch 1, oder die Verwendung gemäß Anspruch 3, wobei die Sequenz, die im Wesentlichen komplementär zu 12 bis 23 zusammenhängenden Nukleotiden einer Zielsequenz ist, nicht mehr als 1, 2, 3, 4 oder 5 Fehlpaarungen zu den 12 bis 23 zusammenhängenden Nukleotiden der Zielsequenz enthält.

6. Der Oligonukleotidwirkstoff zur Verwendung gemäß Anspruch 1, oder die Verwendung gemäß Anspruch 3, wobei die Sequenz, die im Wesentlichen komplementär zu 12 bis 23 zusammenhängenden Nukleotiden einer Zielsequenz ist, ein Duplex mit den 12 bis 23 zusammenhängenden Nukleotiden der Zielsequenz ausbildet.

7. Der Oligonukleotidwirkstoff zur Verwendung gemäß Anspruch 1, oder die Verwendung gemäß Anspruch 3, wobei der Oligonukleotidwirkstoff weiterhin eine nicht-Nukleotidgruppe umfasst.

8. Der Oligonukleotidwirkstoff zur Verwendung gemäß Anspruch 1, oder die Verwendung gemäß Anspruch 3, wobei der Oligonukleotidwirkstoff gegen nukleolytischen Abbau stabilisiert ist.

9. Der Oligonukleotidwirkstoff zur Verwendung gemäß Anspruch 1, oder die Verwendung gemäß Anspruch 3, wobei der Oligonukleotidwirkstoff (i) ein Phosphorothioat an der ersten Internukleotidverknüpfung am 5' Ende der Nukleotidsequenz umfasst, (ii) ein Phosphorothioat an der ersten Internukleotidverknüpfung am 3' Ende der Nukleotidsequenz umfasst, oder (iii) ein Phosphorothioat an der ersten Internukleotidverknüpfung am 5' Ende der Nukleotidsequenz und ein Phosphorothioat an der ersten Internukleotidverknüpfung am 3' Ende der Nukleotidsequenz umfasst.

10. Der Oligonukleotidwirkstoff zur Verwendung gemäß Anspruch 1, oder die Verwendung gemäß Anspruch 3, wobei der Oligonukleotidwirkstoff ein 2'-modifiziertes Nukleotid umfasst.

11. Der Oligonukleotidwirkstoff zur Verwendung gemäß Anspruch 1, oder die Verwendung gemäß Anspruch 10, wobei das 2'-modifizierte Nukleotid LNA umfasst.

12. Der Oligonukleotidwirkstoff zur Verwendung gemäß Anspruch 1, oder die Verwendung gemäß Anspruch 10, wobei das 2'-modifizierte Nukleotid eine Modifikation umfasst, ausgewählt aus der Gruppe bestehend aus: 2'-Deoxy, 2'-Deoxy-2'-Fluoro, 2'-O-Methyl, 2'-O-Methoxyethyl (2'-O-MOE), 2'-O-Aminopropyl (2'-O-AP), 2'-O-Dimethylaminoethyl (2'-O-DMAOE), 2'-O-Dimethylaminopropyl (2'-O-DMAP), 2'-O-Dimethylaminoethyloxyethyl (2'-O-DMAEOE), und 2'-O-N-Methylacetamido (2'-O-NMA).

13. Der Oligonukleotidwirkstoff zur Verwendung gemäß Anspruch 1, oder die Verwendung gemäß Anspruch 10, wobei das 2'-modifizierte Nukleotid ein 2'-O-Methyl umfasst.

14. Der Oligonukleotidwirkstoff zur Verwendung gemäß Anspruch 1, oder die Verwendung gemäß Anspruch 3, wobei der Oligonukleotidwirkstoff weiterhin ein Cholesterolmolekül umfasst, das an das 3'-Ende des Oligonukleotidwirkstoffs gebunden ist.

15. Verwendung eines Oligonukleotidwirkstoffs gemäß Anspruch 1 zur Herstellung eines Medikamentes zur Behandlung einer Störung ausgewählt aus hämolytischer Anämie, Arthrogryposis complex congenita, Ektopie der Hypophyse, Rhabdomyolyse, und Hyperkaliämie.

## Revendications

1. Un agent oligonucléotidique pour utiliser dans une méthode de traitement d'un trouble qui est associé à une déficience de l'aldolase A, dans lequel l'agent oligonucléotidique est essentiellement simple brin, comprend une séquence qui est essentiellement complémentaire de 12 à 23 nucléotides continus de la séquence cible d'ARN micro, et dans lequel la séquence cible diffère de pas plus de 1, 2 ou 3 nucléotides à partir de la séquence telle que dans SEQ ID NO: 1.

2. L'agent oligonucléotidique pour utiliser selon la revendication 1 pour le traitement d'un trouble sélectionné parmi l'anémie hémolytique, l'arthrogrypose congénitale complexe, l'ectopie pituitaire, une rhabdomyolyse et l'hyperkaliémie.

3. Utilisation *in vitro* d'un agent oligonucléotidique pour augmenter le taux de protéine aldolase A dans une cellule, dans laquelle l'agent oligonucléotidique est essentiellement simple brin, comprend une séquence qui est essentiellement complémentaire de 12 à 23 nucléotides continus de la séquence cible d'ARN micro, et dans lequel la séquence cible diffère de pas plus de 1, 2 ou 3 nucléotides à partir de la séquence telle que dans SEQ ID NO: 1.

4. L'agent oligonucléotidique pour utiliser selon la revendication 1, ou l'utilisation selon la revendication 3, dans lequel l'agent oligonucléotidique comprend une séquence nucléotidique suffisamment complémentaire d'une séquence cible d'ARN micro d'environ 15 à 23 nucléotides, dans lequel la séquence cible diffère de pas plus de 1, 2 ou 3 nucléotides à partir de la séquence telle que dans SEQ ID NO: 1.

5. L'agent oligonucléotidique pour utiliser selon la revendication 1, ou l'utilisation selon la revendication 3, dans lequel la séquence qui est essentiellement complémentaire des nucléotides contigus 12 à 23 de la séquence cible contient pas plus de 1, 2, 3, 4 ou 5 inadéquations entre les nucléotides 12 à 23 de la séquence cible.

6. L'agent oligonucléotidique pour utiliser selon la revendication 1, ou l'utilisation selon la revendication 3, dans lequel la séquence qui est essentiellement complémentaire des nucléotides contigus 12 à 23 de la séquence cible forme un duplex avec les nucléotides 12 à 23 de la séquence cible.

7. L'agent oligonucléotidique pour utiliser selon la revendication 1, ou l'utilisation selon la revendication 3, dans lequel l'agent oligonucléotidique comprend en outre un fragment non nucléotidique.

8. L'agent oligonucléotidique pour utiliser selon la revendication 1, ou l'utilisation selon la revendication 3, dans lequel l'agent oligonucléotidique est stabilisé contre la dégradation nucléolytique.

9. L'agent oligonucléotidique pour utiliser selon la revendication 1, ou l'utilisation selon la revendication 3, dans lequel l'agent oligonucléotidique (i) comprend un phosphorothioate à la première liaison internucléotidique à l'extrémité 5' de la séquence de nucléotides, (ii) comprend un phosphorothioate à la première liaison internucléotidique à l'extrémité 3' de la séquence de nucléotides, ou (iii) comprend un phosphorothioate à la première liaison internucléotidique à l'extrémité 5' de la séquence de nucléotide, et un phosphorothioate à la première liaison internucléotidique à l'extrémité 3' de la séquence de nucléotides.

10. L'agent oligonucléotidique pour utiliser selon la revendication 1, ou l'utilisation selon la revendication 3, dans lequel l'agent oligonucléotidique comprend un nucléotide modifié en 2'.

11. L'agent oligonucléotidique pour utiliser selon la revendication 1, ou l'utilisation selon la revendication 3, dans lequel le nucléotide modifié en 2' comprend LNA.

12. L'agent oligonucléotidique pour utiliser selon la revendication 1, ou l'utilisation selon la revendication 3, dans lequel le nucléotide modifié en 2' comprend une modification sélectionnée parmi le groupe consistant en: 2'-déoxy, 2'-déoxy-2'-fluoro, 2'-O-méthyle, 2'-O-méthoxyéthyle (2'-O-MOE), 2'-O-aminopropyle (2'-O-AP), 2'-O-diméthylaminoéthyle (2'-O-DMAOE), 2'-O-diméthylaminopropyle (2'-O-DMAP), 2'-O-diméthylaminoéthyloxyéthyle (2'-O-DMAEOE), et 2'-ON-méthylacetamido (2'-O-NMA).

13. L'agent oligonucléotidique pour utiliser selon la revendication 1, ou l'utilisation selon la revendication 3, dans lequel le nucléotide modifié en 2' comprend un 2'-O-méthyle.

14. L'agent oligonucléotidique pour utiliser selon la revendication 1 ou l'utilisation selon la revendication 3, dans lequel l'agent oligonucléotidique comprend en outre une molécule de cholestérol attachée à l'extrémité 3' de l'agent oligonucléotidique.

15. Utilisation de l'agent oligonucléotidique selon la revendication 1 pour la préparation d'un médicament pour le traitement d'un trouble sélectionné parmi l'anémie hémolytique, l'arthrogrypose congénitale complexe, l'ectopie pituitaire, une rhabdomyolyse et l'hyperkaliémie.


### SEQUENCE LISTING

<110> Alnylam Pharmaceuticals, Inc. and The Rockefeller University
<120> Chemically Modified Oligonucleotides for
   Use in Modulating Micro RNA and Uses Thereof
<130> 14174-121WO1
<150> US 60/706,866
   <151> 2005-08-10
<150> US 60/731,554
   <151> 2005-10-28
<150> US 60/763,201
   <151> 2006-01-26
<160> 44
<170> FastSEQ for Windows Version 4.0
<210> 1
   <211> 23
   <212> RNA
   <213> Mus musculus
<400> 1
   uggaguguga caaugguguu ugu 23
<210> 2
   <211> 22
   <212> RNA
   <213> Mus musculus
<400> 2
   uagcagcacg uaaauauugg cg 22
<210> 3
   <211> 21
   <212> RNA
   <213> Mus musculus
<400> 3
   cugaccuaug aauugacagc c 21
<210> 4
   <211> 22
   <212> RNA
   <213> Mus musculus
<400> 4
   uguaacagca acuccaugug ga 22
<210> 5
   <211> 23
   <212> RNA
   <213> Artificial Sequence
<220>
   <223> Synthetically generated oligonucleotide
<220>
   <221> modified_base
   <222> 1, 2, 20, 21, 22, 23
   <223> /2'-O-methyl modification phosphorothioate linkage corresponding base
<220>
   <221> modified_base
   <222> (3)...(19)
   <223>2'-O-methyl modified nucleotides corresponding base
<400> 5
   acaaacacca uugucacacu cca 23
<210> 6
   <211> 22
   <212> RNA
   <213> Artificial Sequence
<220>
   <223> Synthetically generated oligonucleotide
<220>
   <221> modified_base
   <222> 1, 2, 19, 20, 21, 22
   <223> 2'-O-methyl modification phosphorothioate linkage corresponding base
<220>
   <221> modified_base
   <222> (3) ... (18)
   <223> 2'-O-methyl modified nucleotides corresponding base
<400> 6
   cgccaauauu uacgugcugc ua 22
<210> 7
   <211> 21
   <212> RNA
   <213> Artificial Sequence
<220>
   <223> Synthetically generated oligonucleotide
<220>
   <221> modified_base
   <222> 1, 2, 18, 19, 20, 21
   <223>2'-O-methyl modification phosphorothioate linkage coresponding base
<220>
   <221> modified_base
   <222> (3)... (17)
   <223>2'-O-methyl modified nucleotides corresponding base
<400> 7
   ggcugucaau ucauagguca g 21
<210> 8
   <211> 22
   <212> RNA
   <213> Artificial Sequence
<220>
   <223> Synthetically generated oligonucleotide
<220>
   <221> modified_base
   <222> 1, 2, 19, 20, 21, 22
   <223>2'-O-methyl modification phosphorothioate linkage corresponding base
<220>
   <221> modified_base
   <222> (3)...(18)
   <223>2'-O-methyl modified nucleotides corresponding base
<400> 8
   uccacaugga guugcuguua ca 22
<210> 9
   <211> 25
   <212> DNA
   <213> Artificial Sequence
<220>
   <223> Primer
<400> 9
   ccccttaaat agttgtttat tggca 25
<210> 10
   <211> 23
   <212> RNA
   <213> Artificial Sequence
<220>
   <223> Synthetically generated oligonucleotide
<220>
   <221> modified_base
   <222> (1) ... (23)
   <223>2'-O-Me ribo sugar modification phosphorothioate linkage corresponding base
<400> 10
   acaaacacca uugucacacu cca 23
<210> 11
   <211> 23
   <212> RNA
   <213> Artificial Sequence
<220>
   <223> Synthetically generated oligonucleotide
<220>
   <221> modified_base
   <222> 1, 2, 20, 21, 22, 23
   <223> 2'-O-Me ribo sugar modification phosphorothioate linkage corresponding base
<220>
   <221> modified_base
   <222> (3)...(19)
   <223> 2'-O-Me ribo sugar modification corresponding base
<400> 11
   acaaacacca uugucacacu cca 23
<210> 12
   <211> 23
   <212> RNA
   <213> Artificial Sequence
<220>
   <223> Synthetically generated oligonucleotide
<400> 12
   uggaauguga caguguugug ugu 23
<210> 13
   <211> 23
   <212> RNA
   <213> Artificial Sequence
<220>
   <223> Synthetically generated oligonucleotide
<220>
   <221> modified_base
   <222> (1)...(23)
   <223> 2'-O-Me ribo sugar modification phosphorothioate linkage corresponding base
<400> 13
   acacacaaca cugucacauu cca 23
<210> 14
   <211> 23
   <212> RNA
   <213> Artificial Sequence
<220>
   <223> Synthetically generated oligonucleotide
<220>
   <221> modified_base
   <222> 1, 2, 20, 21, 22, 23
   <223> 2'-O-Me ribo sugar modification phosphorothioate linkage corresponding base
<220>
   <221> modified_base
   <222> (3)...(19)
   <223> 2'-O-Me ribo sugar modification corresponding base
<400> 14
   acacacaaca cugucacauu cca 23
<210> 15
   <211> 31
   <212> DNA
   <213> Artificial Sequence
<220>
   <223> Synthetically generated oligonucleotide
<220>
   <221> modified_base
   <222> (1) ... (23)
   <223>2'-O-Me ribo sugar modification corresponding base
<220>
   <221> modified_base
   <222> 24, 25, 26, 27
   <223> deoxyribo sugar modification
<220>
   <221> modified_base
   <222> 28, 29, 30
   <223> 2'-O-Me ribo sugar modification corresponding base
<220>
   <221> modified_base
   <222> 31
   <223> 2'-O-Me ribo sugar modification phosphorothioate linkage
<400> 15
   acaaacacca uugucacacu ccattttugg a 31
<210> 16
   <211> 31
   <212> DNA
   <213> Artificial Sequence
<220>
   <223> Synthetically generated oligonucleotide
<220>
   <221> modified_base
   <222> (1)...(22)
   <223> 2'-O-Me ribo sugar modification corresponding base
<220>
   <221> modified_base
   <222> 23, 31
   <223> 2'-O-Me ribo sugar modification phosphorothioate linkage corresponding base
<220>
   <221> modified_base
   <222> 24, 25, 26, 27
   <223> deoxyribo sugar modification phosphorothioate linkage
<220>
   <221> modified_base
   <222> 28, 29, 30
   <223>2'-O-Me ribo sugar modification corresponding base
<400> 16
   acaaacacca uugucacacu ccattttugg a 31
<210> 17
   <211> 7
   <212> RNA
   <213> Artificial Sequence
<220>
   <223> Synthetically generated oligonucleotide
<220>
   <221> modified_base
   <222> (1)...(7)
   <223> 2'-O-Me ribo sugar modification corresponding base
<400> 17
   uggagug 7
<210> 18
   <211> 7
   <212> RNA
   <213> Artificial Sequence
<220>
   <223> Synthetically generated oligonucleotide
<220>
   <221> modified_base
   <222> (1) ... (7)
   <223> 2'-O-Me ribo sugar modification corresponding base
<400> 18
   gacaaug 7
<210> 19
   <211> 7
   <212> RNA
   <213> Artificial Sequence
<220>
   <223> Synthetically generated oligonucleotide
<220>
   <221> modified_base
   <222> (1)...(7)
   <223> 2'-O-Me ribo sugar modification corresponding base
<400> 19
   uggaaug 7
<210> 20
   <211> 7
   <212> RNA
   <213> Artificial Sequence
<220>
   <223> Synthetically generated oligonucleotide
<220>
   <221> modified_base
   <222> (1)...(7)
   <223>2'-O-Me ribo sugar modification corresponding base
<400> 20
   gacagug 7
<210> 21
   <211> 23
   <212> RNA
   <213> Artificial Sequence
<220>
   <223> Synthetically generated oligonucleotide
<220>
   <221> modified_base
   <222> 1, 2, 19, 20, 21, 22
   <223> 2'-O-Me ribo sugar modification phosphorothioate linkage corresponding base
<220>
   <221> modified_base
   <222> (3)...(18)
   <223> 2'-O-Me ribo sugar modification corresponding base
<220>
   <221> modified_base
   <222> 23
   <223> 2'-O-Me ribo sugar modification corresponding base
<400> 21
   uggaguguga caaugguguu ugu 23
<210> 22
   <211> 23
   <212> RNA
   <213> Artificial Sequence
<220>
   <223> Synthetically generated oligonucleotide
<220>
   <221> modified_base
   <222> 1, 2, 19, 20, 21, 22
   <223> 2'-O-Me ribo sugar modification phosphorothioate linkage corresponding base
<220>
   <221> modified_base
   <222> (3)...(18)
   <223> 2'-O-Me ribo sugar modification corresponding base
<220>
   <221> modified_base
   <222> 23
   <223> 2'-O-Me ribo sugar modification corresponding base
<400> 22
   uggaauguga caguguugug ugu 23
<210> 23
   <211> 23
   <212> RNA
   <213> Artificial Sequence
<220>
   <223> Synthetically generated oligonucleotide
<220>
   <221> modified_base
   <222> (1)..(22)
   <223> 2'-O-Me ribo sugar modification phosphorothioate linkage corresponding base
<220>
   <221> modified_base
   <222> 23
   <223> 2'-O-Me ribo sugar modification corresponding base
<400> 23
   acaaacacca uugucacacu cca 23
<210> 24
   <211> 23
   <212> RNA
   <213> Artificial Sequence
<220>
   <223> Synthetically generated oligonucleotide
<220>
   <221> modified_base
   <222> 1, 2, 19, 20, 21, 22
   <223> 2'-O-Me ribo sugar modification phosphorothioate linkage corresponding base
<220>
   <221> modified_base
   <222> (3)...(18)
   <223> 2'-O-Me ribo sugar modification corresponding base
<220>
   <221> modified_base
   <222> 23
   <223> 2'-O-Me ribo sugar modification corresponding base
<400> 24
   acaaacacca uugucacacu cca 23
<210> 25
   <211> 22
   <212> RNA
   <213> Artificial Sequence
<220>
   <223> Synthetically generated oligonucleotide
<220>
   <221> modified_base
   <222> 1, 2, 19, 20, 21, 22
   <223> 2'-O-Me ribo sugar modification phosphorothioate linkage corresponding base
<220>
   <221> modified_base
   <222> (3)...(18)
   <223> 2'-O-Me ribo sugar modification corresponding base
<400> 25
   cgccaauauu uacgugcugc ua 22
<210> 26
   <211> 21
   <212> RNA
   <213> Artificial Sequence
<220>
   <223> Synthetically generated oligonucleotide
<220>
   <221> modified_base
   <222> 1, 2, 18, 20, 21
   <223> 2'-O-Me ribo sugar modification phosphorothioate linkage corresponding base
<220>
   <221> modified_base
   <222> (3)...(17
   <223> 2'-O-Me ribo sugar modification corresponding base
<400> 26
   ggcugucaau ucauagguca g 21
<210> 27
   <211> 22
   <212> RNA
   <213> Artificial Sequence
<220>
   <223> Synthetically generated oligonucleotide
<220>
   <221> modified_base
   <222> 1, 2, 19, 20, 21, 22
   <223> 2'-O-Me ribo sugar modification phosphorothioate linkage corresponding base
<220>
   <221> modified_base
   <222> (3)...(18)
   <223> 2'-O-Me ribo sugar modification corresponding base
<400> 27
   uccacaugga guugcuguua ca 22
<210> 28
   <211> 22
   <212> RNA
   <213> Artificial Sequence
<220>
   <223> Synthetically generated oligonucleotide
<220>
   <221> modified_base
   <222> 1, 2, 19, 20, 21, 22
   <223> 2'-O-Me ribo sugar modification phosphorothioate linkage corresponding base
<220>
   <221> modified_base
   <222> (3)...(18)
   <223> 2'-O-Me ribo sugar modification corresponding base
<400> 28
   ucacgcgagc cgaacgaaca aa 22
<210> 29
   <211> 23
   <212> RNA
   <213> Artificial Sequence
<220>
   <223> Synthetically generated oligonucleotide
<220>
   <221> misc_feature
   <222> 4, 10, 12, 13
   <223> n = inosine
<400> 29
   uggngugugn cnnugguguu ugu 23
<210> 30
   <211> 23
   <212> RNA
   <213> Artificial Sequence
<220>
   <223> Synthetically generated oligonucleotide
<220>
   <221> modified_base
   <222> (1)..(23)
   <223> 2'-O-Me ribo sugar modification corresponding base
<400> 30
   acaaacacca uugucacacu cca 23
<210> 31
   <211> 25
   <212> RNA
   <213> Artificial Sequence
<220>
   <223> Synthetically generated oligonucleotide
<220>
   <221> modified_base
   <222> 1, 2, 22, 23, 24, 25
   <223> 2'-O-Me ribo sugar modification phosphorothioate linkage corresponding base
<220>
   <221> modified_base
   <222> (3)...(21
   <223> 2'-O-Me ribo sugar modification corresponding base
<400> 31
   cacaaacacc auugucacac uccac 25
<210> 32
   <211> 21
   <212> RNA
   <213> Artificial Sequence
<220>
   <223> Synthetically generated oligonucleotide
<220>
   <221> modified_base
   <222> 1, 2, 18, 19, 20, 21
   <223> 2'-O-Me ribo sugar modification phosphorothioate linkage corresponding base
<220>
   <221> modified_base
   <222> (3)...(17
   <223> 2'-O-Me ribo sugar modification corresponding base
<400> 32
   caaacaccau ugucacacuc c 21
<210> 33
   <211> 19
   <212> RNA
   <213> Artificial Sequence
<220>
   <223> Synthetically generated oligonucleotide
<220>
   <221> modified_base
   <222> (1) .. (15)
   <223> 2'-O-Me ribo sugar modification corresponding base
<220>
   <221> modified_base
   <222> 16, 17, 18, 19
   <223> 2'-O-Me ribo sugar modification phosphorothioate linkage corresponding base
<400> 33
   aaacaccauu gucacacuc 19
<210> 34
   <211> 17
   <212> RNA
   <213> Artificial Sequence
<220>
   <223> Synthetically generated oligonucleotide
<220>
   <221> modified_base
   <222> (3)..(13)
   <223> 2'-O-Me ribo sugar modification corresponding base
<220>
   <221> modified_base
   <222> 1, 2, 14,15, 16, 17
   <223> 2'-O-Me ribo sugar modification phosphorothioate linkage corresponding base
<400> 34
   aacaccauug ucacacu 17
<210> 35
   <211> 23
   <212> RNA
   <213> Artificial Sequence
<220>
   <223> Synthetically generated oligonucleotide
<220>
   <221> modified_base
   <222> 1, 2, 20, 21, 22, 23
   <223> 2'-O-Me ribo sugar modification phosphorothioate linkage corresponding base
<220>
   <221> modified_base
   <222> (3)...(19)
   <223> 2'-O-Me ribo sugar modification corresponding base
<400> 35
   acaaacaaca cugucacauu cca 23
<210> 36
   <211> 23
   <212> RNA
   <213> Artificial Sequence
<220>
   <223> Synthetically generated oligonucleotide
<400> 36
   acaaacacca cugucacauu cca 23
<210> 37
   <211> 23
   <212> RNA
   <213> Artificial Sequence
<220>
   <223> Synthetically generated oligonucleotide
<220>
   <221> modified_base
   <222> 1, 2, 20, 21, 22, 23
   <223> 2'-O-Me ribo sugar modification phosphorothioate linkage corresponding base
<220>
   <221> modified_base
   <222> (3)...(19)
   <223> 2'-O-Me ribo sugar modification corresponding base
<400> 37
   acaaacacca uugucacauu cca 23
<210> 38
   <211> 23
   <212> RNA
   <213> Artificial Sequence
<220>
   <223> Synthetically generated oligonucleotide
<220>
   <221> modified_base
   <222> 1, 20, 21, 22, 23
   <223> 2'-O-Me ribo sugar modification phosphorothioate linkage corresponding base
<220>
   <221> modified_base
   <222> (2)...(19)
   <223> 2'-O-Me ribo sugar modification corresponding base
<400> 38
   acaaacacca uugucacacu cca 23
<210> 39
   <211> 23
   <212> RNA
   <213> Artificial Sequence
<220>
   <223> Synthetically generated oligonucleotide
<220>
   <221> modified_base
   <222> 1, 20, 21, 22, 23
   <223> 2'-O-Me ribo sugar modification phosphorothioate linkage corresponding base
<220>
   <221> modified_base
   <222> (2)...(19)
   <223> 2'-O-Me ribo sugar modification corresponding base
<400> 39
   acacacaaca cugucacauu cca 23
<210> 40
   <211> 23
   <212> RNA
   <213> Artificial Sequence
<220>
   <223> Synthetically generated oligonucleotide
<400> 40
   acaaacacca uugucacacu cca 23
<210> 41
   <211> 21
   <212> RNA
   <213> Artificial Sequence
<220>
   <223> Synthetically generated oligonucleotide
<400> 41
   uaaggcacgc ggugaaugcc a 21
<210> 42
   <211> 35
   <212> DNA
   <213> Artificial Sequence
<220>
   <223> Primer
<400> 42
   agtcagatgt acagttataa gcacaagagg accag 35
<210> 43
   <211> 25
   <212> DNA
   <213> Artificial Sequence
<220>
   <223> Primer
<400> 43
   ttattcaaga tcccggggct cttcc 25
<210> 44
   <211> 25
   <212> DNA
   <213> Artificial Sequence
<220>
   <223> Primer
<400> 44
   ccagagctga actaaggctg ctcca 25